(19) Europäisches Patentamt
European Patent Office
Office européen des brevets

(11) EP 1 751 093 B1

(12) # EUROPÄISCHE PATENTSCHRIFT

(45) Veröffentlichungstag und Bekanntmachung des Hinweises auf die Patenterteilung:
**06.10.2010 Patentblatt 2010/40**

(21) Anmeldenummer: **05745355.7**

(22) Anmeldetag: **06.05.2005**

(51) Int Cl.:
***C07C 237/14*** (2006.01) ***C07C 231/12*** (2006.01)
***A61K 31/16*** (2006.01) ***A61P 25/04*** (2006.01)

(86) Internationale Anmeldenummer:
**PCT/EP2005/004909**

(87) Internationale Veröffentlichungsnummer:
**WO 2005/110976 (24.11.2005 Gazette 2005/47)**

(54) **SUBSTITUIERTE CYCLOHEXYLESSIGSÄURE-DERIVATE**
SUBSTITUTED CYCLOHEXYLACETIC ACID DERIVATIVES
DERIVES D'ACIDE CYCLOHEXYLE-ACETIQUE SUBSTITUES

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**LV**

(30) Priorität: **10.05.2004 DE 102004023507**

(43) Veröffentlichungstag der Anmeldung:
**14.02.2007 Patentblatt 2007/07**

(73) Patentinhaber: **Grünenthal GmbH**
**52078 Aachen (DE)**

(72) Erfinder:
- **HINZE, Claudia**
**52066 Aachen (DE)**
- **SUNDERMANN, Bernd**
**52074 Aachen (DE)**
- **SCHICK, Hans**
**13086 Berlin-Weissensee (DE)**
- **HENKEL, Birgitta**
**12555 Berlin (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**EP-A- 1 323 710** **WO-A-2004/043949**
**US-A1- 2003 236 250**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 751 093 B1

## Beschreibung

Die vorliegende Erfindung betrifft substituierte Cyclohexylessigsäure-Derivate, Verfahren zu deren Herstellung, Arzneimittel enthaltend diese Verbindungen und die Verwendung von substituierten Cyclohexylessigsäure-Derivate-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische μ-Opioide wie Morphin sind bei der Therapie starker bis stärkster Schmerzen gut wirksam und von größter Bedeutung für die Schmerztherapie. Es kann jedoch von Vorteil sein, wenn neben dem μ-Opioid-Rezeptor auch andere Opioid-Rezeptoren, insbesondere der ORL-1-Rezeptor, beeinflusst werden, da die reinen μ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Applikation, Wiley VCH, 2002).

Darüber hinaus ist bekannt, dass eine sich eine Beeinflussung der Serotonin- und/oder Noradrenalin-Wiederaufnahme günstig auf das Wirk- und Nebenwirkungsspektrum von Opioiden auswirken kann (Beispiel: Tramadol, s. Opioids with Clinical Relevance: Tramadol, 228-230 in: Analgesics - From Chemistry and Pharmacology to Clinical Application, Wiley VCH 2002).

Der ORL1-Rezeptor ist außerdem noch an der Regulation weiterer physiologischer und pathophysiologischer Prozesse beteiligt, Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, In denen der ORL1-Rezeptor eine Rolle spielt oder mit hoher Wahracheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, Intestinale Motilität (Diarrhoe), relaxierende Effekte auf die Aternwege, Mikturations Reflex (Harninkontinenz). Weiter wird die Verwendung von Agonisten und Antagonisten als Anoretika, Analgetika (auch In Coadministration mit Opioiden) oder Nootropika diskutiert.

EP-A-1 323 710 und US 2003/236250 offenbaren Cyclohexylessigsäure-Derivate. Aus dem Stand der Technik (WO 02090317) sind strukturell verwandte Verbindungen bekannt, die eine Affinität zum ORL-1-Rezeptor besitzen. Für diese Strukturklasse wurde bislang kein Einfluss auf die Noradrenalin- und Serotonin-Wiederaufnahme beschrieben.

Aufgabe der vorliegenden Erfindung war es, Arzneimittel zur Verfügung zu stellen, die auf das Opioid-Rezeptor-System wirken und damit für Arzneimittel, insbesondere zur Behandlung der verschiedenen mit diesem System nach dem Stand der Technik in Verbindung stehenden Krankeiten bzw. zum Einsatz in den dort genannten Indikationen, geeignet sind. Daüber hinaus sollten die Verbindungen die Noradrenalin- und Serotonin-Wiederaufnahme beeinflussen.

Gegenstand der Erfindung sind daher substituierte Cyclohexylessigsäure-Derivate der allgemeinen Formel I,

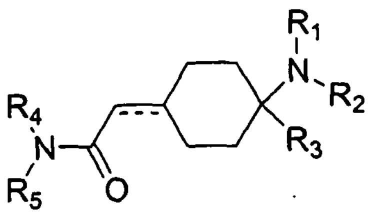

I

, worin

------

für eine C-C-Einfachbindung oder -Doppelbindung steht,

$R^1$ und $R^2$, unabhängig voneinander für H; CHO; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{10}CH_2CH_2$ oder $(CH_2)_{3-6}$ stehen,

wobei $R^{10}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

$R^3$ für $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; über $C_{1-3}$-Alkyl-Gruppe gebundenes Aryl, Heteroaryl oder $C_{3-8}$-Cycloalkyl, jeweils unsubstituiert oder einfach oder mehrfach substituiert; Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-*tert.*-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl, steht;

$R^4$ für für-$(CR^6R^7)_nR^8$ steht,

wobei n 0, 1, 2, 3, 4, 5 oder 6 bedeutet

$R^6$ H oder $C_{1-5}$-Alkyl, gesättigt oder ungesättigt, verzweigt oder unverzweigt, unsubstituiert oder einfach oder mehrfach substituiert, bedeutet

$R^7$ H, $C_{1-5}$-Alkyl, gesättigt oder ungesättigt, verzweigt oder unverzweigt, unsubstituiert oder einfach oder mehrfach substituiert, oder $COOR^9$ bedeutet,

oder $R^6$ und $R^7$ einen Ring $(CH_2)_kCHR^8(CH_2)_m$ bilden, mit k = 1, 2 oder 3 und m = 1 oder 2;

$R^8$ $C_{3-8}$-Cycloalkyl, Aryl oder Heteroaryl, jeweils unsubstituiert oder einfach oder mehfach substituiert, bedeutet,

$R^9$ H oder $C_{1-5}$-Alkyl, gesättigt oder ungesättigt, verzweigt oder unverzweigt, unsubstituiert oder einfach oder mehrfach substituiert, bedeutet;

$R^5$ für H oder für $-(CH_2)_lR^8$ steht,

wobei l für 1, 2 oder 3 steht,

oder zusammen mit $R^4$ für $CH_2CH_2OCH_2CH_2$, $CH_2CH_2NR^{11}CH_2CH_2$ oder $(CH_2)_{3-6}$ steht,

wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Verbindungen zeigen gute Bindung an den μ-Rezeptor und den ORL-1-Rezeptor, aber auch an andere Opioidrezeptoren. Überraschenderweise zeigte es sich, dass die Verbindungen auch gute Inhibitoren der Noradrenalin- und der Serotonin-Wiederaufnahme sind. Damit eignen sie sich auch zur Behandlung von Depressionen, und/oder Bulimie und/oder Anorexie und/oder Katalepsie und/oder zur Anxiolyse und/oder zur Vigilanz- und/oder Libidosteigerung.

Die Ausdrücke "$C_{1-5}$-Alkyl" und "$C_{1-3}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d:h. $C_{1-5}$-Alkanyle, $C_{2-5}$-Alkenyle und $C_{2-5}$-Alkinyle bzw. $C_{1-3}$-Alkanyle, $C_{2-3}$-Alkenyle und $C_{2-3}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $- C\equiv C-CH_3$), 1,1-Dimethylethyl, 1,1-Dimethylpropyl, Butenyl, Butinyl, Pentenyl und Pentinyl, umfaßt.

Der Ausdruck "Cycloalkyl" oder "$C_{3-8}$-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber

nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist $C_{3-8}$-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, =O, -CN, $NH_2$, NH-Alkyl, NH-Aryl, NH-Heteroaryl, NH-Cycloalkyl, NH-Alkyl-Aryl, NH-Alkyl-Heteroaryl, NH-Alkyl-OH, $N(Alkyl)_2$, $N(Alkyl-Aryl)_2$, $N(Alkyl-Heteroaryl)_2$, $N(Cycloalkyl)_2$, $N(Alkyl-OH)_2$, $NO_2$, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, $C(=O)C_{1-6}$-Alkyl, $C(=S)C_{1-6}$-Alkyl, C(=O)Aryl, C(=S)Aryl, $C(=O)C_{1-6}$-Alkyl-Aryl, $C(=S)C_{1-6}$-Alkyl-Aryl, C(=O)-Heteroaryl, C(=S)-Heteroaryl, C(=O)-Cycloalkyl, C(=S)-Cycloalkyl, $CO_2H$, $CO_2$-Alkyl, $CO_2$-Alkyl-Aryl, $C(=O)NH_2$, C(=O)NH-Alkyl, C(=O)NHAryl, C(=O)NH-Cycloalkyl, $C(=O)N(Alkyl)_2$, $C(=O)N(Alkyl-Aryl)_2$, $C(=O)N(Alkyl-Heteroaryl)_2$, $C(=O)N(Cycloalkyl)_2$, SO-Alkyl, $SO_2$-Alkyl, $SO_2NH_2$, $SO_3H$, $PO(O-C_{1-6}-Alkyl)_2$, $Si(C_{1-6}-Alkyl)_3$, $Si(C_{3-8}-Cycloalkyl)_3$, $Si(CH_2-C_{3-8}-Cycloalkyl)_3$, $Si(Phenyl)_3$, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von $CF_3$ oder $-CH_2CF_3$ oder an verschiedenen Stellen wie im Falle von $-CH(OH)-CH=CH-CHCl_2$. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch $-O-CH_2-CH_2-O-CH_2-CH_2-OH$.

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, $NH_2$, NH-Alkyl, NH-Aryl, NH-Heteroaryl, NH-Alkyl-Aryl, NH-Alkyl-Heteroaryl, NH-Cycloalkyl, NH-Alkyl-OH, $N(Alkyl)_2$, $N(Alkyl-Aryl)_2$, $N(Alkyl-Heteroaryl)_2$, $N(Cycloalkyl)_2$, $N(Alkyl-OH)_2$, $NO_2$, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, $C(=O)C_{1-6}$-Alkyl, $C(=S)C_{1-6}$-Alkyl, C(=O)Aryl, C(=S)Aryl, $C(=O)-C_{1-6}$-Alkyl-Aryl, $C(=S)C_{1-6}$-Alkyl-Aryl, C (=O)-Heteroaryl, C(=S)-Heteroaryl, C(=O)-Cycloalkyl, C(=S)-Cycloalkyl, $CO_2H$; $CO_2$-Alkyl, $CO_2$-Alkyl-Aryl, $C(=O)NH_2$, C(=O)NH-Alkyl, C(=O)NHAryl, C(=O)NH-Cycloalkyl, $C(=O)N(Alkyl)_2$, $C(=O)N(Alkyl-Aryl)_2$, $C(=O)N(Alkyl-Heteroaryl)_2$, $C(=O)N(Cycloalkyl)_2$, S(O)-Alkyl, S(O)-Aryl, $SO_2$-Alkyl, $SO_2$-Aryl, $SO_2NH_2$, $SO_3H$, $CF_3$, =O, =S; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren

oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, $\alpha$-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, $\alpha$-Liponsäure, Acetylglycin, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen substituierten Cyclohexylessigsäure-Derivate gilt, dass

$R^1$ und $R^2$ unabhängig voneinander für H; $C_{1-5}$-Alkyl, gesättigt oder ungesättigt, verzweigt oder unverzweigt, einfach oder mehrfach substituiert oder unsubstituiert, stehen;

oder die Reste $R^1$ und $R^2$ zusammen einen Ring bilden und $CH_2CH_2OCH_2CH_2$, $CH_2CH_2NR^{10}CH_2CH_2$ oder $(CH_2)_{3-6}$ bedeuten,

wobei $R^{10}$ H; $C_{1-5}$-Alkyl, gesättigt oder ungesättigt, verzweigt oder unverzweigt, einfach oder mehrfach substituiert oder unsubstituiert, bedeutet.

Besonders bevorzugt sind substituierte Cyclohexylessigsäure-Derivate, worin $R^1$ und $R^2$ unabhängig voneinander für $CH_3$ oder H stehen, wobei $R^1$ und $R^2$ nicht gleichzeitig H bedeuten.

Weiterhin bevorzugt sind substituierte Cyclohexylessigsäure-Derivate, worin

$R^3$ für Cyclopentyl, Cyclohexyl, Naphthyl, Thiophenyl, Benzothiophenyl, Furyl, Benzofuranyl, Benzodioxolanyl, Indolyl, Indanyl, Benzodioxanyl oder Pyridyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-*tert.*-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl steht;

insbesondere

$R^3$ für Naphthyl, Thiophenyl oder Pyridyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl., Phenyl, Naphthyl, Thiophenyl, Pyridyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-*tert*.-Butylphenyl, 4-Fluor-3-chlorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl oder 4-Chlor-3-trifluormethyl, steht.

Besonders bevorzugt sind substituierte Cyclohexylessigsäure-Derivate, worin $R^3$ für Pyridyl, substituiert oder unsubstituiert, oder Phenyl, 2-Fluorphenyl, 3-Fluorphenyl oder 4-Fluorphenyl steht.

Bevorzugt sind auch substituierte Cyclohexylessigsäure-Derivate, bei denen $R^6$ H und $R^7$ H, $CH_3$ oder $COOR^9$ bedeutet oder $R^6$ und $R^7$ einen Ring $(CH_2)_kCHR^8(CH_2)_m$ bilden mit k = 1, 2 oder 3 und m = 1 oder 2.

Außerdem bevorzugt sind substituierte Cyclohexylessigsäure-Derivate, bei denen $R^5$ H bedeutet.

Weiterhin bevorzugt sind substituierte Cyclohexylessigsäure-Derivate, bei denen $R^8$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet,

insbesondere

$R^8$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Besonders bevorzugt sind substituierte Cyclohexylessigsäure-Derivate, bei denen $R^8$ Phenyl oder Indolyl, jeweils einfach oder mehrfach substituiert, bedeutet.

Ganz besonders bevorzugt sind substituierte Cyclohexylessigsäure-Derivate aus der Gruppe

2-(4-Dimethylamino-4-phenyl-cyclohexyliden)-N-[2-(1H-indol-3-yl)-ethyl]-acetamid; hydrochlorid
2-[2-(4-Dimethylamino-4-phenyl-cyclohexyliden)-acetylamino]-3-(1H-indol-3-yl)-propansäure methylester; hydrochlorid
2-(4-Dimethylamino-4-phenyl-cyclohexyliden)-N-(3-phenyl-propyl)-acetamid; hydrochlorid
N-Benzyl-2-(4-dimethylamino-4-phenyl-cyclohexyliden)-acetamid; hydrochlorid
2-[2-(4-Dimethylamino-4-phenyl-cyclohexyliden)-acetylamino]-3-(1H-indol-3-yl)-propansäure methylester; hydrochlorid
2-(4-Dimethylamino-4-phenyl-cyclohexyliden)-N-phenethyl-acetamid; hydrochlorid 2-(4-Dimethylamino-4-phenyl-cyclohexyliden)-N-[2-(4-fluoro-phenyl)-ethyl]-acetamid; hydrochlorid
2-(4-Dimethylamino-4-phenyl-cyclohexyliden)-N-(4-fluoro-benzyl)-acetamid; hydrochlorid
2-(4-Dimethylamino-4-phenyl-cyc(ohexyliden)-N-(2-trifluoromethyl-benzyl)-acetamid; hydrochlorid
2-(4-Dimethylamino-4-phenyl-cyclohexyliden)-N-(4-fluoro-phenyl)-acetamid; hydrochlorid
2-(4-Dimethylamino-4-phenyl-cyclohexyliden)-N-[2-(1H-indol-3-yl)-1-methyl-ethyl]-acetamid; hydrochlorid
2-(4-Dimethylamino-4-phenyl-cyclohexyliden)-N-(4-phenyl-butyl)-acetamid; hydrochlorid
2-(4-Dimethylamino-4-phenyl-cyclohexyliden)-N-(1H-indol-3-ylmethyl)-acetamid; hydrochlorid
2-(4-Dimethylamino-4-phenyl-cyclohexyliden)-N-[4-(1H-indol-3-yl)-butyl]-acetamid; hydrochlorid
2-(4-Dimethylamino-4-phenyl-cyclohexyliden)-N-[3-(1H-indol-3-yl)-propyl]-acetamid; hydrochlorid
2-(4-Dimethylamino-4-phenyl-cyclohexyliden)-N-[5-(1H-indol-3-yl)-pentyl]-acetamid; hydrochlorid
2-(4-Dimethylamino-4-phenyl-cyclohexyliden)-N-[6-(1H-indol-3-yl)-hexyl]-acetamid; hydrochlorid
2-[4-Dimethylamino-4-(4-fluoro-phenyl)-cyclohexyliden]-N-(3-phenyl-propyl)-acetamid; hydrochlorid
2-[4-Dimethylamino-4-(4-fluoro-phenyl)-cyclohexyliden]-N-[2-(1H-indol-3-yl)-ethyl]-acetamid; hydrochlorid
2-[4-Dimethylamino-4-(4-fluoro-phenyl)-cyclohexyliden]-N-[2-(1H-indol-3-yl)-1-methyl-ethyl]-acetamid; hydrochlorid
2-(4-Dimethylamino-4-pyridin-2-yl-cyclohexyliden)-N-[2-(1H-indol-3-yl)-ethyl]-acetamid; hydrochlorid
2-(4-Dimethylamino-4-pyridin-2-yl-cyclohexyliden)-N-(3-phenyl-propyl)-acetamid; hydrochlorid
2-[4-Dimethylamino-4-(3-fluoro-phenyl)-cyclohexyliden]-N-[2-(1H-indol-3-yl)-ethyl]-acetamid; hydrochlorid
2-[4-Dimethylamino-4-(3-fluoro-phenyl)-cyclohexyliden]-N-(3-phenyl-propyl)-acetamid; hydrochlorid
2-[4-Dimethylamino-4-(3-fluoro-phenyl)-cyclohexyliden]-N-[2-(1H-indol-3-yl)-1-methyl-ethyl]-acetamid; hydrochlorid
2-(4-Dimethylamino-4-phenylcyclohexyl)-N-(2-trifluoromethyl-benzyl)-acetamid; hydrochlorid
2-(4-Dimethylamino-4-phenylcyclohexyl)-N-[2-(1H-indol-3-yl)-ethyl]-acetamid; hydrochlorid

2-(4-Dimethylamino-4-phenylcyclohexyl)-N-(3-phenylpropyl)-acetamid; hydrochlorid
2-(4-Dimethylamino-4-phenylcyclohexyl)-N-phenethylacetamid; hydrochlorid
2-(4-Dimethylamino-4-phenylcyclohexyl)-N-(4-phenylbutyl)-acetamid; hydrochlorid
2-[2-(4-Dimethylamino-4-phenylcyclohexyl)-acetylamino]-3-(1H-indol-3-yl)-propansäure-methylester; hydrochlorid
Kalium; 2-[2-(4-dimethylamino-4-phenylcyclohexyl)-acetylamino]-3-(1H-indol-3-yl)-propanat
2-(4-Dimethylamino-4-phenylcyclohexyl)-N-(1H-indol-3-ylmethyl)-acetamid; hydrochlorid
2-(4-Dimethylamino-4-phenylcyclohexyl)-N-[4-(1H-indol-3-yl)-butyl]-acetamid; hydrochlorid
2-(4-Dimethylamino-4-phenylcyclohexyl)-N-[3-(1H-indol-3-yl)-propyl]-acetamid; hydrochlorid
2-(4-Dimethylamino-4-phenylcyclohexyl)-N-[5-(1H-indol-3-yl)-pentyl]-acetamid; hydrochlorid
2-(4-Dimethylamino-4-phenylcyclohexyl)-N-[2-(1H-indol-3-yl)-1-methylethyl]-acetamid; hydrochlorid
2-[4-Dimethylamino-4-(4-fluorphenyl)-cyclohexyl]-N-(3-phenylpropyl)-acetamid; hydrochlorid
2-(4-Dimethylamino-4-phenylcyclohexyl)-N-[6-(1H-indol-3-yl)-hexyl]-acetamid; hydrochlorid
2-[4-Dimethylamino-4-(4-fluorphenyl)-cyclohexyl]-N-[2-(1H-indol-3-yl)-ethyl]-acetamid; hydrochlorid
2-[4-Dimethylamino-4-(4-fluorphenyl)-cyclohexyl]-N-[2-(1H-indol-3-yl)-1-methylethyl]-acetamid; hydrochlorid
2-(4-Dimethylamino-4-pyridin-2-yl-cyclohexyl)-N-(3-phenylpropyl)-acetamid; hydrochlorid
2-(4-Dimethylamino-4-pyridin-2-yl-cyclohexyl)-N-[2-(1H-indol-3-yl)-ethyl]-acetamid; hydrochlorid
2-[4-Dimethylamino-4-(3-fluorphenyl)-cyclohexyl]-N-[2-(1H-indol-3-yl)-ethyl]-acetamid; hydrochlorid
2-[4-Dimethylamino-4-(3-fluorphenyl)-cyclohexyl]-N-(3-phenylpropyl)-acetamid; hydrochlorid
2-[4-Dimethylamino-4-(3-fluorphenyl)-cyclohexyl]-N-[2-(1H-indol-3-yl)-1-methylethyl]-acetamid; hydrochlorid
2-(4-Dimethylamino-4-phenyl-cyclohexyl)-N-phenyl-acetamid-hydrochlorid
2-(4-Dimethylamino-4-phenyl-cyclohexyl)-N-(4-fluor-phenyl)-acetamid-hydrochlorid
2-(4-Dimethylamino-4-phenyl-cyclohexyl)-N-p-tolyl-acetamid-hydrochlorid
2-(4-Dimethylamino-4-phenyl-cyclohexyl)-N-(4-methoxy-phenyl)-acetamid-hydrochlorid
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten ORL-1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Cyclohexycarbonsäure-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Cyclohexylessigsäure-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Cyclohexylessigsäure-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Cyclohexylessigsäure-Derivate verzögert freisetzen. Die erfindungsgemäßen substituierten Cyclohexylessigsäure-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Cyclohexylessigsäure-Derivats appliziert.

Für alle vorstehenden Formen der erfindungsgemäßen Arzneimittel ist es besonders bevorzugt, wenn das Arzneimittel neben wenigstens einem substituierten Cyclohexylessigsäure-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes Cyclohexylessigsäure-Derivat als reines Diastereomer und/oder Enantiomer, als Razemat oder als nicht-äquimolare oder äquimolare Mischung der Diastereomere und/oder Enantiomere vor.

Der ORL-1-Rezeptor, aber auch die anderen Opioid-Rezeptoren, wurden insbesondere im Schmerzgeschehen

identifiziert. Entsprechend können erfindungsgemäße substituierte Cyclohexylessigsäure-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen substituierten Cyclohexylessigsäure-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen substituierten Cyclohexylessigsäure-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes Cyclohexylessigsäure-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten Cyclohexylessigsäure-Derivats, oder eines erfindungsgemäßen Arzneimittels.

Ein weiterer Gegenstand der Erfindung sind Verfahren zur Herstellung substituierter Cyclohexylessigsäure-Derivate.

**Allgemeines Syntheseschema:**

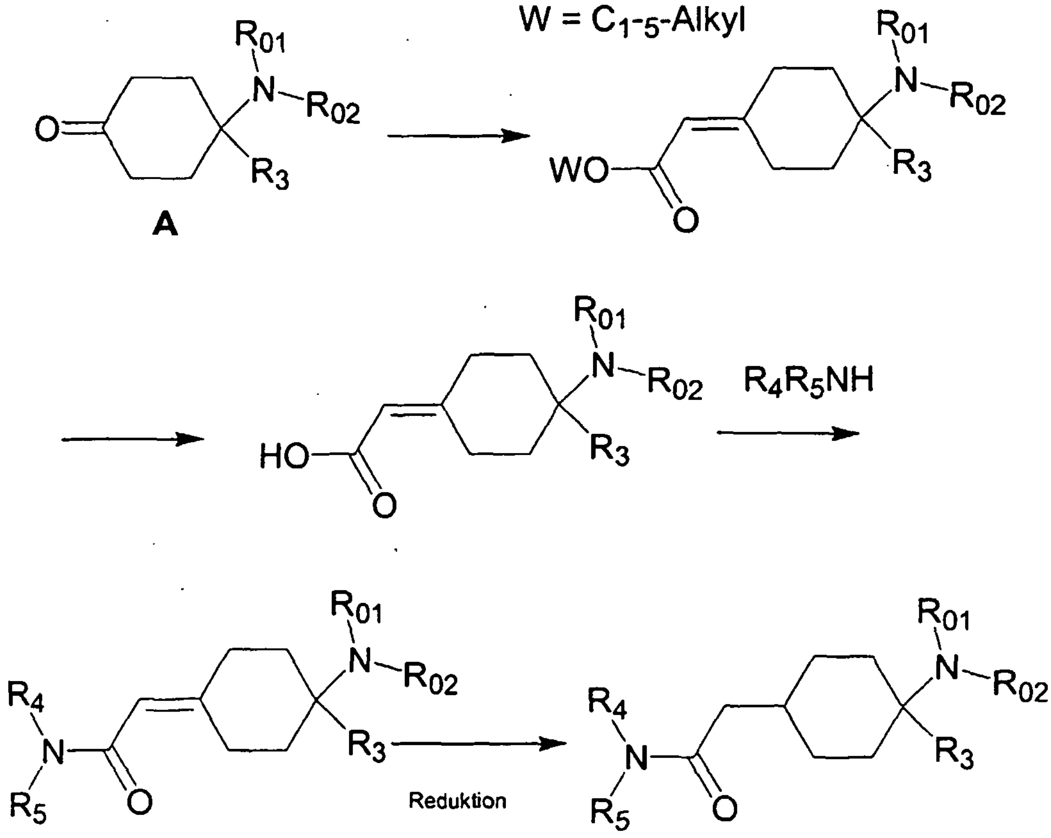


$R^{01}$ und $R^{02}$ haben die Bedeutung von $R^1$ und $R^2$ und können zusätzlich die Bedeutung einer Schutzgruppe annehmen.

Die Herstellung geeigneter 4-Aminocyclohexanone gemäß Formel A ist aus der Literatur bekannt (Lednicer et al., J. Med. Chem., 23, 1980, 424-430; WO 0290317).

Ein Phosphonoessigsäureester, vorzugsweise Phosphonoessigsäure-trimethylester oder Phosphonoessigsäure-triethylester, wird zunächst mit einer starken Base, vorzugsweise Kalium-*tert.*butylat, Natriumhydrid oder Butyllithium, dann mit einem 4-Aminocyclohexanon gemäß Formel A umgesetzt. Dabei entsteht für $R_3 \neq$ 2-Pyridyl der α,β-ungesättigte Ester II, für $R_3$ = 2-Pyridyl der β-Hydroxy-carbonsäureester III.

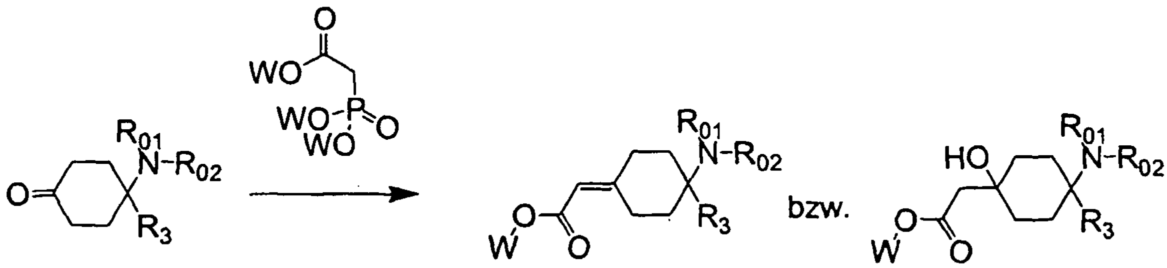


A II III

Die Ester II bzw. III werden mit einer geeigneten wässrigen, basischen Lösung, bevorzugt mit Kaliumhydroxid- oder Lithiumhydroxid-Lösung, bei RT oder leicht erhöhter Temperatur zu den korrespondierenden Carbonsäuren IV bzw. V hydrolysiert.

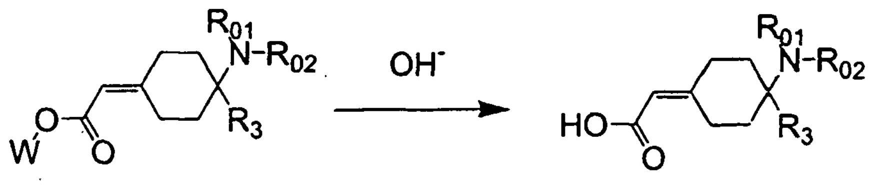


II IV

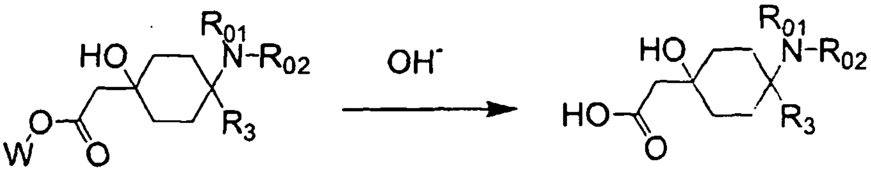


III V

Die Carbonsäuren gemäß Formel IV bzw. V werden als solche oder als ihre korrespondierenden Hydrochloride mit einem wasserentziehenden Reagens, bevorzugt mit einem Carbodiimid, besonders bevorzugt mit Dicyclohexylcarbodiimid, in Gegenwart von einem Aktivierungsreagens, bevorzugt mit 1-Hydroxybenzotriazol, mit einem Amin der Formel $R^4R^5NH$ zu dem korrespondierenden Amid gemäß der Formel Ia umgesetzt.

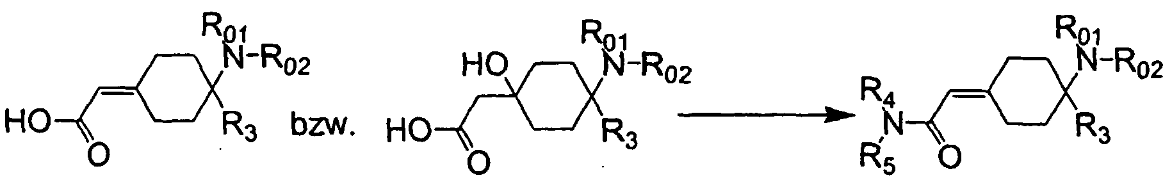

IV bzw. V Ia

Die CC-Doppelbindung eines Cyclohexyliden-acetamid-Derivates gemäß Formel Ia wird gegebenenfalls nach literaturbekannten Methoden reduziert, bevorzugt durch heterogene, katalytische Hydrierung an Palladium- oder Platin-Katalysatoren oder durch homogen katalysierte Hydrierung mit Rhodium-Katalysatoren, jeweils bei Temperaturen zwischen RT und 60°C und unter Wasserstoff-Drücken zwischen 1 bar und 6 bar, besonders bevorzugt bei RT unter einem Wasserstoffdruck zwischen 2 und 3 bar an Palladium auf Kohle, sodass ein Cyclohexylacetamid-Derivat gemäß Formel Ib entsteht.

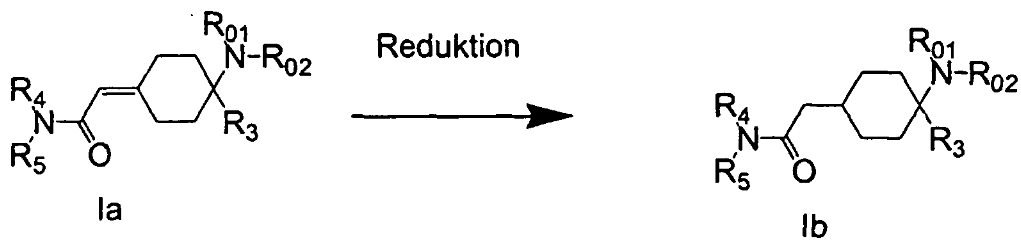


Ia Ib

Die Doppelbindung kann gegebenenfalls auch zu einem anderen Zeitpunkt der Synthese reduziert werden. Hierbei wird nach dem erste Schritt, der Umsetzung mit dem Phosphonoessigsäureester, die Doppelbindung nach literaturbekannten Methoden reduziert, bevorzugt durch heterogene, katalytische Hydrierung an Palladium- oder Platin-Katalysatoren oder durch homogen katalysierte Hydrierung mit Rhodium-Katalysatoren, jeweils bei Temperaturen zwischen RT und 60°C und unter Wasserstoff-Drücken zwischen 1 bar und 6 bar, besonders bevorzugt bei RT unter einem Wasserstoffdruck zwischen 2 und 3 bar an Palladium auf Kohle. Anschließend wird wie oben beschrieben mit der Esterhydrolyse weiterverfahren.

Für den Fall, dass $R^4$ einen Arylrest und $R^5$ H bedeutet kann auch der Ester in Gegenwart einer starken Base, vorzugsweise nBuLi, und einem Anilin direkt zu den erfindungsgemäßen Verbindungen gemäß Formel Ic umgesetzt werden.

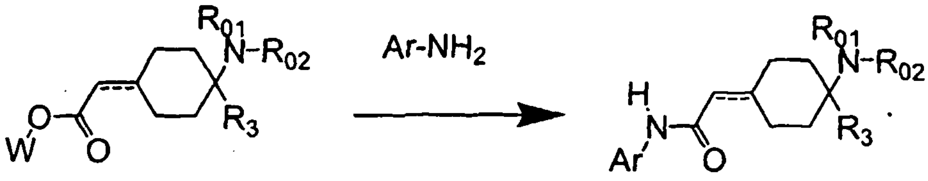


Ic

Gegebenenfalls werden anschließend die Schutzgruppen an $R^{01}$ und $R^{02}$ nach dem Fachmann bekannten Methoden abgespalten.

**Alternativer Syntheseweg :**

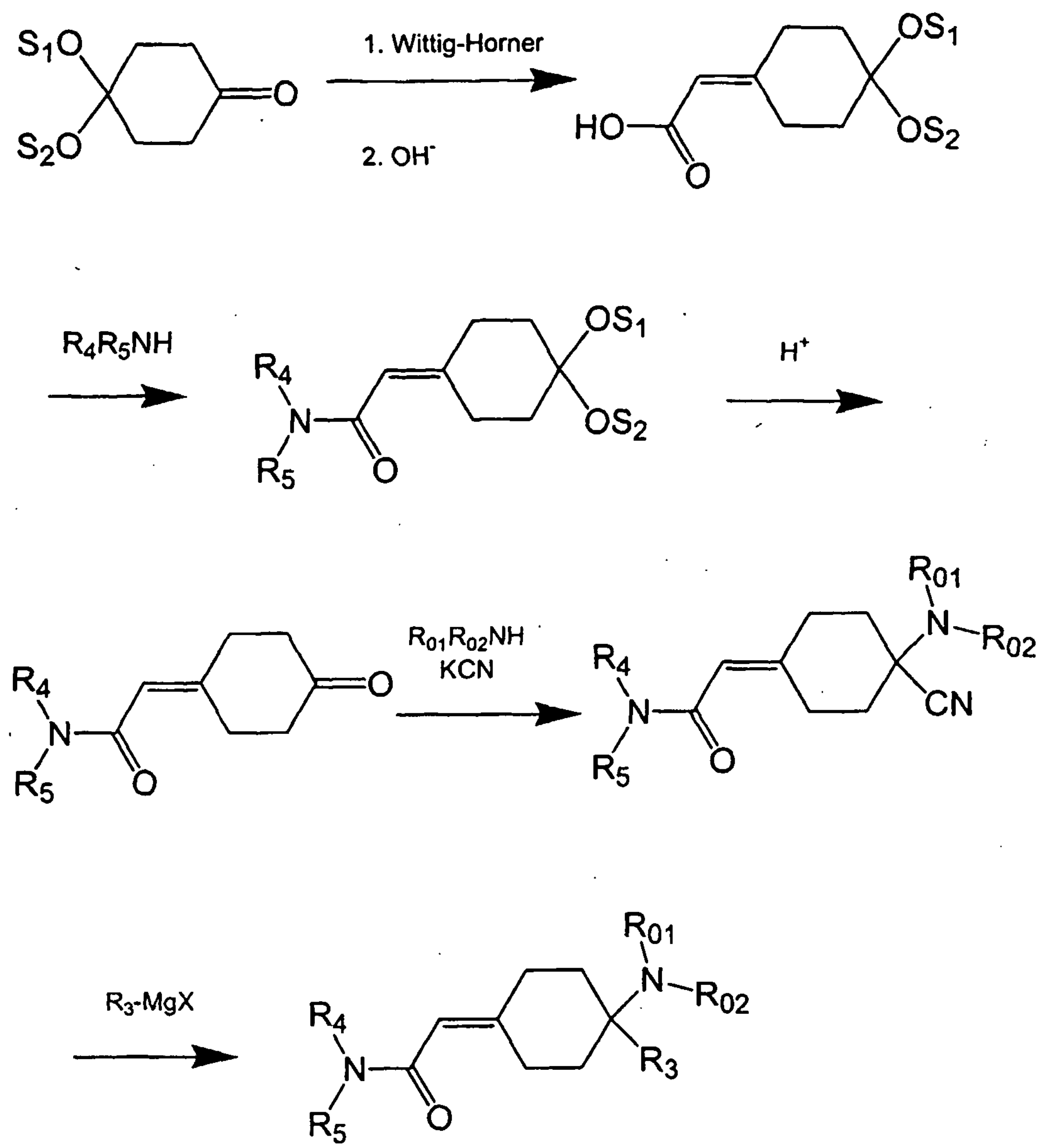


Ein mit den Gruppen $S^1$ und $S^2$, die für Schutzgruppen stehen - beispielsweise substituiertes oder unsubstituiertes Alkyl, insbesondere $(CH_2)_n$ mit n = 2-4 - geschütztes Cyclohexan-1,4-dion gemäß Formel B wird in Gegenwart einer starken Base, vorzugsweise Kalium-*tert.*butylat, Natriumhydrid oder Butyllithium mit einem Phosphonoessigsäureester, vorzugsweise Phosphonoessigsäure-trimethylester oder Phosphonoessigsäure-triethylester, umgesetzt. Dabei entsteht der α,β-ungesättigte Ester VI.

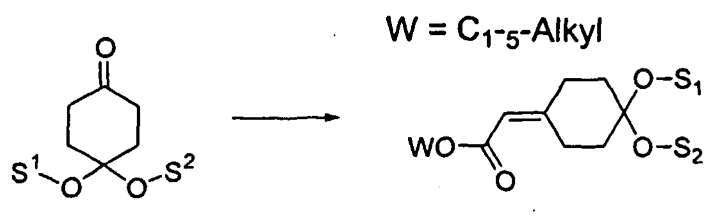


B VI

Der Ester VI wird mit einer geeigneten wässrigen, basischen Lösung, bevorzugt mit Kaliumhydroxid- oder

Lithiumhydroxid-Lösung, bei RT oder leicht erhöhter Temperatur zu der korrespondierenden Carbonsäure VII hydrolysiert.

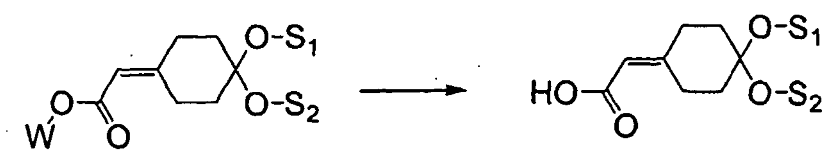

VI VII

Die Carbonsäure gemäß Formel VII wird als solche oder als ihr korrespondierendes Hydrochlorid mit einem wasserentziehenden Reagens, bevorzugt mit einem Carbodiimid, besonders bevorzugt mit Dicyclohexyl-carbodiimid, in Gegenwart von einem Aktivierungsreagens, bevorzugt mit 1-Hydroxybenzotriazol, mit einem Amin der Formel $R^4R^5NH$ zu dem korrespondierenden Amid gemäß der Formel VIII umgesetzt.

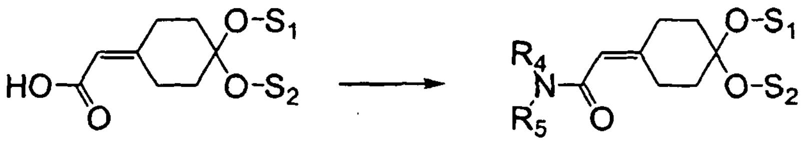

VII VIII

An der Verbindung gemäß Formel VIII werden die Schutzgruppen $S^1$ und $S^2$ abgespalten, so dass ein 4-substituiertes Cyclohexanonderivat gemäß Formel IX entsteht

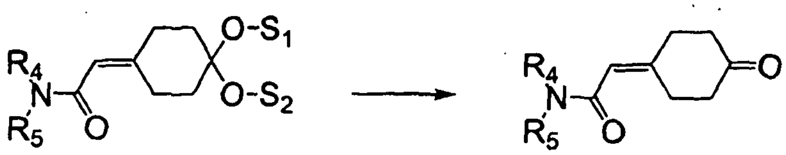

VIII IX

Die Verbindung gemäß Formel IX wird in Gegenwart einer Verbindung der Formel $HNR^{01}R^{02}$ mit einem Cyanid, vorzugsweise Kaliumcyanid oder TMSCN, zu einem 4-substituierten 1-Amino-1-cyano-cyclohexanderivat gemäß Formel X umgesetzt.

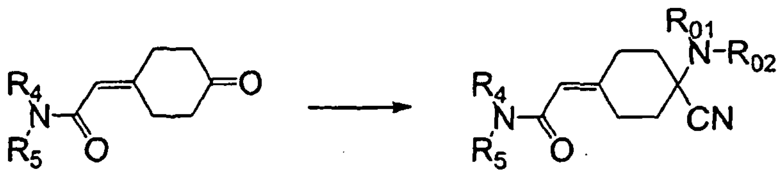

IX X

Das Aminonitril gemäß Formel X wird mit metallorganischen Reagenzien, bevorzugt Grignard- oder Organolithiumreagenzien, der Formel Metall-$R^3$ umgesetzt, so dass die erfindungsgemäßen Verbindungen gemäß Formel Ia entstehen.

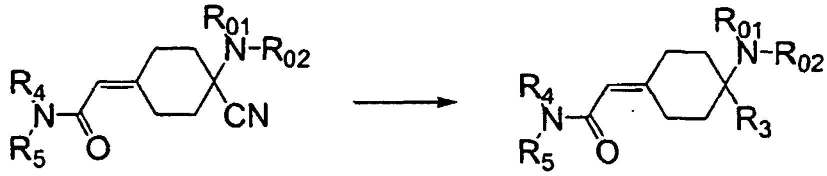

X

Ia

Wie im ersten Syntheseverfahren beschrieben können anschließend Verbindungen der allgemeinen Formel Ia zu Verbindungen der allgemeinen Formel Ib reduziert werden. Alternativ kann in diesem Verfahren die Doppelbindung auch zu einem früheren Zeitpunkt reduziert werden, nämlich Verbindung VIII nach den im ersten Synteseverfahren beschriebenen Methoden. Anschließend wird entsprechend des beschriebenen Verfahrens weiterverfahren. Gegebenenfalls werden anschließend die Schutzgruppen an $R^{01}$ und $R^{02}$ nach dem Fachmann bekannten Methoden abgespalten.

Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), "rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

**(4-Dimethylamino-4-phenylcyclohexyliden)essigsäure-methylester**

Zu einer Lösung von 4-Dimethylamino-4-phenyl-cyclohexanon (2,0 g, 9,2 mmol) in wasserfreiem DMF (60 ml) wurde Phosphonoessigsäuretrimethylester (1,99 ml, 13,8 mmol) unter Argon hinzugefügt. Es wurde Kalium-*tert.*-butylat (1,54 g, 13,8 mmol) zugesetzt. Das Reaktionsgemisch wurde 4 h auf 60 °C erwärmt und nach dem Abkühlen auf Eis (40 g) gegossen. Nach der Zugabe von Ether (150 ml) erfolgte Phasentrennung und die wässrige Phase wurde nochmals mit Ether (2 × 50 ml) extrahiert. Die vereinigten organischen Phasen wurden getrocknet und eingeengt. Der Methylester wurde dabei als gelbes Öl in einer Rohausbeute von 97 % (2,42 g) erhalten.

**(4-Dimethylamino-4-phenylcyclohexyliden)essigsäure-methylesterhydrochlorid**

(4-Dimethylamino-4-phenylcyclohexyliden)essigsäure-methylester (2,4 g, 8,7 mmol) wurde in Ethylmethylketon (70 ml) gelöst und unter Eiskühlung mit Chlortrimethylsilan (1,66 ml, 13,17 mmol) versetzt. Nach einer Reaktionszeit von 5 h konnte das Hydrochlorid des Esters als farbloser Feststoff in einer Ausbeute von 78 % (1,95 g) mit einem Smp. von 204-208 °C erhalten werden.

**(4-Dimethylamino-4-phenylcyclohexyliden)essigsäure-hydrochlorid**

Zu einer Lösung von (4-Dimethylamino-4-phenylcyclohexyliden)essigsäuremethylester-hydrochlorid (1,8 g, 5,8 mmol) in Ethanol (200 ml) wurde 1,7M KOH (70,5 ml, 120 mmol) hinzugefügt und 16 h bei RT gerührt. Die Reaktionsmischung wurde eingeengt und der Rückstand in Wasser (40 ml) aufgenommen. Die wässrige Phase wurde mit Ether (2 × 40 ml) ausgeschüttelt und mit 5,5M Salzsäure (23,6 ml, 130 mmol) versetzt. Nach dem Einengen der wässrigen Phase wurde der Rückstand mit Ethanol (2 × 40 ml) extrahiert, das zurückbleibende Kaliumchlorid abgetrennt und das Filtrat eingeengt. Dabei wurde das Hydrochlorid der Säure in einer Ausbeute von 98 % (1,67 g) als farbloser Feststoff erhalten.

**2-(Dimethylamino-4-phenylcyclohexyliden)-*N*-[2-(1*H*-indol-3-yl)ethyl]acetamid**

Zu einer Lösung von(4-Dimethylamino-4-phenylcyclohexyliden)essigsäurehydrochlorid (200 mg, 0,676 mmol) in trockenem Dimethylformamid (10 ml) wurde unter Argon 1-Hydroxybenzotriazol (182 mg, 1,35 mmol), Tryptamin (108 mg, 0,676 mmol) und *N*-Methylmorpholin (0,148 ml, 1,35 mmol) gegeben. Die klare Lösung wurde auf 0°C abgekühlt und mit Dicyclohexylcarbodiimid (278 mg, 1,35 mmol) versetzt. Die Reaktionsmischung wurde 3 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das erhaltene Filtrat wurde mit Ether extrahiert. Die wässrige Phase wurde mit 5M Natronlauge (2 ml, 10 mmol) versetzt und mit Wasser verdünnt (150 ml). Dabei fiel das Produkt als farbloser Feststoff in einer Ausbeute von 31 % (84 mg) aus.

**2-(Dimethylamino-4-phenylcyclohexyliden)-*N*-[2-(1*H*-indol-3-yl)ethyl]acetamidhydrochlorid (Beispiel 1)**

Die Lösung von 2-(Dimethylamino-4-phenylcyclohexyliden)-*N*-[2-(1*H*-indol-3-yl)ethyl]acetamid (75 mg, 0,178 mmol) in Ethylmethylketon (15 ml) wurde mit Chlortrimethylsilan (0,034 ml, 0,26 mmol) versetzt. Nach 2 h konnte das Hydrochlorid als beigefarbener Feststoff in einer Ausbeute von 75 % (61 mg) mit einem Smp. von 203-207 °C isoliert werden.

**2-[2-(4-Dimethylamino-4-phenylcyclohexyliden)acetylamino]-3-(1*H*-indol-3-yl)propansäure-methylester**

Zu einer Lösung von(4-Dimethylamino-4-phenylcyclohexyliden)essigsäurehydrochlorid (200 mg, 0,676 mmol) in Dimethylformamid (10 ml) wurde unter Argon 1-Hydroxybenzotriazol (182 mg, 1,35 mmol), L-Tryptophanmethylester-hydrochlorid (172 mg, 0,676 mmol) und *N*-Methyl-morpholin (0,148 ml, 1,35 mmol) gegeben. Die Lösung wurde auf 0°C abgekühlt und mit Dicyclohexylcarbodiimid (278 mg, 1,35 mmol) versetzt. Die Reaktionsmischung wurde 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das erhaltene Filtrat wurde mit Ether extrahiert. Die organische Phase wurde eingeengt, wobei das Rohprodukt als gelbes Öl (142 mg) erhalten wurde.

**2-[2-(4-Dimethylamino-4-phenylcyclohexyliden)acetylamino]-3-(1*H*-indol-3-yl)propansäure-methylester-hydrochlorid (unpolareres Diastereoisomer; Beispiel 2)**

2-[2-(4-Dimethylamino-4-phenylcyclohexyliden)acetylamino]-3-(1*H*-indol-3-yl)propansäure-methylester (142 mg, 0,3 mmol) wurde in Ethylmethylketon (9 ml) gelöst und mit Chlortrimethylsilan (0,055 ml, 0,43 mmol) versetzt. Nach 2 h konnte das Produkt als farblose Verbindung in einer Ausbeute von 50 mg (15 %) mit einem Smp. von 164-168 °C erhalten werden.

**(4-Dimethylamino-4-phenylcyclohexyliden)essigsäure-2,5-dioxo-pyrrolidin-1-ylester**

(4-Dimethylamino-4-phenylcyclohexyliden)essigsäure-hydrochlorid (200 mg, 0,676 mmol) wurde in Dichlormethan (10 ml) gelöst und mit Triethylamin (0,28 ml, 2,0 mmol) und *N,N'* Di(succinimidyl)carbonat (345 mg, 1,35 mmol) unter Argon versetzt. Nach einer Reaktionszeit von 24 h wurde die Reaktionsmischung eingeengt, in Ether (20 ml) aufgenommen und mit gesättigter $NaHCO_3$-Lösung (2 × 5 ml) extrahiert. Nach dem Trocknen und Einengen der organischen Phase wurde das Produkt in einer Ausbeute von 52 % (124 mg) als farbloses Öl erhalten.

**(2*S*)-2-[(*RS*)-2-(4-Dimethylamino-4-phenylcyclohexyliden)acetylamino]-3-(1*H*-indol-3-yl)propansäure-methylester**

Zu einer Lösung von (4-Dimethylamino-4-phenylcyclohexyliden)essigsäure-2,5-dioxo-pyrrolidin-1-ylester (104 mg, 0,29 mmol) in Acetonitril (10 ml) wurde unter Argon zunächst der L-Tryptophanmethylester (3, 74 mg, 0,29 mmol) und anschließend Triethylamin (0,071 ml, 0,5 mmol) hinzugegeben. Die Reaktionsmischung wurde 2 d bei RT gerührt,

dann eingeengt und in Ether (30 ml) aufgenommen. Die organische Phase wurde mit gesättigter $NaHCO_3$-Lösung ausgeschüttelt (2 × 10 ml) und nach dem Trocknen eingeengt. Das Rohprodukt wurde dabei als farbloses Öl in einer Ausbeute von 92 mg (69 %) erhalten.

**(2*S*)-2-[(*RS*)-2-(4-Dimethylamino-4-phenylcyclohexyliden)acetylamino]-3-(1*H*-indol-3-yl)propansäure-methylester-hydrochlorid (polareres Diastereoisomer;**

**Beispiel 3)**

Zu einer Lösung von (2*S*)-2-[(*RS*)-2-(4-Dimethylamino-4-phenylcyclohexyliden)-acetylamino]-3-(1*H*-indol-3-yl) propansäure-methylester (92 mg, 0,2 mmol) in Ethylmethylketon (3 ml) wurde Chlortrimethylsilan (0,038 ml, 0,3 mmol) hinzugefügt und 2 h bei RT gerührt. Dabei fiel das Produkt (43 mg, 43 %) mit einem Smp. von 193-195 °C als farbloser Feststoff aus. Die Substanz erwies sich als das polarere von zwei möglichen Diastereoisomeren.

**2-(4-Dimethylamino-4-phenylcyclohexyliden)-*N*-[2-(1*H*-indol-3-yl)-1-methylethyl]acetamid**

Zu einer Lösung von(4-Dimethylamino-4-phenylcyclohexyliden)essigsäurehydrochlorid (500 mg, 1,69 mmol) in trockenem Dimethylformamid (10 ml) wurden unter Argon 1-Hydroxybenzotriazol (456 mg, 3,38 mmol), DL-α-Methyltryptamin (0,142 ml, 1,69 mmol) und *N*-Methylmorpholin (0,375 ml, 3,38 mmol) gegeben. Die Lösung wurde auf 0°C abgekühlt und mit Dicyclohexylcarbodiimid (687 mg, 3,38 mmol) versetzt. Die Reaktionsmischung wurde 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das Filtrat wurde mit 5M Natronlauge (2 ml, 10 mmol) versetzt und mit Wasser verdünnt (200 ml). Dabei fiel das Produkt als beigefarbener Feststoff aus und konnte in einer Ausbeute von 83 % (581 mg) erhalten werden. Die chromatographische Trennung an Kieselgel erfolgte mit EE/Methanol (15 : 1), (10 : 1) und (5 : 1). Das unpolarere Diastereoisomer von 2-(4-Dimethylamino-4-phenylcyclohexyliden)-*N*-[2-(1*H*-indol-3-yl)-1-methylethyl]acetamid wurde dabei in einer Ausbeute von 24 % (165 mg) als farbloser Feststoff mit einem Smp. von 184-187 °C erhalten. Das polarere Diastereoisomer von 2-(4-Dimethylamino-4-phenylcyclohexyliden)-*N*-[2-(1*H*-indol-3-yl)-1-methylethyl]acetamid konnte in einer Ausbeute von 18 % (126 mg) als farbloses Öl isoliert werden. - Beide Amide waren diastereoisomerenrein.

**2-(4-Dimethylamino-4-phenylcyclohexyliden)-*N*-[2-(1*H*-indol-3-yl)-1-methylethyl]acetamid-hydrochlorid (Beispiele 4 und 5)**

Das unpolarere Diastereoisomer von 2-(4-Dimethylamino-4-phenylcyclohexyliden)-*N*-[2-(1*H*-indol-3-yl)-1-methylethyl]acetamid (156 mg, 0,397 mmol) wurde unter leichtem Erwärmen in Ethylmethylketon (11 ml) gelöst und mit Chlortrimethylsilan (0,075 ml, 0,59 mmol) versetzt. Nach 1,5 h konnte das Hydrochlorid (**Beispiel 4**) als farbloser Feststoff in einer Ausbeute von 55 % (96 mg) mit einem Smp. von 175-180 °C isoliert werden.

Zu einer Lösung des polareren Diastereoisomers von 2-(4-Dimethylamino-4-phenylcyclohexyliden)-*N*-[2-(1*H*-indol-3-yl)-1-methylethyl]acetamid (133 mg, 0,32 mmol) in Ethylmethylketon (5 ml) wurde Chlortrimethylsilan (0,06 ml, 0,48 mmol) hinzugefügt und 1,5 h bei RT gerührt. Dabei fiel das Hydrochlorid (**Beispiel 5**) (66 mg, 46 %) mit einem Smp. von 182-186 °C als farbloser Feststoff aus.

**2-(4-Dimethylamino-4-phenylcyclohexyliden)-*N*-(3-phenylpropyl)acetamid**

Zu einer Lösung von (4-Dimethylamino-4-phenylcyclohexyliden)essigsäurehydrochlorid (300 mg, 1,0 mmol) in trockenem Dimethylformamid (10 ml) wurden unter Argon 1-Hydroxybenzotriazol (273 mg, 2,0 mmol), 3-Phenyl-1-propylamin (0,142 ml , 1,0 mmol) und N-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 3 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das erhaltene Filtrat wurde mit Ether extrahiert. Die wässrige Phase wurde mit 5M Natronlauge (2 ml, 10 mmol) versetzt und mit Wasser verdünnt (200 ml). Dabei fiel das Produkt als farbloser Feststoff aus und konnte in einer Ausbeute von 55 % (209 mg) mit einem Smp. von 140-142 °C erhalten werden.

**2-(4-Dimethylamino-4-phenylcyclohexyliden)-*N*-(3-phenylpropyl)acetamidhydrochlorid (Beispiel 6)**

2-(4-Dimethylamino-4-phenylcyclohexyliden)-*N*-(3-phenylpropyl)acetamid (203 mg, 0,536 mmol) wurde unter

leichtem Erwärmen in Ethylmethylketon (12 ml) gelöst und mit Chlortrimethylsilan (0,101 ml, 0,8 mmol) versetzt. Nach 2 h konnte das Produkt als farbloser Feststoff in einer Ausbeute von 95 % (211 mg) mit einem Smp. von 225-230 °C isoliert werden.

**2-(4-Dimethylamino-4-phenylcyclohexyliden)-N-phenethylacetamid**

Zu einer Lösung von (4-Dimethylamino-4-phenylcyclohexyliden)essigsäurehydrochlorid (296 mg, 1,0 mmol) in trockenem Dimethylformamid (10 ml) wurden unter Argon 1-Hydroxybenzotriazol (273 mg, 2,0 mmol), 2-Phenylethylamin (0,125 ml, 1,0 mmol) und N-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 10 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 3 d bei 5 °C aufbewahrt. Dabei fiel das Produkt als farbloser Feststoff in einer Ausbeute von 62 % (223 mg) und mit einem Smp. von 153-155 °C aus.

**2-(4-Dimethylamino-4-phenylcyclohexyliden)-*N*-phenethylacetamidhydrochlorid (Beispiel 7)**

2-(4-Dimethylamino-4-phenylcyclohexyliden)-*N*- phenethylacetamid (225 mg, 0,62 mmol) wurde unter leichtem Erwärmen in Ethylmethylketon (12 ml) gelöst und mit Chlortrimethylsilan (0,118 ml, 0,93 mmol) versetzt. Nach 2 h wurde das Produkt als farbloser Feststoff in einer Ausbeute von 86 % (213 mg) isoliert.

***N*-Benzyl-2-(4-dimethylamino-4-phenylcyclohexyliden)acetamid**

Zu einer Lösung von (4-Dimethylamino-4-phenylcyclohexyliden)essigsäurehydrochlorid (296 mg, 1,0 mmol) in trockenem Dimethylformamid (10 ml) wurden unter Argon 1-Hydroxybenzotriazol (273 mg, 2,0 mmol), Benzylamin (0,109 ml, 1,0 mmol) und *N*-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 12 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 3 d bei 5 °C aufbewahrt. Dabei fiel das Produkt als farbloser Feststoff in einer Ausbeute von 65 % (227 mg) und mit einem Smp. von 145-148 °C aus.

***N*-Benzyl-2-(4-dimethylamino-4-phenylcyclohexyliden)acetamid-hydrochlorid**

**(Beispiel 8)**

*N*-Benzyl-2-(4-dimethylamino-4-phenylcyclohexyliden)acetamid (206 mg, 0,59 mmol) wurde in Ethylmethylketon (12 ml) gelöst und mit Chlortrimethylsilan (0,112 ml, 0,88 mmol) versetzt. Nach 2 h wurde das Hydrochlorid **(Beispiel 8)** als farbloser Feststoff mit einem Smp. von 220-226 °C und einer Ausbeute von 99 % (225 mg) isoliert.

**2-(4-Dimethylamino-4-phenylcyclohexyliden)-*N*-(4-phenylbutyl)acetamid**

Zu einer Lösung von (4-Dimethylamino-4-phenylcyclohexyliden)essigsäurehydrochlorid (296 mg, 1,0 mmol) in trockenem Dimethylformamid (10 ml) wurden unter Argon 1-Hydroxybenzotriazol (273 mg, 2,0 mmol), 4-Phenylbutylamin (0,158 ml, 1,0 mmol) und *N*-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 5 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 16 h bei 5 °C aufbewahrt. Dabei fiel das Produkt in einer Ausbeute von 75 % (294 mg) als farblose ölige Verbindung an.

**2-(4-Dimethylamino-4-phenylcyclohexyliden)-*N*-(4-phenylbutyl)acetamidhydrochlorid (Beispiel 9)**

2-(4-Dimethylamino-4-phenylcyclohexyliden)-*N*-(4-phenylbutyl)acetamid (292 mg, 0,747 mmol) wurde in Ethylmethylketon (5 ml) gelöst und mit Chlortrimethylsilan (0,14 ml, 1,12 mmol) versetzt. Nach 1,5 h wurde das Hydrochlorid

als farbloser Feststoff in einer Ausbeute von 69 % (221 mg) isoliert.

**2-(4-Dimethylamino-4-phenylcyclohexyliden)-*N*-(4-fluorphenyl)acetamid**

Zu einer Lösung von (4-Dimethylamino-4-phenylcyclohexyliden)essigsäurehydrochlorid (296 mg, 1,0 mmol) in trockenem Dimethylformamid (10 ml) wurden unter Argon 1-Hydroxybenzotriazol (273 mg, 2,0 mmol), 4-Fluoranilin (0,095 ml, 1,0 mmol) und *N*-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 7 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 3 d bei 5 °C aufbewahrt. Dabei fiel das Produkt als farbloser Feststoff in einer Ausbeute von 82 % (287 mg) aus.

**2-(4-Dimethylamino-4-phenylcyclohexyliden)-*N*-(4-fluorphenyl)acetamidhydrochlorid (Beispiel 10)**

2-(4-Dimethylamino-4-phenylcyclohexyliden)-*N*-(4-fluorphenyl)acetamid (266 mg, 0,755 mmol) wurde in Ethylmethylketon (5 ml) gelöst und mit Chlortrimethylsilan (0,139 ml, 1,1 mmol) versetzt. Nach 2,5 h wurde das Hydrochlorid als farbloser Feststoff in einer Ausbeute von 52 % (152 mg) isoliert.

**2-(4-Dimethylamino-4-phenylcyclohexyliden)-*N*-(4-fluorbenzyl)acetamid**

Zu einer Lösung von (4-Dimethylamino-4-phenylcyclohexyliden)essigsäurehydrochlorid (296 mg, 1,0 mmol) in trockenem Dimethylformamid (10 ml) wurden unter Argon 1-Hydroxybenzotriazol (273 mg, 2,0 mmol), 4-Fluorbenzylamin (0,114 ml, 1,0 mmol) und *N*-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 12 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 16 h bei 5 °C aufbewahrt. Dabei fiel das Produkt als farbloser Feststoff in einer Ausbeute von 75 % (275 mg) und mit einem Smp. von 149-151 °C aus.

**2-(4-Dimethylamino-4-phenylcyclohexyliden)-*N*-(4-fluorbenzyl)acetamidhydrochlorid (Beispiel 11)**

2-(4-Dimethylamino-4-phenylcyclohexyliden)-*N*-(4-fluorbenzyl)acetamid (252 mg, 0,688 mmol) wurde unter leichtem Erwärmen in Ethylmethylketon (10 ml) gelöst und mit Chlortrimethylsilan (0,126 ml, 1,0 mmol) versetzt. Nach 2,5 h wurde das Hydrochlorid als farlboser Feststoff mit einem Smp. von 214-218 °C und einer Ausbeute von 71 % (196 mg) isoliert.

**2-(4-Dimethylamino-4-phenylcyclohexyliden)-*N*-[2-(4-fluorphenyl)ethyl]acetamid**

Zu einer Lösung von (4-Dimethylamino-4-phenylcyclohexyliden)essigsäurehydrochlorid (296) mg, 1,0 mmol) in trockenem Dimethylformamid (10 ml) wurden unter Argon 1-Hydroxybenzotriazol (273 mg, 2,0 mmol), 2-(4-Fluorphenyl)ethylamin (0,131 ml, 1,0 mmol) und *N*-Methylmorpholin (0,222 ml, 2,0 mmol) gegeben. Die Lösung wurde auf 0°C abgekühlt und mit Dicyclohexylcarbodiimid (417 mg, 2,0 mmol) versetzt. Die Reaktionsmischung wurde 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösun (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 16 h bei 5 °C aufbewahrt. Dabei fiel das Produkt in einer Ausbeute von 66 % (252 mg) als farblose ölige Verbindung an.

**2-(4-Dimethylamino-4-phenylcyclohexyliden)-*N*-[2-(4-fluorphenyl)-ethyl]acetamid-hydrochlorid (Beispiel 12)**

2-(4-Dimethylamino-4-phenylcyclohexyliden)-*N*-[2-(4-fluorphenyl)-ethyl]acetamid (252 mg, 0,66 mmol) wurde in Ethylmethylketon (5 ml) gelöst und mit Chlortrimethylsilan (0,126 ml, 1,0 mmol) versetzt. Nach 2 h wurde das Hydrochlorid als farbloser Feststoff mit einem Smp. von 157-160 °C und einer Ausbeute von 74 % (203 mg) isoliert.

**2-(4-Dimethylamino-4-phenylcyclohexyliden)-*N*-(2-trifluormethylbenzyl)acetamid**

Zu einer Lösung von (4-Dimethylamino-4-phenylcyclohexyliden)essigsäurehydrochlorid (475 mg, 1,6 mmol) in trockenem Dimethylformamid (10 ml) wurden unter Argon 1-Hydroxybenzotriazol (432 mg, 3,2 mmol), 2-(Trifluormethyl)benzylamin (0,225 ml, 1,6 mmol) und *N*-Methylmorpholin (0,355 ml, 3,2 mmol) gegeben. Die Lösung wurde auf 0°C abgekühlt und mit Dicyclohexylcarbodiimid (660 mg, 3,2 mmol) versetzt. Die Reaktionsmischung wurde 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 4 d bei 5 °C aufbewahrt. Dabei fiel das Produkt als beigefarbener Feststoff in einer Ausbeute von 70 % (465 mg) aus.

**2-(4-Dimethylamino-4-phenylcyclohexyliden)-*N*-(2-trifluormethylbenzyl)acetamid-hydrochlorid (Beispiel 13)**

2-(4-Dimethylamino-4-phenylcyclohexyliden)-*N*-(2-trifluormethyl-benzyl)acetamid (432 mg, 1,03 mmol) wurde in Ethylmethylketon (7 ml) gelöst und mit Chlortrimethylsilan (0,195 ml, 1,5 mmol) versetzt. Nach 2 h wurde das Hydrochlorid als farbloser Feststoff mit einem Smp. von 133-136 °C und einer Ausbeute von 89 % (415 mg) isoliert.

**2-(4-Dimethylamino-4-phenylcyclohexyliden)-N-(1H-indol-3-ylmethyl)acetamid**

Zu einer Lösung von (4-Dimethylamino-4-phenylcyclohexyliden)essigsäurehydrochlorid (499 mg, 1,7 mmol) in trockenem Dimethylformamid (10 ml) wurden unter Argon 1-Hydroxybenzotriazol (459 mg, 3,4 mmol), C-(1H-Indol-3-yl)methylamin (2, 249 mg, 1,7 mmol) und *N*-Methylmorpholin (0,377 ml, 3,4 mmol) gegeben. Die Lösung wurde auf 0°C abgekühlt und mit Dicyclohexylcarbodiimid (708 mg, 3,4 mmol) versetzt. Die Reaktionsmischung wurde 5 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (4 ml, 20 mmol) versetzt, mit Wasser verdünnt (300 ml) und 16 h bei 5 °C aufbewahrt. Dabei fiel das Rohprodukt aus (253 mg), der durch Chromatographie an Kieselgel mit EE/Methanol (4 : 1) und Methanol gereinigt wurde. Das Produkt wurde als ölige Verbindung in einer Ausbeute von 18 % (120 mg) erhalten.

**2-(4-Dimethylamino-4-phenylcyclohexyliden)-*N*-(1H-indol-3-ylmethyt)acetamidhydrochlorid (Beispiel 14)**

2-(4-Dimethylamino-4-phenylcyclohexyliden)-*N*-(1H-indol-3-ylmethyl)acetamid (120 mg, 0,309 mmol) wurde in Ethylmethylketon (5 ml) gelöst und mit Chlortrimethylsilan (0,058 ml, 0,46 mmol) versetzt. Nach 1,5 h wurde das Hydrochlorid mit einem Smp. von 173-185 °C in einer Ausbeute von 63 % (82 mg) isoliert.

**2-(4-Dimethylamino-4-phenylcyclohexyliden)-*N*-[4-(1H- indol- 3-yl) bu**tyl]acetamid

Zu einer Lösung von (4-Dimethylamino-4-phenylcyclohexyliden)essigsäurehydrochlorid (592 mg, 2,0 mmol) in trockenem Dimethylformamid (10 ml) wurden unter Argon 1-Hydroxybenzotriazol (546 mg, 4,0 mmol), 4-(1H-Indol-3-yl)butylamin (2, 376 mg, 2,0 mmol) und *N*-Methylmorpholin (0,444 ml, 4,0 mmol) gegeben. Die klare Lösung wurde auf 0°C abgekühlt und mit Dicyclohexylcarbodiimid (834 mg, 4,0 mmol) versetzt. Die Reaktionsmischung wurde 4 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit 5M Natronlauge (5 ml, 25 mmol) versetzt, mit Wasser verdünnt (300 ml) und 16 h bei 5 °C aufbewahrt. Dabei fiel das Produkt als farbloser Feststoff in einer Ausbeute von 45 % (387 mg) mit einem Smp. von 77-88 °C aus.

**2-(4-Dimethylamino-4-phenylcyclohexyliden)-*N*-[4-(1H-indol-3-yl)butyl]-acetamid-hydrochlorid (Beispiel 15)**

2-(4-Dimethylamino-4-phenylcyclohexyliden)-*N*-[4-(1*H*-indol-3-yl)butyl]acetamid (184 mg, 0,428 mmol) wurde in Ethylmethylketon (10 ml) gelöst und mit Chlortrimethylsilan (0,08 ml, 0,64 mmol) versetzt. Nach 1,5 h wurde das Hydrochlorid 4 als farbloser Feststoff mit einem Smp. von 185-188 °C in einer Ausbeute von 74 % (147 mg) isoliert.

**2-(4-Dimethylamino-4-phenylcyclohexyliden)-*N*-[3-(1*H*-indol-3-yl)-propyl]acetamid**

Zu einer Lösung von (4-Dimethylamino-4-phenylcyclohexyliden)essigsäurehydrochlorid (887 mg, 3,0 mmol)

in trockenem Dimethylformamid (10 ml) wurden unter Argon 1-Hydroxybenzotriazol (819 mg, 6,0 mmol), 3-(1*H*-Indol-3-yl)propylamin (522 mg, 3,0 mmol) und *N*-Methylmorpholin (0,666 ml, 6,0 mmol) gegeben. Die Lösung wurde auf 0°C abgekühlt und mit Dicyclohexylcarbodiimid (1,25 g, 6,0 mmol) versetzt. Die Reaktionsmischung wurde 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser verdünnt (400 ml), mit 5M Natronlauge (5 ml, 25 mmol) versetzt und 3 d bei 5 °C aufbewahrt. Dabei fiel das Produkt als beigefarbener Feststoff in einer Ausbeute von 60 % (742 mg) mit einem Smp. von 85-87 °C aus.

**2-(4-Dimethylamino-4-phenylcyclohexyliden)-*N*-[3-(1*H*-indol-3-yl)-propyl]acetamid-hydrochlorid (Beispiel 16)**

2-(4-Dimethylamino-4-phenylcyclohexyliden)-*N*-[3-(1*H*-indol-3-yl)-propyl]acetamid (742 mg, 1,78 mmol) wurde in Ethylmethylketon (25 ml) gelöst und mit Chlortrimethylsilan (0,34 ml, 2,7 mmol) versetzt. Nach 2 h wurde das Hydrochlorid als beigefarbener Feststoff mit einem Smp. von 165-173 °C in einer Ausbeute von 75 % (606 mg) isoliert.

**5-(1*H*-Indol-3-yl)pentansäurecyanmethylester**

5-(1H-Indol-3-yl)-pentansäure (5 g, 23 mmol) wurde in einem Gemisch aus Aceton (25 ml) und Dimethylformamid (25 ml) gelöst und nacheinander mit Caesiumcarbonat (3,75 g, 11,5 mmol), Chloracetonitril (2,16 ml, 34,5 mmol) und Kaliumiodid (20 mg) versetzt. Nach 6,5 h bei 60 °C und 16 h bei RT wurden die festen Rückstände abfiltriert, mit Aceton (2 × 30 ml) gewaschen und das Filtrat eingeengt. Das Rohprodukt wurde chromatographisch an Kieselgel (130 g) mit EE/Cyclohexan (1 : 3) gereinigt. Das Produkt wurde als farbloser Feststoff mit einem Smp. von 76°C in einer Ausbeute von 77 % (4,53 g) erhalten.

**5-(1H-Indol-3-yl)pentansäureamid**

Eine Lösung von 5-(1*H*-Indol-3-yl)pentansäurecyanmethylester (4,5 g, 17,5 mmol) in Tetrahydrofuran (110 ml) wurde zu einer 25-proz. Ammoniaklösung (80 ml) gegeben und 3 d bei Raumtemperatur gerührt. Die Aufarbeitung des Ansatzes erfolgte durch Phasentrennung und Extraktion der wässrigen Phase mit Tetrahydrofuran (2 × 30 ml). Die organischen Phasen wurden vereinigt, mit gesättigter Natriumchloridlösung (50 ml) gewaschen, getrocknet und eingeengt. Der Rückstand wurde mit Wasser (2 × 10 ml) und Ether (2 × 10 ml) gewaschen und getrocknet. Das Produkt blieb als farbloser Feststoff in einer Ausbeute von 68 % (2,56 g) mit einem Smp. von 134-139 °C zurück.

**5-(1*H*-Indol-3-yl)pentylamin**

Lithiumaluminiumhydrid (0,87 g, 23,0 mmol) wurde unter Argon in abs. Tetrahydrofuran (70 ml) gegeben. Eine Lösung von 5-(1*H*-Indol-3-yl)pentansäureamid (2,5 g, 11,5 mmol) in abs. Tetrahydrofuran (60 ml) wurde bei 60°C der $LiAlH_4$-Suspension hinzugefügt. Nach 12 h bei 60°C wurde der Ansatz mit Tetrahydrofuran (30 ml) versetzt und unter Eiskühlung hydrolysiert. Die dabei erhaltenen Aluminiumverbindungen wurden durch Filtration abgetrennt und mit Tetrahydrofuran (3 × 10 ml) gewaschen. Das Filtrat wurde eingeengt. Nach der Zugabe von Wasser (30 ml) wurde das Amin mit EE (3 × 40 ml) extrahiert, die Extrakte vereinigt und mit Wasser (40 ml) gewaschen. Nach dem Trocknen und Einengen der organischen Phase wurde das Produkt als farbloser Feststoff in einer Ausbeute von 95 % (2,21 g) mit einem Smp. von 69-78 °C erhalten.

**2-(4-Dimethylamino-4-phenylcyclohexyliden)-*N*-[5-(1*H*-indol-3-yl)-pentyl]acetamid**

Zu einer Lösung von (4-Dimethylamino-4-phenylcyclohexyliden)essigsäurehydrochlorid (887 mg, 3,0 mmol) in trockenem Dimethylformamid (10 ml) wurden unter Argon 1-Hydroxybenzotriazol (819 mg, 6,0 mmol), 5-(1*H*-Indol-3-yl)pentylamin (606 mg, 3,0 mmol) und *N*-Methylmorpholin (0,666 ml, 6,0 mmol) gegeben. Die Lösung wurde auf 0°C abgekühlt und mit Dicyclohexylcarbodiimid (1,25 g, 6,0 mmol) versetzt. Die Reaktionsmischung wurde 4 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser verdünnt (300 ml), mit 5M Natronlauge (7 ml, 35 mmol) versetzt und 3 d bei 5 °C aufbewahrt. Dabei fiel das Rohprodukt als beigefarbener Feststoff in einer Ausbeute von 69 % (915 mg) aus. Nach Chromatographie an Kieselgel (50 g) mit EE/Methanol (1 : 1) und Methanol wurde das Produkt in einer Ausbeute von 25 % als farbloser Feststoff (333 mg) mit einem Smp. von 68-72 °C isoliert.

**2-(4-Dimethylamino-4-phenylcyclohexyliden)-*N*-[5-(1*H*-indol-3-yl)-pentyl]acetamid-hydrochlorid (Beispiel 17)**

2-(4-Dimethylamino-4-phenylcyclohexyliden)-*N*-[5-(1*H*-indol-3-yl)-pentyl]acetamid (81 mg, 0,182 mmol) wurde in Ethylmethylketon (3 ml) gelöst und mit Chlortrimethylsilan (0,035 ml, 0,27 mmol) versetzt. Nach 1,5, h wurde das Hydrochlorid als farbloser Feststoff mit einem Smp. von 174-183 °C in einer Ausbeute von 71 % (62 mg) isoliert.

**6-(1*H*-Indol-3-yl)hexansäure-cyanmethylester**

6-(1H-Indol-3-yl)-hexansäure (6,01 g, 26,0 mmol) wurde in Aceton (30 ml) und DMF (30 ml) gelöst und nacheinander mit Caesiumcarbonat (4,23 g, 13,0 mmol), Chloracetonitril (2,45 ml, 39,0 mmol) und Kaliumiodid (20 mg) versetzt. Nach einer Reaktionszeit von 9 h bei 60 °C und 60 h bei RT wurden die festen Rückstände durch Filtration abgetrennt, mit Aceton (2 × 10 ml) gewaschen und das Filtrat eingeengt. Das Rohprodukt wurde an Kieselgel (150 g) mit EE/Cyclohexan (1 : 3) chromatographisch gereinigt. Der Cyanmethylester wurde als farbloser Feststoff mit einem Smp. von 74-76 °C in einer Ausbeute von 82 % (5,76 g) erhalten.

**6-(1*H*-Indol-3-yl)hexansäureamid**

Eine Lösung von 6-(1*H*-Indol-3-yl)hexansäure-cyanmethylester (5,72 g, 21,16 mmol) in Tetrahydrofuran (160 ml) wurde zu einer 25-proz. Ammoniaklösung (125 ml) gegeben und 40 h bei RT gerührt. Die Aufarbeitung des Ansatzes erfolgte durch Phasentrennung und Extraktion der wässrigen Phase mit THF (2 × 40 ml). Die organischen Extrakte wurden vereinigt und mit gesättigter NaCl-Lösung (50 ml) gewaschen. Nach dem Trocknen und Einengen der organischen Phase wurde das Produkt als farbloser Feststoff in einer Ausbeute von 98 % (4,75 g) mit einem Smp. von 140-142 °C isoliert.

**6-(1*H*-Indol-3-yl)hexylamin**

Lithiumaluminiumhydrid (1,7 g, 40,8 mmol) wurde unter Argon in abs. Tetrahydrofuran (80 ml) gegeben. Eine Lösung von 6-(1*H*-Indol-3-yl)hexansäureamid (4,7 g, 20,4 mmol) in abs. Tetrahydrofuran (100 ml) wurde unter Rühren innerhalb von 40 min bei 60°C der $LiAlH_4$-Suspension hinzugefügt. Nach einer Reaktionszeit von 13 h bei 60°C unter Argon wurde der Ansatz mit Tetrahydrofuran (50 ml) versetzt und unter Eiskühlung Wasser (42 ml) zugegeben. Die dabei erhaltenen Aluminiumverbindungen wurden durch Filtration abgetrennt, mit THF (3 × 30 ml) gewaschen und das Filtrat eingeengt. Nach der Zugabe von Wasser (30 ml) wurde das Amin mit EE (3 × 40 ml) extrahiert, die organischen Extrakte vereinigt, mit Wasser (40 ml) gewaschen, getrocknet und eingeengt. Das Produkt wurde als beigefarbener, öliger Feststoff in einer Ausbeute von 99 % (4.36 g) erhalten.

**2-(4-Dimethylamino-4-phenylcyclohexyliden)-*N*-[6-(1*H*-indol-3-yl)hexyl]acetamid**

Zu einer Lösung von (4-Dimethylamino-4-phenylcyclohexyliden)essigsäurehydrochlorid (810 mg, 2,73 mmol) in trockenem Dimethylformamid (10 ml) wurden unter Argon 1-Hydroxybenzotriazol (737 mg, 5,46 mmol), 6-(1*H*-Indol-3-yl)hexylamin (590 mg, 2,73 mmol) und *N*-Methylmorpholin (0,606 ml, 5,46 mmol) gegeben. Die Lösung wurde auf 0°C abgekühlt und mit Dicyclohexylcarbodiimid (1,13 g, 5,46 mmol) versetzt. Die Reaktionsmischung wurde 11 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser verdünnt (300 ml), mit 5M Natronlauge (7 ml, 35 mmol) versetzt und 16 h bei 5 °C aufbewahrt. Dabei fiel das Rohprodukt als beigefarbener Feststoff in einer Ausbeute von 16 % (197 mg) aus. Nach Chromatographie an Kieselgel (20 g) mit EE/Methanol (4 : 1) wurde das Produkt in einer Ausbeute von 8 % als ölige Verbindung (92 mg) isoliert.

**2-(4-Dimethylamino-4-phenylcyclohexyliden)-*N*-[6-(1*H*-indol-3-yl)hexyl]acetamid-hydrochlorid (Beispiel 18)**

2-(4-Dimethylamino-4-phenylcyclohexyliden)-*N*-[6-(1*H*-indol-3-yl)hex-yl]acetamid (78 mg, 0,17 mmol) wurde in Ethylmethylketon (5 ml) gelöst und mit Chlortrimethylsilan (0,033 ml, 0,26 mmol) versetzt. Nach 1 h wurde Ether (10 ml) hinzugefügt und nach 10 min das Hydrochlorid als farbloser Feststoff mit einem Smp. von 160-164 °C in einer Ausbeute von 87 % (73 mg) isoliert.

**2-[4-Dimethylamino-4-(4-fluorphenyl)cyclohexyliden]-*N*-(3-phenylpropyl)acetamid**

Zu einer Lösung von (4-Dimethylamino-4-(4-fluorphenyl)cyclohexyliden)essigsäurehydrochlorid (840 mg, 3,2

mmol) in trockenem Dimethylformamid (25 ml) wurden unter Argon 1-Hydroxybenzotriazol (864 mg, 6,4 mmol), 3-Phenylpropylamin (0,455 ml, 3,2 mmol) und *N*-Methylmorpholin (0,703 ml, 6,4 mmol) gegeben. Die Lösung wurde auf 0°C abgekühlt und mit Dicyclohexylcarbodiimid (1,32 g, 6,4 mmol) versetzt. Die Reaktionsmischung wurde 4 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das Filtrat wurde mit Wasser verdünnt (400 ml), mit 5M Natronlauge (5 ml, 25 mmol) versetzt und 3 d bei 5 °C aufbewahrt. Dabei fiel das Produkt als beigefarbener öliger Feststoff in einer Ausbeute von 45 % (570 mg) an.

**2-[4-Dimethylamino-4-(4-fluorphenyl)cyclohexyliden]-*N*-(3-phenylpropyl)-acetamid-hydrochlorid (Beispiel 19)**

2-[4-Dimethylamino-4-(4-fluorphenyl)cyclohexyliden]-*N*-(3-phenylpropyl)acetamid (180 mg, 0,45 mmol) wurde in Ethylmethylketon (10 ml) gelöst und mit Chlortrimethylsilan (0,087 ml, 0,68 mmol) versetzt. Nach 1,5 h wurde das Hydrochlorid als farbloser Feststoff mit einem Smp. von 209-212 °C in einer Ausbeute von 65 % (126 mg) isoliert.

**2-[4-Dimethylamino-4-(4-fluorophenyl)cyclohexyliden]-*N*-[2-(1*H*-indol-3-yl)ethyl]acetamid**

Zu einer Lösung von (4-Dimethylamino-4-(4-fluorphenyl)cyclohexyliden)essigsäurehydrochlorid (840 mg, 3,2 mmol) in abs. Dimethylformamid (25 ml) wurden unter Argon 1-Hydroxybenzotriazol (864 mg, 6,4 mmol), Tryptamin (512 mg, 3,2 mmol) und *N*-Methylmorpholin (0,703 ml, 6,4 mmol) gegeben. Die Lösung wurde auf 0°C abgekühlt und mit Dicyclohexylcarbodiimid (1,32 g, 6,4 mmol) versetzt und 4 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Das Filtrat wurde mit Wasser verdünnt (400 ml), mit 5M Natronlauge (5 ml, 25 mmol) versetzt und 4 d bei 5 °C aufbewahrt. Dabei fiel das Produkt als beigefarbener öliger Feststoff in einer Ausbeute von 95 % (1,27 mg) aus.

**2-[4-Dimethylamino-4-(4-fluorophenyl)cyclohexyliden]-*N*-[2-(1*H*-indol-3-yl)ethyl]-acetamid-hydrochlorid (Beispiel 20)**

2-[4-Dimethylamino-4-(4-fluorophenyl)cyclohexyliden]-*N*-[2-1*H*-indol-3-yl)ethyl]-acetamid (871 mg, 2,08 mmol) wurde in Ethylmethylketon (35 ml) gelöst und mit Chlortrimethylsilan (0,39 ml, 3,11 mmol) versetzt. Nach 1,5 h wurde das Hydrochlorid als beigefarbener Feststoff mit einem Smp. von 217-221 °C in einer Ausbeute von 85 % (810 mg) isoliert.

**2-[4-Dimethylamino-4-(4-fluorphenyl)cyclohexyliden]-*N*-[2-(1*H*-indol-3-yl)-1-methylethyl]acetamid**

Zu einer Lösung von (4-Dimethylamino-4-(4-fluorphenyl)cyclohexyliden)essigsäurehydrochlorid (1,98 g, 7,5 mmol) in abs. Dimethylformamid (50 ml) wurden unter Argon 1-Hydroxybenzotriazol (2,03 g, 15 mmol), DL-α-Methyltryptamin (1,3 g, 7,5 mmol) und *N*-Methylmorpholin (1,65 ml, 15 mmol) gegeben. Die Lösung wurde auf 0°C gekühlt und mit Dicyclohexylcarbodiimid (3,09 g, 15 mmol) versetzt und 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 16 h bei 5 °C aufbewahrt. Dabei fiel das Rohprodukt als gelber Feststoff aus (1,55 g, 55 %). Nach Chromatographie an Kieselgel (120 g) mit EE/Methanol (10 : 1) wurde das unpolarere Diastereoisomer in einer Ausbeute von 19% (550 mg) und das polarere Diastereoisomer in einer Ausbeute von 9 % (250 mg) als farblose Feststoffe erhalten.

**2-[4-Dimethylamino-4-(4-fluorphenyl)cyclohexyliden]-*N*-[2-(1*H*-indol-3-yl)-1-methylethyl]acetamid-hydrochlorid (Beispiele 21 und 22)**

Das unpolarere Diastereoisomer von 2-[4-Dimethylamino-4-(4-fluorphenyl)-cyclohexyliden]-*N*-[2-(1*H*-indol-3-yl)-1-methylethyl]acetamid (150 mg, 0,39 mmol) wurde in Ethylmethylketon (10 ml) gelöst und mit Chlortrimethylsilan (0,075 ml, 0,59 mmol) versetzt. Nach 1,5 h konnte das Hydrochlorid als farbloser Feststoff in einer Ausbeute von 63 % (115 mg) mit einem Smp. von 188-191 °C isoliert werden **(Beispiel 21).**

Zu einer Lösung des polareren Diastereoisomers von 2-[4-Dimethylamino-4-(4-fluorphenyl)cyclohexyliden]-*N*-[2-(1*H*-indol-3-yl)-1-methylethyl]acetamid (250 mg, 0,65 mmol) in Ethylmethylketon (10 ml) wurde Chlortrimethylsilan (0,12 ml, 0,98 mmol) hinzugefügt und 1,5 h bei RT gerührt. Dabei fiel das Hydrochlorid (173 mg, 57 %) mit einem Smp. von 201-203 °C als farbloser Feststoff aus (**Beispiel 22**).

**2-(4-Dimethylamino-4-pyridin-2-ylcyclohexyliden)-*N*-[2-(1*H*-indol-3-yl)ethyl]-acetamid**

Zu einer Lösung von (4-Dimethylamino-1-hydroxy-4-pyridin-2-yl-cyclohexyl)-essigsäure (556 mg, 2,0 mmol) in abs. Dimethylformamid (30 ml) wurden unter Argon 1-Hydroxybenzotriazol (546 mg, 4,0 mmol), Tryptamin (320 mg, 2,0 mmol) und *N*-Methylmorpholin (0,444 ml, 4,0 mmol) gegeben. Die Lösung wurde auf 0°C gekühlt und mit Dicyclohexylcarbodiimid (825 mg, 4,0 mmol) versetzt und 7 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (400 ml) verdünnt, mit 5M Natronlauge (7 ml, 35 mmol) versetzt und 16 h bei 5°C aufbewahrt. Dabei fiel das Rohprodukt als beigefarbener Feststoff aus (313 mg, 37 %). Nach Chromatographie an Kieselgel (45 g) mit EE/Methanol (4 : 1) wurde das Produkt in einer Ausbeute von 25 % (210 mg) als beigefarbener Feststoff erhalten.

**2-(4-Dimethylamino-4-pyridin-2-ylcyclohexyliden)-*N*-[2-(1*H*-indol-3-yl)ethyl]-acetamid-hydrochlorid (Beispiel 23)**

2-(4-Dimethylamino-4-pyridin-2-ylcyclohexyliden)-*N*-[2-(1*H*-indol-3-yl)ethyl]acetamid (210 mg, 0,52 mmol) wurde in Ethylmethylketon (5 ml) gelöst und mit Chlortrimethylsilan (0,19 ml, 1,5 mmol) versetzt. Nach 1,5 h wurde das Hydrochlorid als beigefarbener Feststoff mit einem Smp. von 147-150 °C in einer Ausbeute von 89 % (203 mg) isoliert.

**2-(4-Dimethylamino-4-pyridin-2-ylcyclohexyliden)-*N*-(3-phenylpropyl)acetamid**

Zu einer Lösung von (4-Dimethylamino-1-hydroxy-4-pyridin-2-yl-cyclohexyl)-essigsäure (556 mg, 2,0 mmol) in abs. Dimethylformamid (30 ml) wurden unter Argon 1-Hydroxybenzotriazol (546 mg, 4,0 mmol), 3-Phenylpropylamin (270 mg, 2,0 mmol) und *N*-Methylmorpholin (0,444 ml, 4,0 mmol) gegeben. Die Lösung wurde auf 0°C gekühlt und mit Dicyclohexylcarbodiimid (825 mg, 4,0 mmol) versetzt und 5 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 16 h bei 5 °C aufbewahrt. Dabei fiel das Rohprodukt als beigefarbene ölige Verbindung an (228 mg, 30 %). Nach Chromatographie an Kieselgel (40 g) mit EE/Methanol (4 : 1) wurde das Amid in einer Ausbeute von 27 % (203 mg) als beigefarbenes Öl erhalten.

**2-(4-Dimethylamino-4-pyridin-2-ylcyclohexyliden)-*N*-(3-phenylpropyl)acetamidhydrochlorid (Beispiel 24)**

2-(4-Dimethylamino-4-pyridin-2-ylcyclohexyliden)-*N*-(3-phenylpropyl)acetamid (203 mg, 0,537 mmol) wurde in Ethylmethylketon (5 ml) gelöst und mit Chlortrimethylsilan (0,2 ml, 1,6 mmo) versetzt. Nach 1,5 h wurde das Hydrochlorid als farbloser Feststoff mit einem Smp. von 173-176 °C in einer Ausbeute von 86 % (190 mg) isoliert.

**2-[4-Dimethylamino-4-(3-fluorphenyl)cyclohexyliden]-*N*-[2-(1*H*-indol-3-yl)ethyl]-acetamid**

Zu einer Lösung von [4-Dimethylamino-4-(3-fluoro-phenyl)-cyclohexyliden]-essigsäure (1,55 g, 4,0 mmol) in trockenem Dimethylformamid (15 ml) wurden unter Argon 1-Hydroxybenzotriazol (1,08 g, 8,0 mmol), Tryptamin (640 mg, 40 mmol) und *N*-Methylmorpholin (0,888 ml, 8,0 mmol) gegeben. Die Lösung wurde auf 0°C abgekühlt und mit Dicyclohexylcarbodiimid (1,64 g, 8,0 mmol) versetzt und 4 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser verdünnt (300 ml), mit 5M Natronlauge (7 ml, 35 mmol) versetzt und 16 h bei 5 °C aufbewahrt. Dabei fiel das Rohprodukt als beigefarbener Feststoff aus (1,3 g). Nach Chromatographie an Kieselgel (60 g) mit EE/Methanol (6 : 1) wurde das Produkt in einer Ausbeute von 62 % (1,03 g) mit einem Smp. von 172-176 °C gewonnen.

**2-[4-Dimethylamino-4-(3-fluorphenyl)cyclohexyliden]-*N*-[2-(1*H*-indol-3-yl)ethyl]acetamid-hydrochlorid (Beispiel 25)**

2-[4-Dimethylamino-4-(3-fluorphenyl)cyclohexyliden]-*N*-[2-(1*H*-indol-3-yl)ethyl]-acetamid (350 mg, 0,82 mmol) wurde in Ethylmethylketon (25 ml) gelöst und mit Chlortrimethylsilan (0,164 ml, 1,3 mmol) versetzt. Nach 1,5 h konnte das Hydrochlorid als farbloser Feststoff in einer Ausbeute von 89 % (336 mg) mit einem Smp. von 196-200 °C isoliert

werden.

**2-[4-Dimethylamino-4-(3-fluorphenyl)cyclohexyliden]-*N*-[2-(1*H*-indol-3-yl)-1-methylethyl]acetamid**

Zu einer Lösung von [4-Dimethylamino-4-(3-fluoro-phenyl)-cyclohexyliden]-essigsäure (1,4 g, 5,3 mmol) in trockenem Dimethylformamid (50 ml) wurden unter Argon 1-Hydroxybenzotriazol (1,43 g, 10,6 mmol), DL-α-Methyltryptamin (923,3 mg, 5,3 mmol), *N*-Methylmorpholin (1,07 g, 10,6 mmol) und Dicyclohexylcarbodiimid (2,19 g, 10,6 mmol) gegeben und 6 d bei RT gerührt. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser verdünnt (300 ml), mit 5M Natronlauge (7 ml, 35 mmol) versetzt und 20 h bei 5 °C aufbewahrt. Dabei fiel das Rohprodukt als gelber Feststoff aus (1,2 g). Das Produkt war ein Gemisch der beiden Diastereoisomeren Amide, das durch Chromatographie an Kieselgel G [100 g; EtOAc/MeOH (10:1)] getrennt wurde. Das unpolarere Diastereoisomer wurde in einer Ausbeute von 410,6 mg (18 %) mit einem Smp. von 163-166 °C, das polarere Diastereoisomer in einer Ausbeute von 249,3 mg (11 %) als farbloses Öl erhalten.

**2-[4-Dimethylamino-4-(3-fluorphenyl)cyclohexyliden]-*N*-[2-(1*H*-indol-3-yl)-1-methylethyl]acetamid-hydrochlorid (Beispiele 26 und 27)**

Das unpolarere Diastereoisomer von 2-[4-Dimethylamino-4-(3-fluorphenyl)-cyclohexyliden]-*N*-[2-(1*H*-indol-3-yl)-1-methylethyl]acetamid (150 mg, 0,35 mmol) wurde in Ethylmethylketon (5 ml) gelöst und mit Trimethylchlorsilan (0,066 ml, 0,52 mmol) versetzt. Nach 2 h Rühren bei RT wurde das Hydrochlorid abgesaugt und in einer Ausbeute von 77 % (126,6 mg) als farbloser Feststoff mit einem Smp. von 165-188 °C isoliert **(Beispiel 26).**

Eine Lösung aus dem polareren Diastereoisomer von 2-[4-Dimethylamino-4-(3-fluorphenyl)cyclohexyliden]-*N*-[2-(1*H*-indol-3-yl)-1-methylethyl]acetamid (249 mg, 0,57 mmol) in Ethylmethylketon (10 ml) wurde mit Trimethylchlorsilan (0,109 ml, 0,86 mmol) versetzt und 1,5 h bei RT gerührt. Das ausgefallene Hydrochlorid wurde abgesaugt und in einer Ausbeute von 81 % (218 mg) als farbloser Feststoff mit einem Smp. von 171-174 °C isoliert **(Beispiel 27)**.

2-[4-Dimethylamino-4-(3-fluorphenyl)cyclohexyliden]-*N*-(3-phenylpropyl)acetamid Zu einer Lösung von [4-Dimethylamino-4-(3-fluoro-phenyl)-cyclohexyliden]-essigsäure (1,5 g, 5,68 mmol) in trockenem Dimethylformamid (35 ml) wurden unter Argon 1-Hydroxybenzotriazol (1,53 g, 11,36 mmol), 3-Phenylpropylamin (768 mg, 5,68 mmol), *N*-Methylmorpholin (1,15 g, 11,36 mmol) und Dicyclohexylcarbodiimid (2,34 g, 11,36 mmol) gegeben und 3 d bei RT gerührt. Der ausgefallene Harnstoff wurde abgetrennt und das Filtrats in eine Mischung aus gesättigter NaCl-Lösung (40 ml) und gesättigter $NaHCO_3$-Lösun (10 ml) eingetragen. Dabei fiel noch weiterer Harnstoff aus, der abgetrennt wurde. Die wässrige Phase wurde mit Wasser verdünnt (300 ml), mit 5M Natronlauge (7 ml, 35 mmol) versetzt und 20 h bei 5 °C aufbewahrt. Dabei fiel das Rohprodukt als beigefarbener Feststoff aus (2,16 g). Nach Chromatographie an Kieselgel G [100 g; EtOAc/MeOH (10:1)] wurde das Amid in einer Ausbeute von 29 % (648,5 mg) mit einem Smp. von 129-131 °C gewonnen.

**2-[4-Dimethylamino-4-(3-fluorphenyl)cyclohexyliden]-*N*-(3-phenylpropyl)-acetamid-hydrochlorid (Beispiel 28)**

**2-[4-Dimethylamino-4-(3-fluorphenyl)cyclohexyliden]-*N*-(3-phenylpropyl)-acetamid** (150 mg, 0,35 mmol) wurde unter leichtem Erwärmen in Ethylmethylketon (10 ml) gelöst und mit Trimethylchlorsilan (0,082 ml, 0,65 mmol) versetzt. Nach 2 h Rühren bei RT wurde die Reaktionsmischung bis zur Trockne eingeengt. Der Rückstand wurde kurz mit Ether (10 ml) gerührt und das Hydrochlorid in einer Ausbeute von 98 % (184 mg) als farbloser Feststoff mit einem Smp. von 230-233 °C isoliert.

**2-(4-Dimethylamino-4-phenylcyclohexyl)-*N*-(2-trifluoromethylbenzyl)acetamidhydrochlorid (Beispiel 29)**

Zu einer Lösung von 2-(4-Dimethylamino-4-phenylcyclohexyliden)-*N*-(2-trifluormethyl-benzyl)acetamid-hydrochlorid (Beispiel 13; 236 mg, 0,52 mmol) in Methanol wurde Palladium auf Kohle (5 %, 12 mg) gegeben. Die Reaktionsmischung wurde 4 h bei einem Druck von 2 bar bei RT hydriert. Der Katalysator wurde über Celite abgetrennt und das Filtrat eingeengt. Dabei wurde das Produkt als farbloser, hygroskopischer Feststoff in einer Ausbeute von 58 % (138 mg) isoliert.

**(4-Dimethylamino-4-phenylcyclohexyl)essigsäure-methylester-hydrochlorid**

Zu einer Lösung von (4-Dimethylamino-4-phenylcyclohexyliden)essigsäuremethylester-hydrochlorid (1,79 g, 5,77 mmol) in Methanol wurde Palladium auf Kohle (5 %, 90 mg) gegeben. Die Reaktionsmischung wurde 5 h bei einem

Druck von 3 bar bei RT hydriert. Der Katalysator wurde über Celite abgetrennt und das Filtrat eingeengt. Dabei wurde das Produkt als farblose, ölige Verbindung in quantitativer Ausbeute (1,79 g) isoliert.

**(4-Dimethylamino-4-phenylcyclohexyl)essigsäure-hydrochlorid**

Zu einer Lösung von (4-Dimethylamino-4-phenylcyclohexyl)essigsäure-methylester-, hydrochlorid (1,79 g, 5,77 mmol) in Ethanol (80 ml) wurde 1,7M KOH (70,5 ml, 120 mmol) hinzugefügt. Das Gemisch wurde 16 h bei RT gerührt. Die Reaktionsmischung wurde eingeengt und der Rückstand in Wasser aufgenommen. Die wässrige Phase wurde mit Ether extrahiert und mit 5,5M Salzsäure (23,6 ml, 130 mmol) versetzt. Nach dem Einengen der wässrigen Phase wurde der Rückstand mit Ethanol extrahiert, das zurückbleibende Kaliumchlorid abgetrennt und das Filtrat eingeengt. Dabei wurde das Produkt in einer Ausbeute von 95 % (1,62 g) als farblose, ölige Verbindung erhalten.

**2-(4-Dimethylamino-4-phenylcyclohexyl)-*N*-[2-(1*H*-indol-3-yl)ethyl]acetamid**

Zu einer Lösung von (4-Dimethylamino-4-phenylcyclohexyl)essigsäure-hydrochlorid (298 mg, 1,0 mmol) in trockenem Dimethylformamid wurden unter Argon 1-Hydroxybenzotriazol (273 mg, 2,0 mmol), Tryptamin-hydrochlorid (196 mg, 1,0 mmol) und *N*-Methylmorpholin (0,333 ml, 3,0 mmol) gegeben. Die klare Lösung wurde im Eisbad abgekühlt und mit Dicyclohexylcarbodiimid (417 mg, 2,0 mmol) versetzt. Die Reaktionsmischung wurde 3 d bei RT gerührt und durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml) aufgearbeitet. Es wurde mit Wasser verdünnt, mit 5M Natronlauge (4 ml, 20 mmol) versetzt und 16 h bei 5 °C aufbewahrt. Dabei fiel ein Rohprodukt als beigefarbene Substanz (400 mg) aus. Nach chromatographischer Reinigung an Kieselgel mit Methanol wurde das Produkt als beigefarbener Feststoff in einer Ausbeute von 35 % (144 mg) und mit einem Smp. von 194-197 °C erhalten.

**2-(4-Dimethylamino-4-phenylcyclohexyl)-*N*-[2-(1*H*-indol-3-yl)ethyl]acetamidhydrochlorid (Beispiel 30)**

2-(4-Dimethylamino-4-phenylcyclohexyl)-*N*-[2-(1*H*-indol-3-yl)ethyl]acetamid (141 mg, 0,35 mmol) wurde in Ethylmethylketon leicht erwärmt und mit Chlortrimethylsilan (0,066 ml, 0,52 mmol) versetzt. Nach 1,5 h konnte ein beigefarbenes, hygroskopisches Produkt abgesaugt werden. Das Filtrat wurde auf ein Volumen von 5 ml reduziert und mit Ether versetzt. Dabei fiel das Hydrochlorid als farbloser Feststoff in einer Ausbeute von 47 % (72 mg aus. Smp.: 135-140 °C.

**2-(4-Dimethylamino-4-phenylcyclohexyl)-*N*-(3-phenylpropyl)acetamid**

Zu einer Lösung von (4-Dimethylamino-4-phenylcyclohexyl)essigsäure-hydrochlorid (298 mg, 1,0 mmol) in trockenem Dimethylformamid wurden unter Argon 1-Hydroxybenzotriazol (273 mg, 2,0 mmol), 3-Phenylpropylamin (0,142 ml, 1,0 mmol) und *N*-Methylmorpholin (0,222 ml, 2,0 mmol) gegeben. Die klare Lösung wurde bei 0 °C mit Dicyclohexylcarbodiimid (417 mg, 2,0 mmol) versetzt. Die Reaktionsmischung wurde 3 d bei RT gerührt, der ausgefallene Harnstoffes abgesaugt und das Filtrat in eine Mischung aus gesättigter NaCl-Lösung (40 ml) und gesättigter $NaHCO_3$-Lösun (10 ml) eingetragen. Die wässrige Phase wurde mit 5M Natronlauge (12 ml, 60 mmol) versetzt, mit Wasser verdünnt (300 ml) und 3 d bei 5 °C aufbewahrt. Dabei fiel das Produkt als farblose Verbindung an und konnte durch Filtration, Aufnahme des Rückstandes in Ethanol und Einengen in einer Ausbeute von 58 % (220 mg) erhalten werden.

**2-(4-Dimethylamino-4-phenylcyclohexyl)-*N*-(3-phenylpropyl)acetamid)-hydrochlorid (Beispiel 31)**

2-(4-Dimethylamino-4-phenylcyclohexyl)-*N*-(3-phenylpropyl)acetamid (209 mg, 0,55 mmol) wurde in Ethylmethylketon (5 ml) gelöst und mit Chlortrimethylsilan (0,104 ml, 1,0 mmol) versetzt. Nach 1,5 h wurde das Hydrochlorid als farbloser Feststoff mit einem Smp. von 212-215 °C und in einer Ausbeute von 68 % (153 mg) isoliert.

**2-(4-Dimethylamino-4-phenylcyclohexyl)-*N*-phenethylacetamid**

Zu einer Lösung von (4-Dimethylamino-4-phenylcyclohexyl)essigsäure-hydrochlorid (298 mg, 1,0 mmol) in trockenem Dimethylformamid wurden unter Argon 1-Hydroxybenzotriazol (273 mg, 2,0 mmol), 2-Phenylethylamin (0,125 ml, 1,0 mmol) und *N*-Methylmorpholin (0,222 ml, 2,0 mmol) gegeben. Bei 0 °C wurde mit Dicyclohexylcarbodiimid (417 mg, 2,0 mmol) versetzt und 5 d bei RT gerührt. Der ausgefallene Harnstoffe wurde abgesaugt und das Filtrat in eine Mischung aus gesättigter NaCl-Lösung (40 ml) und gesättigter $NaHCO_3$-Lösung (10 ml) eingetragen. Dabei fiel noch weiterer Harnstoff im Gemisch mit dem Produkt aus (349 mg) und wurde abgetrennt. Das erhaltene Filtrat wurde mit Wasser verdünnt (300 ml), mit 5M Natronlauge (8 ml, 40 mmol) versetzt und 16 h bei 5 °C aufbewahrt. Dabei fiel weiteres

Produkt als farbloser Feststoff (172 mg) aus. Nach chromatographischer Reinigung der mit Dicyclohexylharnstoffs verunreinigten Fraktion an Kieselgel mit Methanol wurde weiteres Produkt (49 mg) erhalten. Das Produkt wurde insgesamt in einer Ausbeute von 61 % (221 mg) mit einem Smp. von 182-186 °C isoliert.

**2-(4-Dimethylamino-4-phenylcyclohexyl)-*N*-phenethylacetamid-hydrochlorid (Beispiel 32)**

2-(4-Dimethylamino-4-phenylcyclohexyl)-*N*- phenethylacetamid (220 mg, 0,6 mmol) wurde unter leichtem Erwärmen in Ethylmethylketon gelöst und mit Chlortrimethylsilan (0,113 ml, 0,9 mmol) versetzt. Nach 1,5 h konnte ein farbloser, hygroskopischer Feststoff in einer Ausbeute von 80 % (191 mg) isoliert werden.

**2-(4-Dimethylamino-4-phenylcyclohexyl)-*N*-(4-phenylbutyl)acetamid**

Zu einer Lösung von (4-Dimethylamino-4-phenylcyclohexyl)essigsäure-hydrochlorid (298 mg, 1,0 mmol) in trockenem Dimethylformamid wurden unter Argon 1-Hydroxybenzotriazol (273 mg, 2,0 mmol), 4-Phenylbutylamin (0,158 ml, 1,0 mmol) und *N*-Methylmorpholin (0,222 ml, 2,0 mmol) gegeben. Bei 0 °C wurde mit Dicyclohexylcarbodiimid (417 mg, 2,0 mmol) versetzt und 5 d bei RT gerührt. Die Aufarbeitung des Ansatzes erfolgte durch Abtrennen des ausgefallenen Harnstoffes und Eintragen des Filtrats in eine Mischung aus gesättigter NaCl-Lösung (40 ml) und gesättigter $NaHCO_3$-Lösung (10 ml). Das Filtrat wurde mit Wasser verdünnt (300 ml), mit 5M Natronlauge (8 ml, 40 mmol) versetzt und 16 h bei 5 °C aufbewahrt. Dabei fiel das Rohprodukt als beigefarbene Substanz aus, die durch Filtration abgetrennt wurde (222 mg). Das Produkt wurde in Ethylmethylketon (7 ml) aufgenommen und durch Filtration von einem unlöslichen Rückstand getrennt. Das Filtrat wurde eingeengt und das saubere Produkt in einer Ausbeute von 194 mg (49 %) erhalten.

**2-(4-Dimethylamino-4-phenylcyclohexyl)-*N*-(4-phenylbutyl)acetamidhydrochlorid (Beispiel 33)**

2-(4-Dimethylamino-4-phenylcyclohexyl)-*N*-(4-phenylbutyl)acetamid (194 mg, 0,49 mmol) wurde in Ethylmethylketon gelöst und mit Chlortrimethylsilan (0,093 ml, 0,74 mmol) versetzt. Nach 1,5 h konnte das Produkt als farbloser, hygroskopischer Feststoff in einer Ausbeute von 30 % (63 mg) abgesaugt werden.

**(2*S*)-2-[2-(4-Dimethylamino-4-phenylcyclohexyl)acetylamino]-3-(1*H*-indol-3-yl)-propansäure-methylester**

Zu einer Lösung von (4-Dimethylamino-4-phenylcyclohexyl)essigsäure-hydrochlorid (596 mg, 2,0 mmol) in trockenem Dimethylformamid wurden unter Argon 1-Hydroxybenzotriazol (546 mg, 4,0 mmol), L-Tryptophan-methylester-hydrochlorid (509 mg, 2,0 mmol) und *N*-Methylmorpholin (0,666 ml, 6,0 mmol) gegeben. Bei 0 °C wurde mit Dicyclohexylcarbodiimid (834 mg, 4,0 mmol) versetzt und 4 d bei RT gerührt Die Aufarbeitung des Ansatzes erfolgte durch Abtrennen des ausgefallenen Feststoffes (1,1 g), der aus einem Gemisch von Dicyclohexylharnstoff und dem Hydrochlorid des Amids bestand. Das Filtrat wurde zu einem Gemisch aus gesättigter NaCl-Lösung (40 ml) und gesättigter $NaHCO_3$-Lösung (10 ml) gegeben. Das Filtrat der Mischung wurde mit Dichlormethan ($3 \times 50$ ml) ausgeschüttelt und die organische Phase nach dem Trocknen eingeengt. Dabei wurde ein öliges Rohprodukt erhalten (660 mg), aus dem nach chromatographischer Reinigung an Kieselgel mit EE/Methanol (4 : 1) das Produkt gewonnen werden konnte (188 mg). Das zu Beginn der Aufarbeitung ausgefallene Substanzgemisch wurde in Dichlormethan und gesättigter Natriumhydrogencarbonatlösung aufgenommen. Die wässrige Phase wurde mit Dichlormethan extrahiert. Die organische Phase wurde eingeengt (517 mg) und chromatographisch mit EE/Methanol (4 : 1) aufgetrennt, wobei eine weitere Fraktion des Amids (357 mg) isoliert werden konnte. Die Gesamtausbeute des Produktes betrug 59 % (545 mg).

**(2*S*)-2-[2-(4-Dimethylamino-4-phenylcyclohexyl)acetylamino]-3-(1*H*-indol-3-yl)propansäure-methylester-hydrochlorid (Beispiel 34)**

(2*S*)-2-[2-(4-Dimethylamino-4-phenylcyclohexyl)acetylamino]-3-(1*H*-indol-3-yl)propansäure-methylester (271 mg, 0,587 mmol) wurde in Ethylmethylketon gelöst und Chlortrimethylsilan (0,111 ml, 0,88 mmol) hinzugefügt. Nach 1 h wurde das Gemisch mit Ether (30 ml) versetzt und weitere 10 min gerührt. Das Hydrochlorid wurde abfiltriert und als farbloser Feststoff in einer Ausbeute von 96 % (278 mg) mit einem Smp. von 239-241 °C erhalten.

**(2*S*)-2-[2-(4-Dimethylamino-4-phenylcyclohexyl)acetylamino]-3-(1*H*-indol-3-yl)propansäure-methylester-Kaliumsalz (Beispiel 35)**

(2*S*)-2-[2-(4-Dimethylamino-4-phenylcyclohexyl)acetylamino]-3-(1*H*-indol-3-yl)propansäure-methylester (249 mg, 0,54 mmol) wurde in Ethanol gelöst und mit 1,7M KOH (6,3 ml, 10,8 mmol) versetzt. Nach einer Reaktionszeit von 16 h bei RT erfolgte die Aufarbeitung des Ansatzes durch Einengen, Zugabe von EE und 2 h Rühren bei RT. Das

Kaliumsalz fiel dabei als farbloser Feststoff aus und wurde in einer Ausbeute von 80 % (207 mg) mit einem Smp. von 184-186 °C isoliert.

**2-(4-Dimethylamino-4-phenylcyclohexyl)-*N*-(1*H*-indol-3-ylmethyl)acetamid**

Zu einer Lösung (4-Dimethylamino-4-phenylcyclohexyl)essigsäure-hydrochlorid (596 mg, 2,0 mmol) in trockenem Dimethylformamid wurden unter Argon 1-Hydroxybenzotriazol (546 mg, 4,0 mmol), C-(1*H*-Indol-3-yl)methylamin (292 mg, 2,0 mmol) und *N*-Methylmorpholin (0,444 ml, 4,0 mmol) gegeben. Bei 0 °C wurde mit Dicyclohexylcarbodiimid (834 mg, 4,0 mmol) versetzt und 7 d bei RT gerührt. Die Aufarbeitung des Ansatzes erfolgte durch Abtrennen des ausgefallenen Harnstoffes und Eintragen des Filtrats in eine Mischung aus gesättigter NaCl-Lösung (40 ml) und gesättigter $NaHCO_3$-Lösung (10 ml). Dabei fiel ein Gemisch aus Harnstoff und Produkt aus (482 mg), das abgetrennt wurde. Das erhaltene Filtrat wurde mit Wasser verdünnt (300 ml), mit 5M Natronlauge (4 ml, 20 mmol) versetzt und 16 h bei 5 °C aufbewahrt. Dabei fiel ein Rohprodukt als beigefarbene Substanz (234 mg) aus, die durch Filtration abgetrennt wurde. Nach chromatographischer Reinigung der beiden Produktfraktionen an Kieselgel mit EE/Methanol (4 : 1) und Methanol wurde das Produkt als beigefarbener Feststoff in einer Ausbeute von 30 % (230 mg) isoliert.

**2-(4-Dimethylamino-4-phenylcyclohexyl)-*N*-(1*H*-indol-3-ylmethyl)acetamidhydrochlorid (Beispiel 36)**

2-(4-Dimethylamino-4-phenylcyclohexyl)-*N*-(1*H*-indol-3-ylmethyl)acetamid (213 mg, 0,54 mmol) wurde unter leichtem Erwärmen in Ethylmethylketon gelöst und mit Chlortrimethylsilan (0,103 ml, 0,82 mmol) versetzt. Nach 1,5 h konnte das Hydrochlorid als farbloser Feststoff in einer Ausbeute von 93 % (214 mg) mit einem Smp. von 233-235 °C erhalten werden.

**2-(4-Dimethylamino-4-phenylcyclohexyl)-*N*-[4-(1*H*-indol-3-yl)butyl]acetamid**

Zu einer Lösung von (±)-2-(4-Dimethylamino-4-phenylcyclohexyliden)-*N*-[4-(1*H*-indol-3-yl)butyl]-acetamid (196 mg, 0,456 mmol) in Methanol wurde Palladium auf Kohle (5%, 10 mg) gegeben. Die Reaktionsmischung wurde 21 h bei einem Druck von 2 bar bei RT hydriert. Der Katalysator wurde über Celite abgetrennt und das Filtrat eingeengt. Dabei wurde ein farbloser Feststoff (186 mg) isoliert. Das Rohprodukt wurde chromatographisch an Kieselgel mit Methanol gereinigt. 2-(4-Dimethylamino-4-phenylcyclohexyl)-*N*-[4-(1*H*-indol-3-yl)butyl]acetamid wurde in einer Ausbeute von 30 % (58 mg) als farbloses Produkt mit einem Smp. von 186 °C isoliert.

**2-(4-Dimethylamino-4-phenylcyclohexyl)-*N*-[4-(1*H*-indol-3-yl)butyl]acet-amid-hydrochlorid (Beispiel 37)**

2-(4-Dimethylamino-4-phenylcyclohexyl)-*N*-[4-(1*H*-indol-3-yl)butyl]acetamid (58 mg, 0,142 mmol) wurde unter leichtem Erwärmen in Ethylmethylketon gelöst und mit Chlortrimethylsilan (0,027 ml, 0,213 mmol) versetzt. Nach 1,5 h konnte das Hydrochlorid als farbloser Feststoff in einer Ausbeute von 65 % (43 mg) mit einem Smp. von 165-174 °C erhalten werden.

**2-(4-Dimethylamino-4-phenylcyclohexyl)-*N*-[3-(1*H*-indol-3-yl)propyl]-acetamid**

Zu einer Lösung von 2-(4-Dimethylamino-4-phenyl-cyclohexyliden)-*N*-[3-(1H-indol-3-yl)-propyl]-acetamid (Base von Beispiel 16; 305 mg, 0,734 mmol) in abs. Methanol (30 ml) wurde Palladium auf Kohle (5 %, 20 mg) gegeben. Die Reaktionsmischung wurde 6 h bei einem Druck von 3 bar bei RT hydriert. Der Katalysator wurde über Celite abgetrennt und das Filtrat eingeengt. Nach chromatographischer Auftrennung des Rückstandes (289 mg) an Kieselgel (20 g) mit EE/Methanol (2 : 1) wurde das Produkt als beigefarbenes Öl in einer Ausbeute von 29 % (95 mg) isoliert.

**2-(4-Dimethylamino-4-phenylcyclohexyl)-*N*-[3-(1*H*-indol-3-yl)propyl]-acetamidhydrochlorid (Beispiel 38)**

2-(4-Dimethylamino-4-phenylcyclohexyl)-*N*-[3-(1*H*-indol-3-yl)propyl]-acetamid (68 mg, 0,162 mmol) wurde in Ethylmethylketon (5 ml) gelöst und mit Chlortrimethylsilan (0,033 ml, 0,245 mmol) versetzt. Nach 3 h konnte das Produkt als farbloser Feststoff in einer Ausbeute von 65 % (54 mg) mit einem Smp. von 162-170 °C isoliert werden.

**2-(4-Dimethylamino-4-phenylcyclohexyl)-*N*-[5-(1*H*-indol-3-yl)pentyl]-acetamid**

Zu einer Lösung von 2-(4-Dimethylamino-4-phenyl-cyclohexyliden)-N-[5-(1H-indol-3-yl)-pentyl]-acetamid (Base zu Beispiel 17; 246 mg, 0,55 mmol) in abs. Methanol (30 ml) wurde Palladium auf Kohle (5 %, 15 mg) gegeben. Die Reaktionsmischung wurde 6 h bei einem Druck von 2 bar bei RT hydriert. Der Katalysator wurde über Celite abgetrennt

und das Filtrat eingeengt. Nach chromatographischer Auftrennung des Rückstandes (234 mg) an Kieselgel (30 g) mit EE/Methanol (1 : 1) wurde das Produkt als beigefarbenes Öl in einer Ausbeute von 46 % (112 mg) isoliert.

**2-(4-Dimethylamino-4-phenylcyclohexyl)-*N*-[5-(1*H*-indol-3-yl)pentyl]-acetamidhydrochlorid (Beispiel 39)**

2-(4-Dimethylamino-4-phenylcyclohexyl)-*N*-[5-(1*H*-indol-3-yl)pentyl]-acetamid (112mg, 0,25 mmol) wurde in Ethylmethylketon (5 ml) gelöst und mit Chlortrimethylsilan (0,048 ml, 0,38 mmol) versetzt. Nach 1 h wurde die Reaktionsmischung mit Ether (15 ml) versetzt. Das Produkt wurde als rosafarbener Feststoff in einer Ausbeute von 90 % (108 mg) erhalten.

**2-(4-Dimethylamino-4-phenylcyclohexyl)-*N*-[6-(1*H*-indol-3-yl)hexyl]acetamid**

Zu einer Lösung von 4-Dimethylamino-4-phenylcyclohexyl-essigsäure-hydrochlorid (595 mg, 2,0 mmol) in trockenem Dimethylformamid (10 ml) wurden unter Argon 1-Hydroxybenzotriazol (540 mg, 4.0 mmol), 6-(1*H*-Indol-3-yl) hexylamin (432 mg, 2,0 mmol) und *N*-Methylmorpholin (0,444 ml, 4,0 mmol) gegeben. Die klare Lösung wurde im Eisbad abgekühlt, mit Dicyclohexylcarbodiimid (825 mg, 4,0 mmol) versetzt und 5 d bei RT gerührt, wobei Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Aus dem Filtrat fiel nach 10 min das Rohprodukt aus und wurde abfiltriert. Die wässrige Phase wurde mit Wasser (300 ml) verdünnt, mit 5M Natronlauge (7 ml, 35 mmol) versetzt und 3 d bei 5 °C aufbewahrt. Dabei fiel weiteres Produkt als beigefarbener Feststoff in einer Ausbeute von 9 % (82 mg) mit einem Smp. von 152-156 °C aus. Das Rohprodukt wurde chromatographisch an Kieselgel (40 g) mit EE/Methanol (4:1) und (1 : 1) gereinigt. Das Produkt wurde dabei in einer Ausbeute von 58 % als beigefarbene Verbindung (534 mg) isoliert.

**2-(4-Dimethylamino-4-phenylcyclohexyl)-*N*-[6-(1*H*-indol-3-yl)hexyl]acetamidhydrochlorid (Beispiel 40)**

2-(4-Dimethylamino-4-phenylcyclohexyl)-*N*-[6-(1*H*-indol-3-yl)hexyl]acetamid (516 mg, 1,12 mmol) wurde in einem Gemisch von Ethylmethylketon (25 ml) und Ethanol (5 ml) gelöst und mit 5M isopropanolischer Salzsäure (0,44 ml, 2,2 mmol) versetzt. Die Reaktionsmischung wurde nach 3 h eingeengt, mit Ether (15 ml) versetzt und nochmals eingeengt. Der Rückstand wurde mit Ether (30 ml) digeriert. Nach Filtration wurde das Produkt als beigefarbener Feststoff in einer Ausbeute von 62 % (342 mg) erhalten.

**2-(4-Dimethylamino-4-phenylcyclohexyl)-*N*-[2-(1*H*-indol-3-yl)-1-methylethyl]-acetamid**

Zu einer Lösung von 4-Dimethylamino-4-phenylcyclohexyl-essigsäure-hydrochlorid (744 mg, 2,5 mmol) in trockenem Dimethylformamid (10 ml) wurden unter Argon 1-Hydroxybenzotriazol (675 mg, 5,0 mmol), α-Methyltryptamin (435 mg, 2,5 mmol) und *N*-Methylmorpholin (0,555 ml, 5,0 mmol) gegeben. Die klare Lösung wurde im Eisbad abgekühlt, mit Dicyclohexylcarbodiimid (1,03 g, 5,0 mmol) versetzt und 5 d bei RT gerührt, wobei Dicyclohexylharnstoff ausfiel. Die Aufarbeitung des Ansatzes erfolgte durch Abtrennen des ausgefallenen Harnstoffes und Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser verdünnt (300 ml), mit 5M Natronlauge (7 ml, 35 mmol) versetzt und 16 h bei 5 °C aufbewahrt. Dabei fiel das Produkt als Gemisch mit Dicyclohexylharnstoff als beigefarbener Feststoff in einer Ausbeute von 93 % (968 mg) aus.

**2-(4-Dimethylamino-4-phenylcyclohexyl)-*N*-[2-(1*H*-indol-3-yl)-1-methylethyl]-acetamid-hydrochlorid (Beispiel 41)**

Ein Gemisch von 2-(4-Dimethylamino-4-phenylcyclohexyl)-*N*-[2-(1*H*-indol-3-yl)-1-methylethyl]acetamid mit Dicyclohexylharnstoff (939 mg) wurde mit Ethylmethylketon (55 ml) versetzt, auf 40°C erwärmt und die Mischung filtriert. Dabei blieb das Acetamid als farbloser Feststoff (327 mg, 0,78 mmol) mit einem Smp. von 198-200 °C zurück. Es wurde in Ethanol (20 ml) gelöst und mit 5M propanolischer Salzsäure (0,22 ml, 1,11 mmol) versetzt. Die Reaktionsmischung wurde nach 2 h auf 2 ml eingeengt, mit Ether (50 ml) versetzt und 1 h bei RT gerührt. Das Produkt konnte als farbloser Feststoff mit einem Smp. von 150-155 °C in einer Ausbeute von 33 % (338 mg) isoliert werden.

**2-[4-Dimethylamino-4-(4-fluorphenyl)cyclohexyl]-*N*-(3-phenylpropyl)acetamid**

Zu einer Lösung von 2-[4-Dimethylamino-4-(4-fluorphenyl)-cyclohexyliden]-N-(3-phenylpropyl)-acetamid (Base zu Beispiel 19; 390 mg, 0,99 mmol) in abs. Methanol (25 ml) wurde Palladium auf Kohle (5 %, 24 mg) gegeben. Die

Reaktionsmischung wurde 17 h bei einem Druck von 2 bar bei RT hydriert. Der Katalysator wurde über Celite abgetrennt und das Filtrat eingeengt. Nach chromatographischer Auftrennung des Rückstandes (402 mg) an Kieselgel (80 g) mit EE/Methanol (2 : 1) wurde das unpolarere Diastereoisomer von 2-[4-Dimethylamino-4-(4-fluorphenyl)cyclohexyl]-*N*-(3-phenylpropyl)acetamid als beigefarbenes Öl in einer Ausbeute von 24 % (78 mg) und das polarere Diastereoisomer von 2-[4-Dimethylamino-4-(4-fluorphenyl)-cyclohexyl]-*N*-(3-phenylpropyl)acetamid in einer Ausbeute von 76 % (297 mg) als farbloser öliger Feststoff isoliert.

**2-[4-Dimethylamino-4-(4-fluorphenyl)cyclohexyl]-*N*-(3-phenylpropyl)acetamid-hydrochlorid (Beispiele 42 und 43)**

Das unpolarere Diastereoisomer von 2-[4-Dimethylamino-4-(4-fluorphenyl)-cyclohexyl]-*N*-(3-phenylpropyl) acetamid (78 mg, 0,2 mmol) wurde in Ethylmethylketon (10 ml) gelöst und mit Chlortrimethylsilan (0,038 ml, 0,3 mmol) versetzt. Nach 1,5 h wurde Ether (15 ml) hinzugefügt und 30 min gerührt. Das Produkt (Beispiel 42) wurde als farbloser Feststoff in einer Ausbeute von 71 % (62 mg) mit einem Smp. von 109-111 °C erhalten.

Das polarere Diastereoisomer von 2-[4-Dimethylamino-4-(4-fluorphenyl)cyclohexyl]-N-(3-phenylpropyl)äcetamid (295 mg, 0,74 mmol) wurde in einem Gemisch von Ethylmethylketon (10 ml) und Ethanol (10 ml) gelöst, mit 5M propanolischer Salzsäure (0,22 ml, 1,1 mmol) versetzt und 1,5 h bei RT gerührt. Nach der Zugabe von Ether (50 ml) wurde 2 h gerührt und das ausgefallene Hydrochlorid abgetrennt. Das Produkt wurde in einer Ausbeute von 67 % (213 mg) erhalten (Beispiel 43).

**2-[4-Dimethylamino-4-(4-fluorphenyl)cyclohexyl]-*N*-[2-(1*H*-indol-3-yl)ethyl]-acetamid**

Zu einer Lösung von 2-[4-Dimethylamino-4-(4-fluorphenyl)-cyclohexyliden]-N-[2-(1H-indol-3-yl)-ethyl]-acetamid (Base zu Beispiel 20; 442 mg, 1,05 mmol) in abs. Methanol (80 ml) wurde Palladium auf Kohle (5 %, 40 mg) gegeben. Die Reaktionsmischung wurde 17 h bei einem Druck von 2 bar bei RT hydriert. Der Katalysator wurde über Celite abgetrennt und das Filtrat eingeengt. Nach chromatographischer Auftrennung des Rückstandes an Kieselgel (80 g) mit EE/Methanol (2 : 1) wurde das unpolarere Diastereoisomer von 2-[4-Dimethylamino-4-(4-fluorphenyl)cyclohexyl]-*N*-[2-(1*H*-indol-3-yl)ethyl]acetamid als Gemisch mit einem zweiten nicht identifizierbaren Produkt als farbloses Öl (90 mg) und das reine polarere Diastereoisomer von 2-[4-Dimethylamino-4-(4-fluorphenyl)cyclohexyl]-*N*-[2-(1*H*-indol-3-yl)ethyl]acetamid in einer Ausbeute von 50 % (224 mg) als farbloser Feststoff mit einem Smp. von 210-213°C isoliert.

**2-[4-Dimethylamino-4-(4-fluorphenyl)cyclohexyl]-*N*-[2-(1*H*-indol-3-yl)-ethyl]acetamid-hydrochlorid (Beispiel 44)**

Das polarere Diastereoisomer von 2-[4-Dimethylamino-4-(4-fluorphenyl)cyclohexyl]-*N*-[2-(1*H*-indol-3-yl)ethyl] acetamid (224 mg, 0,74 mmol) wurde in einem Gemisch von Ethylmethylketon (15 ml) und Ethanol (15 ml) gelöst, mit 5M isopropanolischer Salzsäure (0,16 ml, 0,795 mmol) versetzt und 2,5 h bei RT gerührt. Das Produkt wurde abgesaugt und in einer Ausbeute von 75 % (181 mg) mit einem Smp. von 252-255 °C erhalten.

**2-[4-Dimethylamino-4-(4-fluorphenyl)cyclohexyl]-*N*-[2-(1*H*-indol- 3-yl)- 1-**methylethyl]acetamid

Zu einer Lösung von 2-[4-Dimethylamino-4-(4-fluoro-phenyl)-cyclohexyliden]-N-[2-(1H-indol-3-yl)-1-methylethyl]-acetamid (370 mg, 0,97 mmol) in abs. Methanol (40 ml) wurde Palladium auf Kohle (5 %, 40 mg) gegeben. Die Reaktionsmischung wurde 23 h bei einem Druck von 3 bar bei RT hydriert. Der Katalysator wurde über Celite abgetrennt und das Filtrat eingeengt. Nach chromatographischer Auftrennung des Rückstandes (339 mg) an Kieselgel (50 g) mit EE/Methanol (2 : 1) wurde das unpolarere Diastereoisomer von 2-[4-Dimethylamino-4-(4-fluorphenyl)cyclohexyl]-*N*-[2-(1H-indol-3-yl)-1-methylethyl]acetamid als farbloses Öl in einer Ausbeute von 11 % (45 mg) und das polarere Diastereoisomer von 2-[4-Dimethylamino-4-(4-fluorphenyl)cyclohexyl]-*N*-[2-(1*H*-indol-3-yl)-1-methylethyl]acetamid in einer Ausbeute von 57 % (240 mg) als farbloser Feststoff mit einem Smp. von 220-222 °C isoliert.

**2-[4-Dimethylamino-4-(4-fluorphenyl)cyclohexyl]-*N*-[2-(1*H*-indol- 3-yl)- 1-**methylethyl]acetamid-hydrochlorid (Beispiele 45 und 46)

Das unpolarere Diastereoisomer von 2-[4-Dimethylamino-4-(4-fluorphenyl)-cyclohexyl]-*N*-[2-(1*H*-indol-3-yl)-1-methylethyl]acetamid (44 mg, 0,1 mmol) wurde in Ethylmethylketon (5 ml) gelöst und mit Chlortrimethylsilan (0,019 ml, 0,15 mmol) versetzt. Nach 1,5 h wurde Ether (5 ml) hinzugefügt und 30 min gerührt. Das Produkt wurde als farblose ölige Verbindung in einer Ausbeute von 38 % (18 mg) erhalten (Beispiel 45).

Das polarere Diastereoisomer von 2-[4-Dimethylamino-4-(4-fluorphenyl)cyclohexyl]-N-[2-(1H-indol-3-yl)-1-methylethyl]acetamid (223 mg, 0,5 mmol) wurde in Ethanol (20 ml) gelöst, mit 5M isopropanolischer Salzsäure (0,154 ml, 0,75 mmol) versetzt und 1 h bei RT gerührt. Die Reaktionsmischung wurde eingeengt und 16 h mit Ether (10 ml) gerührt. Das ausgefallene farblose Hydrochlorid wurde abgetrennt und in einer Ausbeute von 88 % (207 mg) mit einem Smp. von 188-191°C erhalten (Beispiel 46).

**2-(4-Dimethylamino-4-pyridin-2-ylcyclohexyl)-*N*-[2-(1*H*-indol-3-yl)-ethyl]-acetamid**

Zu einer Lösung des unpolareren Diastereoisomers von (4-Dimethylamino-4-pyridin-2-yl-cyclohexyl)-essigsäure (598 mg, 2,0 mmol) in abs. Dimethylformamid (20 ml) wurden unter Argon 1-Hydroxybenzotriazol (546 mg, 4,0 mmol), Tryptamin (320 mg, 2,0 mmol) und N-Methylmorpholin (0,444 ml, 4,0 mmol) gegeben. Die Lösung wurde im Eisbad gekühlt, mit Dicyclohexylcarbodiimid (825 mg, 4,0 mmol) versetzt und 6 d bei RT gerührt, wobei Dicyclohexylharnstoff ausfiel. Die Aufarbeitung des Ansatzes erfolgte durch Abtrennen des ausgefallenen Harnstoffes und Eintragen des Filtrats in eine Mischung aus gesättigter NaCl-Lösung (40 ml) und gesättigter $NaHCO_3$-Lösung (10ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 16 h bei 5 °C aufbewahrt. Dabei fiel das Rohprodukt als beigefarbene ölige Verbindung an (303 mg, 37 %). Nach chromatographischer Reinigung an Kieselgel (45 g) mit EE/Methanol (2 : 1) wurde das Produkt in einer Ausbeute von 17 % (141 mg) als beigefarbenes Öl erhalten.

Zu einer Lösung des polareren Diastereoisomers von (4-Dimethylamino-4-pyridin-2-yl-cyclohexyl)-essigsäure (476 mg, 1,6 mmol) in abs. Dimethylformamid (20 ml) wurden unter Argon 1-Hydroxybenzotriazol (432 mg, 3,2 mmol), Tryptamin (256 mg, 1,6 mmol) und N-Methymorpholin (0,352 ml, 3,2 mmol) gegeben. Die Lösung wurde im Eisbad gekühlt, mit Dicyclohexylcarbodiimid (660 mg, 3,2 mmol) versetzt und 13 d bei RT gerührt, wobei Dicyclohexylharnstoff ausfiel. Die Aufarbeitung des Ansatzes erfolgte durch Abtrennen des ausgefallenen Harnstoffes und das Eintragen des Filtrats in eine Mischung aus gesättigter NaCl-Lösung (40 ml) und gesättigter $NaHCO_3$-Lösung (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 16 h bei 5 °C aufbewahrt. Dabei fiel das Rohprodukt als beigefarbene ölige Verbindung aus (319 mg, 49 %). Nach chromatographischer Reinigung an Kieselgel (45 g) mit Methanol und Methanol/30-proz. wässrigem Ammoniak (100 : 1) wurde das Produkt in einer Ausbeute von 12 % (80 mg) als beigefarbenes Öl erhalten.

**2-(4-Dimethylamino-4-pyridin-2-ylcyclohexyl)-*N*-[2-(1*H*-indol-3-yl)-ethyl]-acetamid-hydrochlorid (Beispiele 47 und 48)**

Das unpolarere Diastereoisomer von 2-(4-Dimethylamino-4-pyridin-2-ylcyclohexyl)-*N*-[2-(1*H*-indol-3-yl)-ethyl] acetamid (141 mg, 0,348 mmol) wurde in Ethylmethylketon (6 ml) gelöst und mit Chlortrimethylsilan (0,126 ml, 1,0 mmol) versetzt. Nach einer Reaktionszeit von 1 h wurde das Hydrochlorid als beigefarbener Feststoff in einer Ausbeute von 84 % (130 mg) mit einem Smp. von 150-153°C erhalten (Beispiel 47).

Das polarere Diastereoisomer von 2-(4-Dimethylamino-4-pyridin-2-ylcyclohexyl)-*N*-[2-(1*H*-indol-3-yl)-ethyl] acetamid (80 mg, 0,20 mmol) wurde in Ethylmethylketon (5 ml) gelöst und mit Trimethylchlorsilan (0,038 ml, 0,3 mmol) versetzt und 1,5 h bei RT gerührt. Das ausgefallene Hydrochlorid wurde in einer Ausbeute von 95 % (84 mg) erhalten (Beispiel 48).

**2-(4-Dimethylamino-4-pyridin-2-ylcyclohexyl)-*N*-(3-phenylpropyl)acetamid**

Zu einer Lösung des unpolareren Diastereoisomers von (4-Dimethylamino-4-pyridin-2-yl-cyclohexyl)-essigsäure (760 mg, 2,55 mmol) in abs. Dimethylformamid (30 ml) wurden unter Argon 1-Hydroxybenzotriazol (689 mg, 5,1 mmol), 3-Phenylpropylamin (345 mg, 2,55 mmol) und N-Methylmorpholin (0,560 ml, 5,1 mmol) gegeben. Die Lösung wurde im Eisbad gekühlt, mit Dicyclohexylcarbodiimid (1,05 g, 5,1 mmol) versetzt und 5 d bei RT gerührt, wobei Dicyclohexylharnstoff ausfiel. Die Aufarbeitung des Ansatzes erfolgte durch Abtrennen des ausgefallenen Harnstoffes und Eintragen des Filtrats in eine Mischung aus gesättigter NaCl-Lösung (40 ml) und gesättigter $NaHCO_3$-Lösun (10 ml). Dabei fiel noch weiterer Harnstoff aus, der abgetrennt wurde. Die wässrige Phase wurde mit Wasser (300 ml) verdünnt, mit 5M Natronlauge (7 ml, 35 mmol) versetzt und 16 h bei 5 °C aufbewahrt. Dabei fiel das Rohprodukt als beigefarbene ölige Verbindung an (890 mg, 92 %). Nach chromatographischer Reinigung an Kieselgel (50 g) mit EE/Methanol (10 : 1) wurde das unpolarere Diastereoisomer von 2-(4-Dimethylamino-4-pyridin-2-ylcyclohexyl)-*N*-(3-phenylpropyl)acetamid in einer Ausbeute von 42 % (403 mg) als farbloses Öl erhalten.

**2-(4-Dimethylamino-4-pyridin-2-ylcyclohexyl)-*N*-(3-phenylpropyl)acetamid-hydrochlorid (Beispiel 49)**

Das unpolarere Diastereoisomer von 2-(4-Dimethylamino-4-pyridin-2-ylcyclohexyl)-*N*-(3-phenylpropyl)acetamid (400 mg, 1,05 mmol) wurde in Ethylmethylketon (10 ml) gelöst und mit Chlortrimethylsilan (0,2 ml, 1,58 mmol)

versetzt. Nach einer Reaktionszeit von 2 h wurde das Hydrochlorid als beigefarbener Feststoff in einer Ausbeute von 94 % (411 mg) mit einem Smp. von 218-220 °C erhalten.

**2-[4-Dimethylamino-4-(3-fluorphenyl)cyclohexyl]-*N*-[2-(1*H*-indol-3-yl)ethyl]-acetamid**

Zu einer Lösung von 2-[4-Dimethylamino-4-(3-fluorphenyl)-cyclohexyliden]-N-[2-(1H-indol-3-yl)-ethyl]-acetamid (Base zu Beispiel 25; 660 mg, 1,55 mmol) in abs. Methanol (100 ml) wurde Palladium auf Kohle (5 %, 120 mg) gegeben. Die Reaktionsmischung wurde 20 h bei einem Druck von 3 bar bei 40 °C hydriert. Der Katalysator wurde über Celite abgetrennt und das Filtrat eingeengt. Nach chromatographischer Auftrennung des Rückstandes an Kieselgel (45 g) mit EE/Methanol (5 : 1) und Methanol wurde das unpolarere Diastereoisomer von 2-[4-Dimethylamino-4-(3-fluorphenyl)cyclohexyl]-*N*-[2-(1*H*-indol-3-yl)ethyl]acetamid in einer Ausbeute von 13 % (86 mg) und das polarere Diastereoisomer von 2-[4-Dimethylamino-4-(3-fluorphenyl)cyclohexyl]-*N*-[2-(1*H*-indol-3-yl)ethyl]acetamid in einer Ausbeute von 59 % (391 mg) isoliert. Beide Amide waren farblose Öle und diastereoisomerenrein.

**2-[4-Dimethylamino-4-(3-fluorphenyl)cyclohexyl]-*N*-[2-(1*H*-indol-3-yl)ethyl]-acetamid hydrochlorid (Beispiele 50 und 51)**

Das unpolarere Diastereoisomer von 2-[4-Dimethylamino-4-(3-fluorphenyl)-cyclohexyl]-*N*-[2-(1*H*-indol-3-yl) ethyl]acetamid (86 mg, 0,204 mmol) wurde in Ethylmethylketon (5 ml) gelöst und mit Chlortrimethylsilan (0,038 ml, 0,3 mmol) versetzt. Nach 1 h wurde die Reaktionsmischung mit Ether (30 ml) versetzt und 30 min bei RT gerührt. Das Hydrochlorid wurde als farbloser Feststoff in einer Ausbeute von 94 % (87 mg) mit einem Smp. von 135-140 °C isoliert (Beispiel 50).

Zu einer Lösung des polareren Diastereoisomers von 2-[4-Dimethylamino-4-(3-fluorphenyl)cyclohexyl]-*N*-[2-(1*H*-indol-3-yl)ethyl]acetamid (372 mg, 0,776 mmol) in Ethylmethylketon (30 ml) wurde Chlortrimethylsilan (0,164 ml, 1,3 mmol) gegeben und 1,5 h bei RT gerührt. Das Reaktionsgemisch wurde auf 10 ml eingeengt, mit Ether (60 ml) versetzt und 20 min bei RT gerührt. Das Hydrochlorid wurde als farbloser Feststoff in einer Ausbeute von 87 % (348 mg) gewonnen (Beispiel 51).

**2-[4-Dimethylamino-4-(3-fluorphenyl)cyclohexyl]-*N*-[2-(1*H*-indol-3-yl)-1-methyl-ethyl]acetamid**

Zu einer Lösung von 2-[4-Dimethylamino-4-(3-fluorphenyl)-cyclohexyliden]-N-[2-(1H-indol-3-yl)-1-methylethyl]-acetamid (417 mg, 0,96 mmol) in abs. Methanol (40 ml) wurde Palladium auf Kohle (5 %, 40 mg) gegeben. Die Reaktionsmischung wurde 15 h bei einem Druck von 3 bar bei RT hydriert. Der Katalysator wurde über Celite abgetrennt und das Filtrat eingeengt. Nach chromatographischer Auftrennung des Rückstandes an Kieselgel G [50 g; EE/MeOH (10:1) - (5:1)] wurde das unpolarere Diastereoisomer von 2-[4-Dimethylamino-4-(3-fluorphenyl)cyclohexyl]-*N*-[2-(1*H*-indol-3-yl)-1-methylethyl]acetamid in einer Ausbeute von 4 % (15 mg) und das polarere Diastereoisomer von 2-[4-Dimethylamino-4-(3-fluorphenyl)cyclohexyl]-*N*-[2-(1*H*-indol-3-yl)-1-methylethyl]acetamid in einer Ausbeute von 41 % (170,2 mg) isoliert. Beide Produkte waren farblose Öle und diastereoisomerenrein.

**2-[4-Dimethylamino-4-(3-fluorphenyl)cyclohexyl]-*N*-[2-(1*H*-indol-3-yl)-1-methylethyl]acetamid-hydrochlorid (Beispiel 52)**

Das polarere Diastereoisomer von 2-[4-Dimethylamino-4-(3-fluorphenyl)cyclohexyl]-N-[2-(1H-indol-3-yl)-1-methylethyl]acetamid (139 mg, 0,32 mmol) in Ethanol (10 ml) unter leichtem Erwärmen gelöst, mit 5M isopropanolischer Salzsäure (0,096 ml, 0,48 mmol) versetzt und bei RT 2 h gerührt. Die klare Lösung wurde auf ca. 1 ml eingeengt und mit Ether (10 ml) 20 h gerührt. Der dabei entstandene Feststoff wurde abgesaugt. Das polarere Diastereoisomer von 2-[4-Dimethylamino-4-(3-fluorphenyl)-cyclohexyl]-*N*-[2-(1*H*-indol-3-yl)-1-methylethyl]acetamid-hydrochlorid wurde so in einer Ausbeute von 93,5 mg (62 %) als rosafarbener Feststoff erhalten.

**2-[4-Dimethylamino-4-(3-fluorphenyl)cyclohexyl]-*N*-(3-phenylpropyl)acetamid**

Zu einer Lösung von 2-[4-Dimethylamino-4-(3-fluorphenyl)-cyclohexyliden]-*N*-(3-phenylpropyl)-acetamid (440 mg, 1,1 mol) in abs. Methanol (40 ml) wurde Palladium auf Kohle (5 %, 22 mg) gegeben. Die Reaktionsmischung wurde 16 h bei einem Druck von 3 bar bei RT hydriert. Der Katalysator wurde über Celite abgetrennt und das Filtrat eingeengt. Nach chromatographischer Auftrennung des Rückstandes an Kieselgel G [(80 g); EE/MeOH (2:1)] wurde das unpolarere Diastereoisomer von 2-[4-Dimethylamino-4-(3-fluorphenyl)cyclohexyl]-*N*-(3-phenylpropyl)acetamid in einer Ausbeute von 15 % (64,3 mg) als farbloses Öl und das polarere Diastereoisomer von 2-[4-Dimethylamino-4-(3-fluorphenyl)cyclohexyl]-*N*-(3-phenylpropyl)acetamid in einer Ausbeute von 72 % (314 mg) als farbloser Feststoff mit einem Smp. von

119-122 °C isoliert.

**2-[4-Dimethylamino-4-(3-fluorphenyl)cyclohexyl]-*N*-(3-phenylpropyl)-acetamidhydrochlorid (Beispiele 53 und 54)**

Das unpolarere Diastereoisomer von 2-[4-Dimethylamino-4-(3-fluorphenyl)-cyclohexyl]-*N*-(3-phenylpropyl)-acetamid (64,3 mg, 0,16 mmol) wurde in Ethylmethylketon (10 ml) gelöst und mit Chlortrimethylsilan (0,03 ml, 0,24 mmol) versetzt. Nach 2 h wurde zur Reaktionsmischung Ether (10 ml) gegeben und 30 min bei RT gerührt. Das Hydrochlorid wurde als farbloser, hygroskopischer Feststoff in einer Ausbeute von 78 % (54 mg) isoliert (Beispiel 53).

Zu einer Lösung des polareren Diastereoisomers von 2-[4-Dimethylamino-4-(3-fluorphenyl)cydohexyl]-*N*-(3-phenylpropyl)-acetamid (274 mg, 0,69 mmol) in Ethylmethylketon (20 ml) wurde Chlortrimethylsilan (0,132 ml, 1,04 mmol) gegeben und 1 h bei RT gerührt. Das Reaktionsgemisch wurde mit Ether (20 ml) versetzt und 1 h bei RT gerührt. Das Hydrochlorid wurde als farbloser Feststoff in einer Ausbeute von 82.% (245,5 mg) mit einem Smp. von 205-207 °C gewonnen (Beispiel 54).

**2-(4-Dimethylamino-4-phenyl-cyclohexyl)-N-phenyl-acetamid**

Bei 0°C wurde zu einer Lösung von Anilin (0,53 g; 5,7 mmol) in 10 ml THF n-Butyllithium (1,6M Lösung in Hexan; 7,1 ml; 11,4 mmol) getropft. Man ließ auf RT kommen und rührte 1 h nach. Bei -78°C wurde eine Lösung von (4-Dimethylamino-4-phenyl-cyclohexyl)-essigsäure-ethylester (1,5 g; 5,2 mmol) in 15 ml THF getropft. Es wurde 1 h nach gerührt und dann auf RT kommen gelassen. Der Ansatz wurde mit Ammoniumchlorid-Lösung hydrolysiert und mit DCM extrahiert. Nach dem Einengen wurde das Rohprodukt an Kieselgel chromatographiert (Eluens: Ether). Man erhielt 308 mg (18%) des unpolareren und 620 mg (36%) des polareren Diastereoisomers.

**2-(4-Dimethylamino-4-phenyl-cyclohexyl)-N-phenyl-acetamid-hydrochlorid (Beispiele 55 und 56)**

Eine Lösung des unpolareren Diastereoisomers von 2-(4-Dimethylamino-4-phenylcyclohexyl)-N-phenyl-acetamid (300 mg, 0,9 mmol) in Ethylmethylketon (2 ml) wurde mit Chlortrimethylsilan (0,124 ml) und Wasser (9 µl) versetzt. Nach 18 h konnte das Hydrochlorid als farbloser Feststoff in einer Ausbeute von 64 % (210 mg) isoliert werden (Beispiel 55).

Analog wurde mit dem polareren Diastereoisomer (600 mg; 1,8 mmol) verfahren. Da kein Niederschlag ausfiel, wurde das Produkt mit Hexan ausgeölt. Man erhielt 670 mg (1,797 mmol; 99%) des Produktes (Beispiel 56).

**2-(4-Dimethylamino-4-phenyl-cyclohexyl)-N-(4-fluor-phenyl)-acetamid**

Bei 0°C wurde zu einer Lösung von 4-Fluoranilin (1,06 g; 9,5 mmol) in 10 ml THF n-Butyllithium (1,6M Lösung in Hexan; 11,9 ml; 19 mmol) getropft. Man ließ auf RT kommen und rührte 1 h nach. Bei -78°C wurde eine Lösung von (4-Dimethylamino-4-phenyl-cyclohexyl)-essigsäure-ethylester (2,5 g; 8,6 mmol) in 15 ml THF getropft. Es wurde 1 h nach gerührt und dann auf RT kommen gelassen. Der Ansatz wurde mit Ammoniumchlorid-Lösung hydrolysiert und mit DCM extrahiert. Nach dem Einengen wurde das Rohprodukt in Ether aufgenommen. Dabei blieb ein Feststoff zurück (unpolareres Diastereoisomer; 1,37 g; 45 %); der Überstand wurde an Kieselgel chromatographiert (Eluens: Ether). Man erhielt 315 mg (10%) des polareren Diastereoisomers.

**2-(4-Dimethylamino-4-phenyl-cyclohexyl)-N-(4-fluor-phenyl)-acetamidhydrochlorid (Beispiele 57 und 58)**

Eine Lösung des unpolareren Diastereoisomers von 2-(4-Dimethylamino-4-phenylcyclohexyl)-N-(4-fluor-phenyl)-acetamid (300 mg, 0,8 mmol) in Ethylmethylketon (2 ml) und etwas Methanol wurde mit Chlortrimethylsilan (0,118 ml) und Wasser (8 µl) versetzt. Nach dem Einengen konnte das Hydrochlorid in einer Ausbeute von 397 mg (quantitativ) isoliert werden (Beispiel 57).

Eine Lösung des polareren Diastereoisomers von 2-(4-Dimethylamino-4-phenylcyclohexyl)-N-(4-fluor-phenyl)-acetamid (310 mg, 0,9 mmol) in Ethylmethylketon (2 ml) wurde mit Chlortrimethylsilan (0,122 ml) und Wasser (9 µl) versetzt. Nach 18 h konnte das Hydrochlorid als Feststoff in einer Ausbeute von 64 % (220 mg; 0,6 mmol) isoliert werden (Beispiel 58).

**2-(4-Dimethylamino-4-phenyl-cyclohexyl)-N-p-tolyl-acetamid**

Bei 0°C wurde zu einer Lösung von p-Tolylamin (1,02 g; 9,5 mmol) in 10 ml THF n-Butyllithium (1,6M Lösung in Hexan; 11,9 ml; 19 mmol) getropft. Man ließ auf RT kommen und rührte 1 h nach. Bei -78°C wurde eine Lösung von (4-Dimethylamino-4-phenyl-cyclohexyl)-essigsäure-ethylester (2,5 g; 8,6 mmol) in 15 ml THF getropft. Es wurde 1 h

nach gerührt und dann auf RT kommen gelassen. Der Ansatz wurde mit Ammoniumchlorid-Lösung hydrolysiert und mit DCM extrahiert. Nach dem Einengen wurde das Rohprodukt in Ether aufgenommen. Dabei blieb ein Feststoff zurück (unpolareres Diastereoisomer; 1,518 g; 50 %); der Überstand wurde an Kieselgel chromatographiert (Eluens: Ether, dann Ether/Methanol 4:1). Man erhielt 142 mg (4,7%) des unpolareren und 1,20 g (40%) des polareren Diastereoisomers.

**2-(4-Dimethylamino-4-phenyl-cyclohexyl)-N-p-tolyl-acetamid-hydrochlorid (Beispiele 59 und 60)**

Eine Lösung des unpolareren Diastereoisomers von 2-(4-Dimethylamino-4-phenylcyclohexyl)-N-p-tolyl-acetamid (142 mg, 0,4 mmol) in Ethylmethylketon (1 ml) wurde mit Chlortrimethylsilan (0,056 ml) und Wasser (4 µl) versetzt. Nach kurzer Zeit fiel ein farbloser Feststoff aus und konnte in einer Ausbeute von 100 mg (63%) isoliert werden (Beispiel 59).
Eine Lösung des polareren Diastereoisomers von 2-(4-Dimethylamino-4-phenylcyclohexyl)-N-p-tolyl-acetamid (1,2 g, 3,4 mmol) in Ethylmethylketon (10 ml) wurde mit Chlortrimethylsilan (0,477 ml) und Wasser (34 µl) versetzt. Das Hydrochlorid wurde mit Hexan ausgeölt und in einer Ausbeute von 78 % (1,04 g; 2,7 mmol) isoliert (Beispiel 60).

**2-(4-Dimethylamino-4-phenyl-cyclohexyl)-N-(4-methoxy-phenyl)-acetamid**

Bei 0°C wurde zu einer Lösung von 4-Methoxy-phenylamin (430 mg; 3,5 mmol) in 5 ml THF n-Butyllithium (1,6M Lösung in Hexan; 4,3 ml; 7,0 mmol) getropft. Man ließ auf RT kommen und rührte 1 h nach. Bei -78°C wurde eine Lösung von trans-(4-Dimethylamino-4-phenyl-cyclohexyl)-essigsäure-ethylester (0,92 g; 3,2 mmol) in 5 ml THF getropft. Es wurde 1 h nach gerührt und dann auf RT kommen gelassen. Der Ansatz wurde mit Ammoniumchlorid-Lösung hydrolysiert und mit DCM extrahiert. Nach dem Einengen wurde an Kieselgel chromatographiert (Eluens: Ether, dann Ether/Methanol 4:1). Man erhielt 255 mg (22%) des unpolareren Diastereoisomers.

**2-(4-Dimethylamino-4-phenyl-cylclohexyl)-N-(4-methoxy-phenyl)-acetamidhydrochlorid (Beispiel 61)**

Eine Lösung des unpolareren Diastereoisomers von 2-(4-Dimethylamino-4-phenylcyclohexyl)-N-(4-methoxy-phenyl)-acetamid (255 mg, 0,7 mmol) in ,Ethylmethylketon (2 ml) wurde mit Chlortrimethylsilan (0,097 ml) und Wasser (7 µl) versetzt. Das Hydrochlorid wurde mit Hexan ausgeölt und in quantitativer Ausbeute isoliert

**Untersuchungen zur Wirksamkeit der erfindungsgemäßen Verbindungen:**

**<u>Messung der ORL1-Bindung</u>**

Die Cyclohexan-Derivate der allgemeinen Formel I wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$-Wert in oder % Inhibition bei c=1 µM angegeben.

**<u>Messung der µ-Bindung</u>**

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten Cyclohexylessigsäure-Derivate mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

**Messung der Serotonin-Wiederaufnahme**

Um diese in vitro Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus der Medulla + Pons-Region von männlichen Rattengehirnen isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

**Messung der Noradrenalin-Wiederaufnahme**

Um diese in vitro Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

Beispielhaft wurden die folgenden Daten bestimmt:

| Beispiel-Nummer | ORL1 % 1μm | ORL1 Ki | ORμNal % 1uM | ORμNal Ki |
|---|---|---|---|---|
| 1 | 98,00 | 0,0016 | 100.5 | 0.0005 |
| 2 | 97,00 | 0,0046 | 100.5 | 0.0012 |
| 3 | 94,00 | 0,0032 | 101,00 | 0,0014 |
| 4 | 98.5 | 0,0025 | 97.5 | 0.0014 |
| 6 | 90,00 | 0,0130 | 101,00 | 0,0031 |
| 7 | 84,00 | 0,0500 | 100,00 | 0,0082 |
| 8 | 85,00 | 0,0210 | 103,00 | 0,0011 |

(fortgesetzt)

| Beispiel-Nummer | ORL1 % 1μm | ORL1 Ki | ORμNal % 1uM | ORμNal Ki |
|---|---|---|---|---|
| **9** | 93,00 | 0,0110 | 96,00 | 0.0014 |
| **10** | 37,00 | - | 91.5 | 0.021 |
| **11** | 88,00 | 0,0360 | 99,00 | 0,0043 |
| **12** | 93,00 | 0,0170 | 105,00 | 0,0057 |
| **13** | 84,00 | 0,0530 | 104,00 | 0,0028 |
| **14** | 85 | 0,0270 | 96.5 | 0.0089 |
| **15** | 92 | 0,0057 | 101 | 0.0052 |
| **16** | 88 | 0,0360 | 98 | 0.0041 |
| **17** | 94 | 0,0170 | 100 | 0.008 |
| **18** | 90 | 0,0490 | 102 | 0.037 |
| **19** | 49 | - | 93 | 0.0075 |
| **20** | 81 | 0,0780 | 81 | 0.0052 |
| **21** | 88 | 0,0430 | 92 | 0.0013 |
| **22** | 83 | 0,0800 | 107 | 0.0021 |
| **23** | 92 | 0,0280 | 106 | 0.0027 |
| **24** | 46 | - | 83 | 0.0091 |
| **25** | 98 | 0,0029 | 97 | 0.0016 |
| **26** | 98 | 0,0023 | 90 | 0.0007 |
| **27** | 98 | 0,0026 | 96 | 0.0016 |
| **28** | 96 | 0,0110 | 102 | 0.0012 |
| **29** | 63,00 | - | 91.5 | 0.0059 |
| **30** | 98,00 | 0.001 | 91.5 | 0.0003 |
| **31** | 97,00 | 0.0026 | 96 | 0.0024 |
| **32** | 91,00 | 0.018 | 97 | 0.0026 |
| **33** | 95,00 | 0.0079 | 101 | 0.0009 |
| **34** | 94 | 0.0021 | 97 | 0.0028 |
| **35** | 72,00 | 0.059 | 95 | 0.017 |
| **36** | 66,00 | - | 93.5 | 0.0086 |
| **37** | 99,00 | 0.004 | 99 | 0.0013 |
| **38** | 88,00 | 0.021 | 97 | 0.0057 |
| **39** | 98,00 | 0.0033 | 104 | 0.0018 |
| **40** | 91,00 | 0.028 | 104 | 0.0098 |
| **41** | 98,00 | 0.0024 | 103 | 0.0009 |
| **42** | 56,00 | - | 107 | 0.0052 |
| **43** | 71,00 | 0.012 | 98 | 0.021 |
| **44** | 89,00 | 0.039 | 106 | 0.0012 |
| **45** | 65,00 | - | 114 | 0.0076 |

(fortgesetzt)

| Beispiel-Nummer | ORL1 % 1μm | ORL1 Ki | ORμNal % 1uM | ORμNal Ki |
|---|---|---|---|---|
| **46** | 84,00 | 0.093 | 66 | 0.0009 |
| **50** | 97,00 | 0.0058 | 102 | 0.002 |
| **51** | 98,00 | 0.0015 | 96 | 0.011 |
| **52** | 99,00 | 0.0024 | 99 | 0.0013 |
| **53** | 97,00 | 0.0067 | 106 | 0.0014 |
| **54** | 99,00 | 0.0021 | 107 | 0.0017 |

| Beispiel-Nummer | 5HT-Uptake % (10) | NA-Uptake % (10) |
|---|---|---|
| **1** | 96 | 102 |
| **2** | 90 | 78.5 |
| **3** | 94 | 72 |
| **4** | 92 | 101 |
| **6** | 101 | 102 |
| **7** | 89 | 64 |
| **8** | 97 | 93 |
| **9** | 93 | 94 |
| **10** | 92.5 | 84.5 |
| **11** | 92 | 90 |
| **12** | 90 | 85 |
| **13** | 95 | 98 |
| **14** | 95 | 79.5 |
| **15** | 93 | 96 |
| **16** | 91 | 95 |
| **17** | 91 | 94 |
| **18** | 91 | 91 |
| **19** | 88 | 101 |
| **20** | 92 | 101 |
| **21** | 84 | 99 |
| **22** | 84 | 95 |
| **23** | 89 | 92 |
| **24** | 82 | 87 |
| **25** | 91 | 96 |
| **26** | 95 | 81 |
| **27** | 96 | 88 |
| **28** | 96 | 94 |
| **29** | 90 | 52.5 |

(fortgesetzt)

| Beispiel-Nummer | 5HT-Uptake % (10) | NA-Uptake % (10) |
|---|---|---|
| **30** | 85 | 78.5 |
| **31** | 83 | 95 |
| **32** | 76 | 44 |
| **33** | 81 | 86 |
| **34** | 77 | 57 |
| **36** | 94.5 | 50.5 |
| **37** | 99 | 83 |
| **38** | 92 | 92 |
| **39** | 96 | 89 |
| **40** | 93 | 84 |
| **41** | 83 | 55 |
| **42** | 90 | 97 |
| **43** | 76 | 100 |
| **44** | 92 | 69 |
| **45** | 87 | 90 |
| **46** | 87 | 62 |
| **47** | 46 | 63 |
| **48** | 85 | 74 |
| **49** | 32 | 94 |
| **50** | 86 | 94 |
| **51** | 72 | 67 |
| **52** | 96 | 57 |
| **53** | 94 | 96 |
| **54** | 92 | 90 |

| Beispiel-Nummer | TFMiv % [1 mg/kg] |
|---|---|
| **2** | 62 |
| **4** | 69 |
| **8** | 93 |
| **9** | 74 |
| **10** | 100 (10 mg/kg) |
| **11** | 81 |
| **13** | 99 |
| **25** | 42 |
| **30** | 90 (10 mg/kg) |
| **32** | 93 (10 mg/kg) |
| **34** | 89 (10 mg/kg) |

**Parenterale Lösung eines erfindungsgemäßen substituierten Cyclohexylessigsäure-Derivats**

38 g eines der erfindungsgemäßen substituierten Cyclohexylessigsäure-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte Cyclohexylessigsäure-Derivate-Derivate der allgemeinen Formel I,

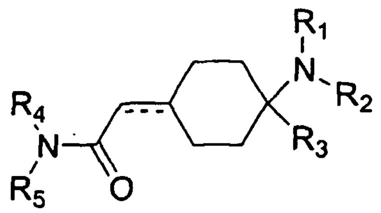

I

, worin

------

für eine C-C-Einfachbindung oder-Doppelbindung steht,

$R^1$ und $R^2$, unabhängig voneinander für H; CHO; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{10}CH_2CH_2$ oder $(CH_2)_{3-6}$ stehen, wobei $R^{10}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

$R^3$ für $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; über $C_{1-3}$-Alkyl-Gruppe gebundenes Aryl, Heteroaryl oder $C_{3-8}$-Cycloalkyl, jeweils unsubstituiert oder einfach oder mehrfach substituiert; Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-*tert.*-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl, steht;

$R^4$ für für-$(CR^6R^7)_nR^8$ steht,

wobei n 0, 1, 2, 3, 4, 5 oder 6 bedeutet

$R^6$ H oder $C_{1-5}$-Alkyl, verzweigt oder unverzweigt, gesättigt oder ungesättigt, unsubstituiert oder einfach oder mehrfach substituiert, bedeutet

$R^7$ H, $C_{1-5}$-Alkyl, verzweigt oder unverzweigt, gesättigt oder ungesättigt, unsubstituiert oder einfach oder mehrfach substituiert, oder $COOR^9$ bedeutet,

oder $R^6$ und $R^7$ einen Ring $(CH_2)_kCHR^8(CH_2)_m$ bilden, mit k = 1, 2, 3 und m=1,2

$R^8$ $C_{3-8}$-Cycloalkyl, Aryl oder Heteroaryl, jeweils unsubstituiert oder einfach oder mehfach substituiert, bedeutet, $R^9$ H oder $C_{1-5}$-Alkyl, verzweigt oder unverzweigt, gesättigt oder ungesättigt, unsubstituiert oder einfach oder mehrfach substituiert, bedeutet
$R^5$ für H oder für-$(CH_2)_lR^8$ steht,
wobei I für 1, 2 oder 3 steht,
oder zusammen mit $R^4$ für $CH_2CH_2OCH_2CH_2$, $CH_2CH_2NR^{11}CH_2CH_2$ oder $(CH_2)_{3-6}$ stehen,
wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte Cyclohexylessigsäure-Derivate gemäß Anspruch 1, **dadurch gekennzeichnet, dass** $R^1$ und $R^2$ unabhängig voneinander für H; $C_{1-5}$-Alkyl, gesättigt oder ungesättigt, verzweigt oder unverzweigt, einfach oder mehrfach substituiert oder unsubstituiert, stehen;
oder die Reste $R^1$ und $R^2$ zusammen einen Ring bilden und $CH_2CH_2OCH_2CH$ $CH_2CH_2NR^{10}CH_2CH_2$ oder $(CH_2)_{3-6}$ bedeuten,
wobei $R^{10}$ H; $C_{1-5}$-Alkyl, gesättigt oder ungesättigt, verzweigt oder unverzweigt, einfach oder mehrfach substituiert oder unsubstituiert, bedeutet.

3. Substituierte Cyclohexylessigsäure-Derivate gemäß Anspruch 1, **dadurch gekennzeichnet, dass** dass $R^1$ und $R^2$ unabhängig voneinander für $CH_3$ oder H stehen, wobei $R^1$ und $R^2$ nicht gleichzeitig H bedeuten.

4. Substituierte Cyclohexylessigsäure -Derivate gemäß einem der Ansprüche 1 bis 3, **dadurch gekennzeichnet, dass** $R^3$ für Cyclopentyl, Cyclohexyl, Naphthyl, Thiophenyl, Benzothiophenyl, Furyl, Benzofuranyl, Benzodioxolanyl, Indolyl, Indanyl, Benzodioxanyl oder Pyridyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-*tert.*-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl steht;
insbesondere
$R^3$ für Naphthyl, Thiophenyl oder Pyridyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Thiophenyl, Pyridyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-*tert.*-Butylphenyl, 4-Fluor-3-chlorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl oder 4-Chlor-3-trifluormethyl, steht.

5. Substituierte Cyclohexylessigsäure-Derivate gemäß einem der Ansprüche 1-3, **dadurch gekennzeichnet, dass**

$R^3$ für Pyridyl, Phenyl, 3-Fluorphenyl oder 4-Fluorphenyl steht.

6. Substituierte Cyclohexylessigsäure-Derivate gemäß einem der Ansprüche 1-5, **dadurch gekennzeichnet, dass** $R^6$ H und $R^7$ H, $CH_3$ oder $COOR^9$ bedeutet oder $R^6$ und $R^7$ einen Ring $(CH_2)_kCHR^8(CH_2)_m$ bilden mit k = 1, 2 oder 3 und m = 1 oder 2.

7. Substituierte Cyclohexylessigsäure-Derivate gemäß einem der Ansprüche 1-6, **dadurch gekennzeichnet, dass** $R^5$ H bedeutet.

8. Substituierte Cyclohexylessigsäure-Derivate gemäß einem der Ansprüche 1-7, **dadurch gekennzeichnet, dass** $R^8$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet,
insbesondere
$R^8$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

9. Substituierte Cyclohexylessigsäure-Derivate gemäß einem der Ansprüche 1-7, **dadurch gekennzeichnet, dass** $R^8$ Phenyl oder Indolyl, jeweils einfach oder mehrfach substituiert, bedeutet.

10. Substituierte Cyclohexylessigsäure-Derivate gemäß einem der Ansprüche 1-9
aus der Gruppe
2-(4-Dimethylamino-4-phenyl-cyclohexyliden)-N-[2-(1H-indol-3-yl)-ethyl]-acetamid; hydrochlorid
2-[2-(4-Dimethylamino-4-phenyl-cyclohexyliden)-acetylamino]-3-(1H-indol-3-yl)-propansäure methylester; hydrochlorid
2-(4-Dimethylamino-4-phenyl-cyclohexyliden)-N-(3-phenyl-propyl)-acetamid; hydrochlorid
N-Benzyl-2-(4-dimethylamino-4-phenyl-cyclohexyliden)-acetamid; hydrochlorid 2-[2-(4-Dimethylamino-4-phenyl-cyclohexyliden)-acetylamino]-3-(1H-indol-3-yl)-propansäure methylester; hydrochlorid
2-(4-Dimethylamino-4-phenyl-cyclohexyliden)-N-phenethyl-acetamid; hydrochlorid
2-(4-Dimethylamino-4-phenyl-cyclohexyliden)-N-[2-(4-fluoro-phenyl)-ethyl]-acetamid; hydrochlorid
2-(4-Dimethylamino-4-phenyl-cyclohexyliden)-N-(4-fluoro-benzyl)-acetamid; hydrochlorid
2-(4-Dimethylamino-4-phenyl-cyclohexyliden)-N-(2-trifluoromethyl-benzyl)-acetamid; hydrochlorid
2-(4-Dimethylamino-4-phenyl-cyclohexyliden)-N-(4-fluoro-phenyl)-acetamid; hydrochlorid
2-(4-Dimethylamino-4-phenyl-cyclohexyliden)-N-[2-(1H-indol-3-yl)-1-methyl-ethyl]-acetamid; hydrochlorid
2-(4-Dimethylamino-4-phenyl-cyclohexyliden)-N-(4-phenyl-butyl)-acetamid; hydrochlorid
2-(4-Dimethylamino-4-phenyl-cyclohexyliden)-N-(1H-indol-3-ylmethyl)-acetamid; hydrochlorid
2-(4-Dimethylamino-4-phenyl-cyclohexyliden)-N-[4-(1H-indol-3-yl)-butyl]-acetamid; hydrochlorid
2-(4-Dimethylamino-4-phenyl-cyclohexyliden)-N-[3-(1H-indol-3-yl)-propyl]-acetamid; hydrochlorid
2-(4-Dimethylamino-4-phenyl-cyclohexyliden)-N-[5-(1H-indol-3-yl)-pentyl]-acetamid; hydrochlorid
2-(4-Dimethylamino-4-phenyl-cyclohexyliden)-N-[6-(1H-indol-3-yl)-hexyl]-acetamid; hydrochlorid
2-[4-Dimethylamino-4-(4-fluoro-phenyl)-cyclohexyliden]-N-(3-phenyl-propyl)-acetamid; hydrochlorid
2-[4-Dimethylamino-4-(4-fluoro-phenyl)-cyclohexyliden]-N-[2-(1H-indol-3-yl)-ethyl]-acetamid; hydrochlorid
2-[4-Dimethylamino-4-(4-fluoro-phenyl)-cyclohexyliden]-N-[2-(1H-indol-3-yl)-1-methyl-ethyl]-acetamid; hydrochlorid
2-(4-Dimethylamino-4-pyridin-2-yl-cyclohexyliden)-N-[2-(1H-indol-3-yl)-ethyl]-acetamid; hydrochlorid
2-(4-Dimethylamino-4-pyridin-2-yl-cyclohexyliden)-N-(3-phenyl-propyl)-acetamid; hydrochlorid
2-[4-Dimethylamino-4-(3-fluoro-phenyl)-cyclohexyliden]-N-[2-(1H-indol-3-yl)-ethyl]-acetamid; hydrochlorid
2-[4-Dimethylamino-4-(3-fluoro-phenyl)-cyclohexyliden]-N-(3-phenyl-propyl)-acetamid; hydrochlorid
2-[4-Dimethylamino-4-(3-fluoro-phenyl)-cyclohexyliden]-N-[2-(1H-indol-3-yl)-1-methyl-ethyl]-acetamid; hydrochlorid
2-(4-Dimethylamino-4-phenylcyclohexyl)-N-(2-trifluoromethyl-benzyl)-acetamid; hydrochlorid
2-(4-Dimethylamino-4-phenylcyclohexyl)-N-[2-(1H-indol-3-yl)-ethyl]-acetamid; hydrochlorid
2-(4-Dimethylamino-4-phenylcyclohexyl)-N-(3-phenylpropyl)-acetamid; hydrochlorid
2-(4-Dimethylamino-4-phenylcyclohexyl)-N-phenethylacetamid; hydrochlorid

2-(4-Dimethylamino-4-phenylcyclohexyl)-N-(4-phenylbutyl)-acetamid; hydrochlorid
2-[2-(4-Dimethylamino-4-phenylcyclohexyl)-acetylamino]-3-(1H-indol-3-yl)-propansäure-methylester; hydrochlorid
Kalium; 2-[2-(4-dimethylamino-4-phenylcyclohexyl)-acetylamino]-3-(1H-indol-3-yl)-propanat
2-(4-Dimethylamino-4-phenylcyclohexyl)-N-(1H-indol-3-ylmethyl)-acetamid; hydrochlorid
2-(4-Dimethylamino-4-phenylcyclohexyl)-N-[4-(1H-indol-3-yl)-butyl]-acetamid; hydrochlorid
2-(4-Dimethylamino-4-phenylcyclohexyl)-N-[3-(1H-indol-3-yl)-propyl]-acetamid; hydrochlorid
2-(4-Dimethylamino-4-phenylcyclohexyl)-N-[5-(1H-indol-3-yl)-pentyl]-acetamid; hydrochlorid
2-(4-Dimethylamino-4-phenylcyclohexyl)-N-[2-(1H-indol-3-yl)-1-methylethyl]-acetamid; hydrochlorid
2-[4-Dimethylamino-4-(4-fluorphenyl)-cyclohexyl]-N-(3-phenylpropyl)-acetamid; hydrochlorid
2-(4-Dimethylamino-4-phenylcyclohexyl)-N-[6-(1H-indol-3-yl)-hexyl]-acetamid; hydrochlorid
2-[4-Dimethylamino-4-(4-fluorphenyl)-cyclohexyl]-N-[2-(1H-indol-3-yl)-ethyl]-acetamid; hydrochlorid
2-[4-Dimethylamino-4-(4-fluorphenyl)-cyclohexyl]-N-[2-(1H-indol-3-yl)-1-methylethyl]-acetamid; hydrochlorid
2-(4-Dimethylamino-4-pyridin-2-yl-cyclohexyl)-N-(3-phenylpropyl)-acetamid; hydrochlorid
2-(4-Dimethylamino-4-pyridin-2-yl-cyclohexyl)-N-[2-(1H-indol-3-yl)-ethyl]-acetamid; hydrochlorid
2-[4-Dimethylamino-4-(3-fluorphenyl)-cyclohexyl]-N-[2-(1H-indol-3-yl)-ethyl]-acetamid; hydrochlorid
2-[4-Dimethylamino-4-(3-fluorphenyl)-cyclohexyl]-N-(3-phenylpropyl)-acetamid; hydrochlorid
2-[4-Dimethylamino-4-(3-fluorphenyl)-cyclohexyl]-N-[2-(1H-indol-3-yl)-1-methylethyl]-acetamid; hydrochlorid
2-(4-Dimethylamino-4-phenyl-cyclohexyl)-N-phenyl-acetamid-hydrochlorid
2-(4-Dimethylamino-4-phenyl-cyclohexyl)-N-(4-fluor-phenyl)-acetamid-hydrochlorid
2-(4-Dimethylamino-4-phenyl-cyclohexyl)-N-p-tolyl-acetamid-hydrochlorid
2-(4-Dimethylamino-4-phenyl-cyclohexyl)-N-(4-methoxy-phenyl)-acetamid-hydrochloride
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

**11.** Verfahren zur Herstellung von substituierten Cyclohexylessigsäure-Derivaten gemäß einem der Ansprüche 1-10, **dadurch gekennzeichnet, dass**

(i) Phosphonoessigsäureester in Gegenwart einer starken Base, vorzugsweise Kalium-tert.butylat, Natriumhydrid oder Butyllithium, mit Aminocyclohexanon-Derivaten umgesetzt werden, und die Produkte nach Esterhydrolyse unter Einsatz von wasserentziehenden Reagenzien, vorzugsweise einem Carbodiimid, mit Aminen der Formel $R_4R_5NH$ zu den korrespondierenden erfindungsgemäßen Verbindungen gemäß der Formel Ia

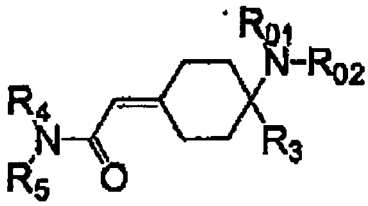

Ia

umgesetzt werden, die gegebenenfalls zu erfindungsgemäßen Verbindungen gemäß Formel Ib

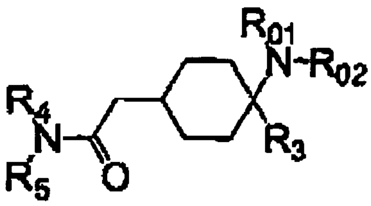

Ib

reduziert werden, vorzugsweise durch heterogene, katalytische Hydrierung an Palladium- oder Platin-Katalysatoren oder durch homogen katalysierte Hydrierung mit Rhodium-Katalysatoren; oder
(ii) der Phosphonoessigsäureester in Gegenwart einer starken Base; vorzugsweise nBuLi, und einem Anilin

direkt zu den erfindungsgemäßen Verbindungen gemäß Formel Ic

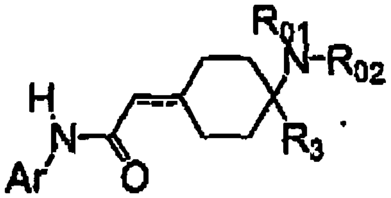

Ic

umgesetzt werden; oder (iii) Phosphonoessigsäureester in Gegenwart einer starken Base, vorzugsweise Kalium-tert.butylat, Natriumhydrid oder Butyllithium, mit Cyclohexan-1,4-dion-Derivaten umgesetzt werden und die Produkte nach Esterhydrolyse unter Einsatz von wasserentziehenden Reagenzien, vorzugsweise einem Carbodiimid, mit Aminen der Formel $R_4R_5NH$ umgesetzt und anschließend in Gegenwart von $HNR_{01}R_{02}$ mit einem Cyanid, vorzugsweise Kaliumcyanid oder TMSCN umgesetzt, und schließlich mit metallorganischen Reagenzien, bevorzugt Grignard- oder Organolithiumreagenzien der Formel Metall-$R_3$ zu den erfindungsgemäßen Verbindungen gemäß Formel Ia

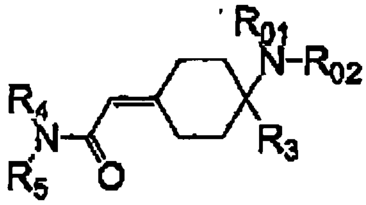

Ia

umgesetzt werden, die gegebenenfalls zu erfindungsgemäßen Verbindungen gemäß Formel Ib

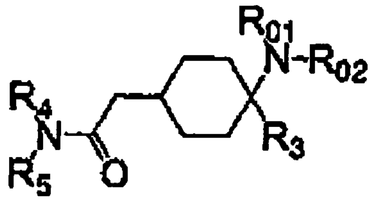

Ib

reduziert werden, vorzugsweise durch heterogene, katalytische Hydrierung an Palladium- oder Platin-Katalysatoren oder durch homogen katalysierte Hydrierung mit Rhodium-Katalysatoren.

**12.** Verfahren zur Herstellung von substituierten Cyclohexylessigsäure-Derivaten gemäß Anspruch 11, Alternative (i), **dadurch gekennzeichnet, dass** im Anschluss an die Umsetzung mit einem Phosphonoessigsäureester, die Doppelbindung reduziert wird, vorzugsweise durch heterogene, katalytische Hydrierung an Palladium- oder Platin-Katalysatoren oder durch homogen katalysierte Hydrierung mit Rhodium-Katalysatoren.

**13.** Verfahren zur Herstellung von substituierten Cyclohexylessigsäure-Derivaten gemäß Anspruch 11, Alternative (iii), **dadurch gekennzeichnet, dass** im Anschluss an die Umsetzung mit Aminen der Formel $R_4R_5NH$ die Doppelbindung reduziert wird, vorzugsweise durch heterogene, katalytische Hydrierung an Palladium- oder Platin-Katalysatoren oder durch homogen katalysierte Hydrierung mit Rhodium-Katalysatoren.

**14.** Arzneimittel enthaltend mindestens ein substituiertes Cyclohexylessigsäure-Derivat gemäß einem der Ansprüche 1 bis 10 sowie gegebenenfalls geeignete Zusatz- und/oder Hilfsstoffe und/oder gegebenenfalls weitere Wirkstoffe.

**15.** Verwendung eines substituierten Cyclohexylessigsäure-Derivats gemäß einem der Ansprüche 1 bis 10 zur Herstellung eines Arzneimittels zur Behandlung von Schmerz, insbesondere von akutem, viszeralem, neuropathischem oder chronischem Schmerz; oder zur Behandlung von Angstzuständen, von Stress und mit Stress verbundenen Syndromen, Depressionen, Katalepsie, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder - abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

**1.** Substituted cyclohexylacetic acid derivatives of the general formula I

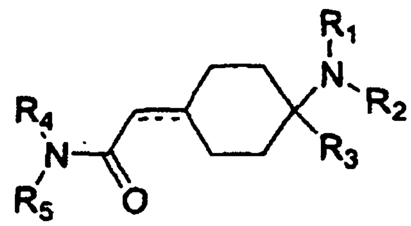

I

wherein
---------- stands for a single or double C-C bond,
$R^1$ and $R^2$, independently of one another, stand for H; CHO; $C_{1-5}$ alkyl, respectively saturated or unsaturated, branched or unbranched, mono- or polysubstituted or unsubstituted; $C_{3-8}$ cycloalkyl, respectively saturated or unsaturated, mono- or polysubstituted or unsubstituted; or aryl, $C_{3-8}$ cycloalkyl or heteroaryl bonded via $C_{1-3}$ alkyl, respectively mono- or polysubstituted or unsubstituted;
or the residues $R^1$ and $R^2$ together stand for $CH_2CH_2OCH_2CH_2$, $CH_2CH_2NR^{10}CH_2CH_2$ or $(CH_2)_{3-6}$,
wherein $R^{10}$ represents H; $C_{1-5}$ alkyl, respectively saturated or unsaturated, branched or unbranched, mono- or polysubstituted or unsubstituted; $C_{3-8}$ cycloalkyl, respectively saturated or unsaturated, mono- or polysubstituted or unsubstituted; aryl or heteroaryl, respectively mono- or polysubstituted or unsubstituted; or aryl, $C_{3-8}$ cycloalkyl or heteroaryl bonded via $C_{1-3}$ alkyl, respectively mono- or polysubstituted or unsubstituted;
$R^3$ stands for $C_{1-5}$ alkyl, respectively saturated or unsaturated, branched or unbranched, mono- or polysubstituted or unsubstituted; $C_{3-8}$ cycloalkyl, respectively saturated or unsaturated, mono- or polysubstituted or unsubstituted; aryl, heteroaryl or $C_{3-8}$ cycloalkyl bonded via $C_{1-3}$ alkyl group, respectively unsubstituted or mono- or polysubstituted; naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl, respectively unsubstituted or mono- or polysubstituted; phenyl, 2-fluorophenyl, 3-fluorophenyl, 4-fluorophenyl, 2-chlorophenyl, 4-chlorophenyl, 3-chlorophenyl, 2-bromophenyl, 3-bromophenyl, 4-bromophenyl, 2-cyanophenyl, 3-cyanophenyl, 4-cyanophenyl, 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 4-trifluoromethylphenyl, 2-ethylphenyl, 3-ethylphenyl, 4-ethylphenyl, 2-ethoxyphenyl, 3-ethoxyphenyl, 4-ethoxyphenyl, 2-hydroxyphenyl, 3-hydroxyphenyl, 4-hydroxyphenyl, 2,3-dichlorophenyl, 3,4-dichlorophenyl, 3,5-dichlorophenyl, 2,4-dichlorophenyl, 2,3-difluorophenyl, 3,4-difluorophenyl, 3,5-difluorophenyl, 2,4-difluorophenyl, 2-fluoro-3-chlorophenyl, 2-chloro-3-fluorophenyl, 2-chloro-4-fluorophenyl, 2-fluoro-4-chlorophenyl, 4-fluoro-3-chlorophenyl, 4-fluoro-3-methylphenyl, 4-tert-butylphenyl, 4-fluoro-3-chlorophenyl, 4-bromo-3-fluorophenyl, 3,5-bis(trifluoromethyl)phenyl, 4-chloro-2-trifluoromethylphenyl, 2-methoxy-5-methylphenyl, 5-chloro-2-methoxyphenyl, 4-phenoxyphenyl, 2-methylthiophenyl, 3-methylthiophenyl, 4-methylthiophenyl, 5-fluoro-2-methoxyphenyl, 4-chloro-3-trifluoromethyl or 4-bromo-2-methylphenyl;

$R^4$ stands for $-(CR^6R^7)_nR^8$,
wherein n represents 0, 1, 2, 3, 4, 5 or 6
$R^6$ represents H or $C_{1-5}$ alkyl, branched or unbranched, saturated or unsaturated, unsubstituted or mono- or polysubstituted
$R^7$ represents H, $C_{1-5}$ alkyl, branched or unbranched, saturated or unsaturated, unsubstituted or mono- or polysubstituted, or $COOR^9$,
or $R^6$ and $R^7$ form a $(CH_2)_kCHR^8(CH_2)_m$ ring, where k = 1, 2, 3 and m = 1, 2 $R^8$ represents $C_{3-8}$ cycloalkyl, aryl or heteroaryl, respectively unsubstituted or mono- or polysubstituted,
$R^9$ represents H or $C_{1-5}$ alkyl, branched or unbranched, saturated or unsaturated, unsubstituted or mono- or polysubstituted
$R^5$ stands for H or for $-(CH_2)_lR^8$,
wherein l stands for 1, 2 or 3,
or together with $R^4$ stands for $CH_2CH_2OCH_2CH_2$, $CH_2CH_2NR^{11}CH_2CH_2$ or $(CH_2)_{3-6}$, wherein $R^{11}$ represents H, $C_{1-5}$ alkyl, respectively saturated or unsaturated, branched or unbranched, mono- or polysubstituted or unsubstituted; $C_{3-8}$ cycloalkyl, respectively saturated or unsaturated, mono- or polysubstituted or unsubstituted; aryl or heteroaryl, respectively mono- or polysubstituted or unsubstituted; or aryl, $C_{3-8}$ cycloalkyl or heteroaryl bonded via $C_{1-3}$ alkyl, respectively mono- or polysubstituted or unsubstituted;
in the form of the racemate; enantiomers, diastereomers, mixtures of enantiomers or diastereomers or a single enantiomer or diastereomer; of bases and/or acids of physiologically compatible acids or cations.

2. Substituted cyclohexylacetic acid derivatives according to claim 1, **characterised in that** $R^1$ and $R^2$, independently of one another, stand for H; $C_{1-5}$ alkyl, saturated or unsaturated, branched or unbranched, mono- or polysubstituted or unsubstituted;
or the residues $R^1$ and $R^2$ together form a ring and represent $CH_2CH_2OCH_2CH_2$, $CH_2CH_2NR^{10}CH_2CH_2$ or $(CH_2)_{3-6}$, wherein $R^{10}$ represents H; $C_{1-5}$ alkyl, saturated or unsaturated, branched or unbranched, mono- or polysubstituted or unsubstituted.

3. Substituted cyclohexylacetic acid derivatives according to claim 1, **characterised in that** $R^1$ and $R^2$, independently of one another, stand for $CH_3$ or H, wherein $R^1$ and $R^2$ do not simultaneously represent H.

4. Substituted cyclohexylacetic acid derivatives according to one of claims 1 to 3, **characterised in that** $R^3$ stands for cyclopentyl, cyclohexyl, naphthyl, thiophenyl, benzothiophenyl, furyl, benzofuranyl, benzodioxolanyl,indolyl, indanyl, benzodioxanyl or pyridyl, respectively unsubstituted or mono- or polysubstituted; $C_{5-6}$ cycloalkyl, phenyl, naphthyl, antracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, respectively unsubstituted or mono- or polysubstituted, bonded via a saturated, unbranched $C_{1-2}$ alkyl group; phenyl, 2-fluorophenyl, 3-fluorophenyl, 4-fluorophenyl, 2-chlorophenyl, 4-chlorophenyl, 3-chlorophenyl, 2-bromophenyl, 3-bromophenyl, 4-bromophenyl, 2-cyanophenyl, 3-cyanophenyl, 4-cyanophenyl, 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 4-trifluoromethylphenyl, 2-ethylphenyl, 3-ethylphenyl, 4-ethylphenyl, 2-ethoxyphenyl, 3-ethoxyphenyl, 4-ethoxyphenyl, 2-hydroxyphenyl, 3-hydroxyphenyl, 4-hydroxyphenyl, 2,3-dichlorophenyl, 3,4-dichlorophenyl, 3,5-dichlorophenyl, 2,4-dichlorophenyl, 2,3-difluorophenyl, 3,4-difluorophenyl, 3,5-difluorophenyl, 2,4-difluorophenyl, 2-fluoro-3-chlorophenyl, 2-chloro-3-fluorophenyl, 2-chloro-4-fluorophenyl, 2-fluoro-4-chlorophenyl, 4-fluoro-3-chlorophenyl, 4-fluoro-3-methylphenyl, 4-tert-butylphenyl, 4-fluoro-3-chlorophenyl, 4-bromo-3-fluorophenyl, 3,5-bis(trifluoromethyl)phenyl, 4-chloro-2-trifluoromethylphenyl, 2-methoxy-5-methylphenyl, 5-chloro-2-methoxyphenyl, 4-phenoxyphenyl, 2-methylthiophenyl, 3-methylthiophenyl, 4-methylthiophenyl, 5-fluoro-2-methoxyphenyl, 4-chloro-3-trifluoromethyl or 4-bromo-2-methylphenyl;
in particular
$R^3$ stands for naphthyl, thiophenyl or pyridyl, respectively unsubstituted or mono- or polysubstituted; $C_{5-6}$ cycloalkyl, phenyl, naphthyl, thiophenyl, pyridyl, respectively unsubstituted or mono- or polysubstituted, bonded via a saturated, unbranched $C_{1-2}$ alkyl group; phenyl, 2-fluorophenyl, 3-fluorophenyl, 4-fluorophenyl, 2-chlorophenyl, 4-chlorophenyl, 3-chlorophenyl, 2-cyanophenyl, 3-cyanophenyl, 4-cyanophenyl, 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 4-trifluoromethylphenyl, 2-ethylphenyl, 3-ethylphenyl, 4-ethylphenyl, 2-ethoxyphenyl, 3-ethoxyphenyl, 4-ethoxyphenyl, 2,3-dichlorophenyl, 3,4-dichlorophenyl, 3,5-dichlorophenyl, 2,4-dichlorophenyl, 2,3-difluorophenyl, 3,4-difluorophenyl, 3,5-difluorophenyl, 2,4-difluorophenyl, 2-fluoro-3-chlorophenyl, 2-chloro-3-fluorophenyl, 2-chloro-4-fluorophenyl, 2-fluoro-4-chlorophenyl, 4-fluoro-3-chlorophenyl, 4-fluoro-3-methylphenyl, 4-tert-butylphenyl, 4-fluoro-3-chlorophenyl, 3,5-bis(trifluoromethyl)phenyl, 4-chloro-2-trifluoromethylphenyl, 2-methoxy-5-methylphenyl, 5-chloro-2-methoxyphenyl, 4-phenoxyphenyl, 2-methylthiophenyl, 3-methylthiophenyl, 4-methylthiophenyl or 4-chloro-3-trifluoromethyl.

5. Substituted cyclohexylacetic acid derivatives according to one of claims 1-3, **characterised in that** $R^3$ stands for pyridyl, phenyl, 3-fluorophenyl or 4-fluorophenyl.

6. Substituted cyclohexylacetic acid derivatives according to one of claims 1-5, **characterised in that** $R^6$ represents H, $R^7$ represents H, $CH_3$ or $COOR^9$ or $R^6$ and $R^7$ form a $(CH_2)_kCHR^8(CH_2)_m$ ring, where k = 1, 2 or 3 and m = 1 or 2.

7. Substituted cyclohexylacetic acid derivatives according to one of claims 1-6, **characterised in that** $R^5$ represents H.

8. Substituted cyclohexylacetic acid derivatives according to one of claims 1-7, **characterised in that** $R^8$ represents cyclobutyl, cyclopropyl, cyclopenyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluoroenyl, fluoroanthenyl, benzothiazolyl, benzotriazolyl or benzo[1,3,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, respectively unsubstituted or mono- or polysubstituted,
in particular
$R^8$ represents cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, respectively unsubstituted or mono- or polysubstituted.

9. Substituted cyclohexylacetic acid derivatives according to one of claims 1-7, **characterised in that** $R^8$ represents phenyl or indolyl, respectively mono- or polysubstituted.

10. Substituted cyclohexylacetic acid derivatives according to one of claims 1-9, from the group
2-(4-dimethylamino-4-phenyl-cyclohexylidene)-N-[2-(1H-indol-3-yl)-ethyl] acetamide; hydrochloride
2-[2-(4-dimethylamino-4-phenyl-cyclohexylidene)-acetylamino]-3-(1H-indol-3-yl) methyl propionate; hydrochloride
2-(4-dimethylamino-4-phenyl-cyclohexylidene)-N-(3-phenyl-propyl) acetamide; hydrochloride
N-benzyl-2-(4-dimethylamino-4-phenyl-cyclohexylidene) acetamide; hydrochloride 2-[2-(4-dimethylamino-4-phenyl-cyclohexylidene)-acetylamino]-3-(1H-indol-3-yl) methyl propionate; hydrochloride 2-(4-dimethylamino-4-phenyl-cyclohexylidene)-N-phenethyl acetamide; hydrochloride 2-(4-dimethylamino-4-phenyl-cyclohexylidene)-N-[2-(4-fluoro-phenyl)-ethyl] acetamide; hydrochloride 2-(4-dimethylamino-4-phenyl-cyclohexylidene)-N-(4-fluoro-benzyl) acetamide; hydrochloride 2-(4-dimethylamino-4-phenyl-cyclohexylidene)-N-(2-trifluoromethyl-benzyl) acetamide; hydrochloride 2-(4-dimethylamino-4-phenyl-cyclohexylidene)-N-(4-fluoro-phenyl) acetamide; hydrochloride 2-(4-dimethylamino-4-phenyl-cyclohexylidene)-N-[2-(1H-indol-3-yl)-1-methyl-ethyl] acetamide; hydrochloride 2-(4-dimethylamino-4-phenyl-cyclohexylidene)-N-(4-phenyl-butyl) acetamide; hydrochloride
2-(4-dimethylamino-4-phenyl-cyclohexylidene)-N-(1H-indol-3-ylmethyl) acetamide; hydrochloride
2-(4-dimethylamino-4-phenyl-cyclohexylidene)-N-[4-(1H-indol-3-yl)-butyl] acetamide; hydrochloride 2-(4-dimethylamino-4-phenyl-cyclohexylidene)-N-[3-(1H-indol-3-yl)-propyl] acetamide; hydrochloride
2-(4-dimethylamino-4-phenyl-cyclohexylidene)-N-[5-(1H-indol-3-yl)-pentyl] acetamide; hydrochloride 2-(4-dimethylamino-4-phenyl-cyclohexylidene)-N-[6-(1H-indol-3-yl)-hexyl] acetamide; hydrochloride 2-[4-dimethylamino-4-(4-fluoro-phenyl)-cyclohexylidene]-N-(3-phenyl-propyl) acetamide; hydrochloride
2-[4-dimethylamino-4-(4-fluoro-phenyl)-cyclohexylidene]-N-[2-(1H-indol-3-yl)-ethyl] acetamide; hydrochloride 2-[4-dimethylamino-4-(4-fluoro-phenyl)-cyclohexylidene]-N-[2-(1H-indol-3-yl)-1-methyl-ethyl] acetamide; hydrochloride 2-(4-dimethylamino-4-pydridin-2-yl-cyclohexylidene)-N-[2-(1H-indol-3-yl)-ethyl] acetamide; hydrochloride 2-(4-dimethylamino-4-pydridin-2-yl-cyclohexylidene)-N-(3-phenyl-propyl) acetamide; hydrochloride 2-[4-dimethylamino-4-(3-fluoro-phenyl)-cyclohexylidene]-N-[2-(1H-indol-3-yl)-ethyl] acetamide; hydrochloride 2-[4-dimethylamino-4-(3-fluoro-phenyl)-cyclohexylidene]-N-(3-phenyl-propyl) acetamide; hydrochloride 2-[4-dimethylamino-4-(3-fluoro-phenyl)-cyclohexylidene]-N-[2-(1H-indol-3-yl)-1-methyl- ethyl] acetamide; hydrochloride 2-(4-dimethylamino-4-phenyl-cyclohexyl)-N-(2-trifluoromethyl-benzyl) acetamide; hydrochloride 2-(4-dimethylamino-4-phenylcyclohexyl)-N-[2-(1H-indol-3-yl)-ethyl] acetamide; hydrochloride
2-(4-dimethylamino-4-phenylcyclohexyl)-N-(3-phenylpropyl) acetamide; hydrochloride
2-(4-dimethylamino-4-phenylcyclohexyl)-N-phenethyl acetamide; hydrochloride 2-(4-dimethylamino-4-phenylcyclohexyl)-N-(4-phenylbutyl) acetamide; hydrochloride 2-[2-(4-dimethylamino-4-phenylcyclohexyl)-acetylamino]-3-(1H-indol-3-yl) methyl propionate; hydrochloride
potassium 2-[2-(4-dimethylamino-4-phenylcyclohexyl)-acetylamino]-3-(1H-indol-3-yl) propionate
2-(4-dimethylamino-4-phenylcyclohexyl)-N-(1H-indol-3-ylmethyl) acetamide; hydrochloride 2-(4-dimethylamino-4-phenylcyclohexyl)-N-[4-(1H-indol-3-yl)-butyl] acetamide; hydrochloride 2-(4-dimethylamino-4-phenylcyclohexyl)-N-[3-(1H-indol-3-yl)-propyl] acetamide; hydrochloride
2-(4-dimethylamino-4-phenylcyclohexyl)-N-[5-(1H-indol-3-yl)-pentyl]acetamide; hydrochloride
2-(4-dimethylamino-4-phenylcyclohexyl)-N-[2-(1H-indol-3-yl)-1-methylethyl] acetamide; hydrochloride
2-[4-dimethylamino-4-(4-fluorophenyl)-cyclohexyl]-N-(3-phenylpropyl) acetamide; hydrochloride
2-(4-dimethylamino-4-phenylcyclohexyl)-N-[6-(1H-indol-3-yl)-hexyl] acetamide; hydrochloride
2-[4-dimethylamino-4-(4-fluorophenyl)-cyclohexyl]-N-[2-(1H-indol-3-yl)-ethyl] acetamide; hydrochloride
2-[4-dimethylamino-4-(4-fluorophenyl)-cyclohexyl]-N-[2-(1H-indol-3-yl)-1-methylethyl] acetamide; hydrochloride
2-(4-dimethylamino-4-pyridin-2-yl-cyclohexyl)-N-(3-phenylpropyl) acetamide; hydrochloride
2-(4-dimethylamino-4-pyridin-2-yl-cyclohexyl)-N-[2-(1H-indol-3-yl)-ethyl] acetamide; hydrochloride
2-[4-dimethylamino-4-(3-fluorophenyl)-cyclohexyl]-N-[2-(1H-indol-3-yl)-ethyl] acetamide; hydrochloride
2-[4-dimethylamino-4-(3-fluorophenyl)-cyclohexyl]-N-(3-phenylpropyl) acetamide; hydrochloride
2-[4-dimethylamino-4-(3-fluorophenyl)-cyclohexyl]-N-[2-(1H-indol-3-yl)-1-methylethyl] acetamide; hydrochloride
2-(4-dimethylamino-4-phenyl-cyclohexyl)-N-phenyl acetamide; hydrochloride
2-(4-dimethylamino-4-phenyl-cyclohexyl)-N-(4-fluoro-phenyl) acetamide; hydrochloride
2-(4-dimethylamino-4-phenyl-cyclohexyl)-N-p-tolyl acetamide; hydrochloride
2-(4-dimethylamino-4-phenyl-cyclohexyl)-N-(4-methoxy-phenyl) acetamide; hydrochloride
in the form of the racemate; enantiomers, diastereomers, mixtures of enantiomers or diastereomers or a single enantiomer or diastereomer; of bases and/or acids of physiologically compatible acids or cations.

**11.** Process for the production of substituted cyclohexylacetic acid derivatives according to one of claims 1-10, **characterised in that**

(i) phosphonacetates are converted in the presence of a strong base, preferably potassium tert. butylate, sodium hydride or butyl lithium, with aminocyclohexanone derivatives, and after ester hydrolysis using dehydrating reagents, preferably a carbodiimide, the products are converted with amines of the formula $R_4R_5NH$ to the corresponding compounds according to the invention with the formula Ia,

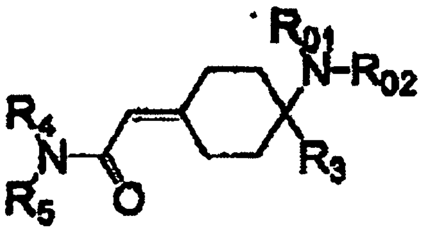

Ia

which are possibly reduced to the compounds according to the invention with the formula Ib

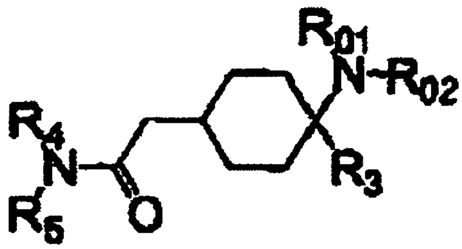

Ib

preferably by heterogeneous, catalytic hydration on palladium or platinum catalysts or by homogeneously catalysed hydration with rhodium catalysts; or
(ii) the phosphonacetates are converted in the presence of a strong base, preferably nBuLi, and an anilin directly

to the compounds according to the invention with the formula Ic

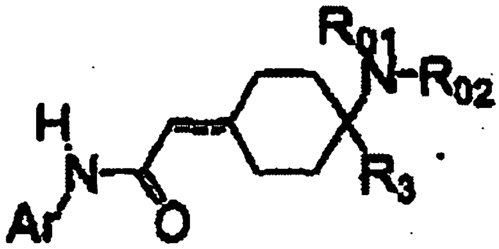

Ic

or

(iii)phosphonacetates are converted in the presence of a strong base, preferably potassium tert. butylate, sodium hydride or butyl lithium, with cyclohexan-1,4-dione derivatives, and after ester hydrolysis using dehydrating reagents, preferably a carbodiimide, the products are converted with amines of the formula $R_4R_5NH$ and are then converted in the presence of $HNR_{01}R_{02}$ with a cyanide, preferably potassium cyanide or TMSCN, and are finally converted with organometallic reagents, preferably Grignard or organolithium reagents of the formula metal $R_3$ to the compounds according to the invention with the formula Ia,

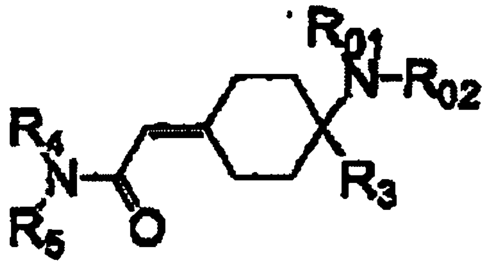

Ia

which are possibly reduced to the compounds according to the invention with the formula Ib

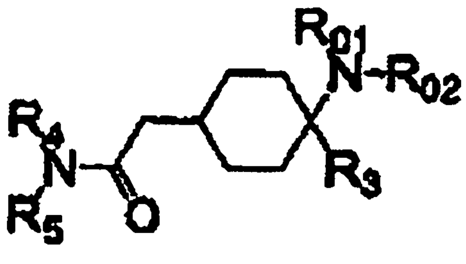

Ib

preferably by heterogeneous, catalytic hydration on palladium or platinum catalysts or by homogeneously catalysed hydration with rhodium catalysts.

**12.** Process for the production of substituted cyclohexylacetic acid derivatives according to claim 11, alternative (i), **characterised in that** subsequently to the conversion with a phosphonacetate, the double bond is reduced, preferably by heterogeneous, catalytic hydration on palladium or platinum catalysts or by homogeneously catalysed hydration with rhodium catalysts.

**13.** Process for the production of substituted cyclohexylacetic acid derivatives according to claim 11, alternative (iii), **characterised in that** subsequently to the conversion with amines of the formula $R_4R_5NH$, the double bond is reduced, preferably by heterogeneous, catalytic hydration on palladium or platinum catalysts or by homogeneously catalysed hydration with rhodium catalysts.

**14.** Medication containing at least one substituted cyclohexylacetate derivative according to one of claims 1 to 10 as well as possibly suitable additives and/or adjuvants and/or possibly further active substances.

**15.** Use of a substituted cyclohexylacetate derivative according to one of claims 1 to 10 for the production of a medication for the treatment of pain, in particular acute, visceral, neuropathic or chronic pain; or for the treatment of anxiety conditions, stress and syndromes associated with stress, depressive illnesses, catalepsy, epilepsy, Alzheimer's disease, senile dementia, general cognitive dysfunction disorders, learning and memory disorders (as nootropic), withdrawal symptoms, alcohol and/or drug and/or medication abuse, and/or dependence, sexual dysfunction, cardiovascular diseases, hypotension, hypertension, tinitus, pruritus, migraine, impaired hearing, poor intestinal motility, eating disorders, anorexia, obesity, locomotive disorders, diarrhoea, cachexia, urinary incontinence, or as muscle relaxant, anti-convulsant or anaesthetic, or for co-administration in treatment with an opioid analgesic or with an anaesthetic, for diuresis or antinatriuresis, anxiolysis, for modulation of movement activity, for modulation of neurotransmitter release and treatment of neurodegenerative disorders associated therewith, for the treatment of withdrawal symptoms and/or for reducing the addiction potential of opioids.

## Revendications

**1.** Dérivés de dérivés d'acide cyclohexylacétique substitués de formule générale I,

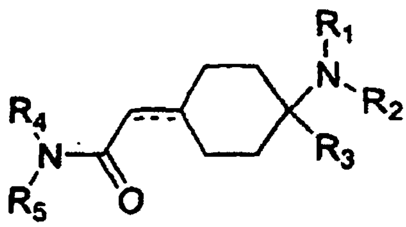

I

dans laquelle

------

représente une liaison simple ou une double liaison C-C,

$R^1$ et $R^2$ représentent, indépendamment l'un de l'autre, H ; CHO ; un groupe alkyle en $C_1$-$C_5$ respectivement saturé ou insaturé, ramifié ou non ramifié, non substitué ou une ou plusieurs fois substitué ; cycloalkyle en $C_3$-$C_8$, respectivement saturé ou insaturé, non substitué ou une ou plusieurs fois substitué ; ou un radical aryle, cycloalkyle en $C_3$-$C_8$ ou hétéroaryle, lié par alkyle en $C_1$-$C_3$, chaque fois non substitué ou une ou plusieurs fois substitué ;

ou les radicaux $R^1$ et $R^2$ représentent ensemble $CH_2CH_2OCH_2CH_2$, $CH_2CH_2NR^{10}CH_2CH_2$ ou $(CH_2)_{3-6}$,

$R^{10}$ représentant H ; un groupe alkyle en $C_1$-$C_5$ respectivement saturé ou insaturé, ramifié ou non ramifié, non substitué ou une ou plusieurs fois substitué ; cycloalkyle en $C_3$-$C_8$, respectivement saturé ou insaturé, non substitué ou une ou plusieurs fois substitué ; un groupe aryle ou hétéroaryle, chacun non substitué ou une ou plusieurs fois substitué ; ou un radical aryle, cycloalkyle en $C_3$-$C_8$ ou hétéroaryle, lié par alkyle en $C_1$-$C_3$, chaque fois non substitué ou une ou plusieurs fois substitué ;

$R^3$ représente un radical alkyle en $C_1$-$C_5$, respectivement saturé ou insaturé, ramifié ou non ramifié, non substitué ou une ou plusieurs fois substitué ; cycloalkyle en $C_3$-$C_8$, respectivement saturé ou insaturé, non substitué ou une ou plusieurs fois substitué ; un radical aryle, hétéroaryle ou cycloalkyle en $C_3$-$C_8$, lié par alkyle en $C_1$-$C_3$, chaque fois non substitué ou une ou plusieurs fois substitué ; un radical naphtyle, anthracényle, thiophényle, benzothiophényle, furyle, benzofurannyle, benzodioxolanyle, indolyle, indanyle, benzodioxanyle, pyrrolyle, pyridyle, pyrimidyle

ou pyrazinyle, chacun non substitué ou une ou plusieurs fois substitué ; phényle, 2-fluorophényle, 3-fluorophényle, 4-fluorophényle, 2-chlorophényle, 4-chlorophényle, 3-chlorophényle, 2-bromophényle, 3-bromophényle, 4-bromophényle, 2-cyanophényle, 3-cyanophényle, 4-cyanophényle, 2-méthylphényle, 3-méthylphényle, 4-méthylphényle, 2-méthoxyphényle, 3-méthoxyphényle, 4-méthoxyphényle, 2-trifluorométhylphényle, 3-trifluorométhylphényle, 4-trifluorométhylphényle, 2-éthylphényle, 3-éthylphényle, 4-éthylphényle, 2-éthoxyphényle, 3-éthoxyphényle, 4-éthoxyphényle, 2-hydroxyphényle, 3-hydroxyphényle, 4-hydroxyphényle, 2,3-dichlorophényle, 3,4-dichlorophényle, 3,5-dichlorophényle, 2,4-dichlorophényle, 2,3-difluorophényle, 3,4-difluorophényle, 3,5-difluorophényle, 2,4-difluorophényle, 2-fluoro-3-chlorophényle, 2-chloro-3-fluorophényle, 2-chloro-4-fluorophényle, 2-fluoro-4-chlorophényle, 4-fluoro-3-chlorophényle, 4-fluoro-3-méthylphényle, 4-tert-butylphényle, 4-fluoro-3-chlorophényle, 4-bromo-3-fluorophényle, 3,5-bis(trifluoro-méthyl)phényle, 4-chloro-2-trifluorométhylphényle, 2-méthoxy-5-méthylphényle, 5-chloro-2-méthoxyphényle, 4-phénoxyphényle, 2-méthylthiophényle, 3-méthylthiophényle, 4-méthylthiophényle, 5-fluoro-2-méthoxyphényle, 4-chloro-3-trifluorométhyle ou 4-bromo-2-méthylphényle ;
$R^4$ représente $-(CR^6R^7)_nR^8$,
n représentant 0, 1, 2, 3, 4, 5 ou 6
$R^6$ représentant H ou un groupe alkyle en $C_1$-$C_5$, ramifié ou non ramifié, saturé ou insaturé, non substitué ou une ou plusieurs fois substitué,
$R^7$ représentant H, un groupe alkyle en $C_1$-$C_5$, ramifié ou non ramifié, saturé ou insaturé, non substitué ou une ou plusieurs fois substitué, ou $COOR^9$,
ou $R^6$ et $R^7$ formant un cycle $(CH_2)_kCHR^8(CH_2)_m$, où k = 1, 2, 3 et m = 1, 2
$R^8$ représentant un radical cycloalkyle en $C_3$-$C_8$, aryle ou hétéroaryle, chacun non substitué ou une ou plusieurs fois substitué,
$R^9$ représentant H ou un groupe alkyle en $C_1$-$C_5$, ramifié ou non ramifié, saturé ou insaturé, non substitué ou une ou plusieurs fois substitué
$R^5$ représente H ou $-(CH_2)_1R^8$,
1 représentant 1, 2 ou 3,
ou représente conjointement avec $R^4$ $CH_2CH_2OCH_2CH_2$, $CH_2CH_2NR^{11}CH_2CH_2$ ou $(CH_2)_{3-6}$,
$R^{11}$ représentant H ; un groupe alkyle en $C_1$-$C_5$ respectivement saturé ou insaturé, ramifié ou non ramifié, non substitué ou une ou plusieurs fois substitué ; cycloalkyle en $C_3$-$C_8$, respectivement saturé ou insaturé, non substitué ou une ou plusieurs fois substitué ; un radical aryle ou hétéroaryle, chacun non substitué ou une ou plusieurs fois substitué ; ou un radical aryle, cycloalkyle en $C_3$-$C_8$ ou hétéroaryle, lié par alkyle en $C_1$-$C_3$, chacun non substitué ou une ou plusieurs fois substitué ;
sous forme du racémate ; des énantiomères, des diastéréoisomères, de mélanges des énantiomères ou diastéréoisomères ou d'un énantiomère ou diastéréoisomère individuel ; des bases et/ou des sels avec des acides ou cations physiologiquement acceptables.

2. Dérivés d'acide cyclohexylacétique substitués, selon la revendication 1, **caractérisés en ce que** $R^1$ et $R^2$ représentent, indépendamment l'un de l'autre, H ; un groupe alkyle en $C_1$-$C_5$ saturé ou insaturé, ramifié ou non ramifié, non substitué ou une ou plusieurs fois substitué ;
ou les radicaux $R^1$ et $R^2$ forment ensemble un cycle et représentent $CH_2CH_2OCH_2CH_2$, $CH_2CH_2NR^{10}CH_2CH_2$ ou $(CH_2)_{3-6}$,
$R^{10}$ représentant H ; un groupe alkyle en $C_1$-$C_5$ saturé ou insaturé, ramifié ou non ramifié, non substitué ou une ou plusieurs fois substitué.

3. Dérivés d'acide cyclohexylacétique substitués, selon la revendication 1, **caractérisés en ce que** $R^1$ et $R^2$ représentent, indépendamment l'un de l'autre, $CH_3$ ou H, $R^1$ et $R^2$ ne représentant pas simultanément H.

4. Dérivés d'acide cyclohexylacétique substitués, selon l'une quelconque des revendications 1 à 3, **caractérisés en ce que** $R^3$ représente un radical cyclopentyle, cyclohexyle, naphtyle, thiophényle, benzothiophényle, furyle, benzofurannyle, benzodioxolanyle, indolyle, indanyle, benzodioxanyle ou pyridyle, chacun non substitué ou une ou plusieurs fois substitué ; un radical cycloalkyle en $C_5$-$C_6$, phényle, naphtyle, anthracényle, thiophényle, benzothiophényle, pyridyle, furyle, benzofurannyle, benzodioxolanyle, indolyle, indanyle, benzodioxanyle, pyrrolyle, pyrimidyle ou pyrazinyle, chacun non substitué ou une ou plusieurs fois substitué, lié par un groupe alkyle en $C_1$-$C_2$ saturé, non ramifié ; un radical phényle, 2-fluorophényle, 3-fluorophényle, 4-fluorophényle, 2-chlorophényle, 4-chlorophényle, 3-chlorophényle, 2-bromophényle, 3-bromophényle, 4-bromophényle, 2-cyanophényle, 3-cyanophényle, 4-cyanophényle, 2-méthylphényle, 3-méthylphényle, 4-méthylphényle, 2-méthoxyphényle, 3-méthoxyphényle, 4-méthoxyphényle, 2-trifluorométhylphényle, 3-trifluorométhylphényle, 4-trifluorométhylphényle, 2-éthylphényle, 3-éthylphényle, 4-éthylphényle, 2-éthoxyphényle, 3-éthoxyphényle, 4-éthoxyphényle, 2-hydroxyphényle, 3-hydroxyphényle, 4-hydroxyphényle, 2,3-dichlorophényle, 3,4-dichlorophényle, 3,5-dichlorophényle, 2,4-dichlorophényle, 2,3-difluorophényle, 3,4-difluorophényle, 3,5-difluorophényle, 2,4-difluorophényle, 2-fluoro-3-chlorophényle, 2-chloro-3-fluorophényle, 2-chloro-4-fluorophényle, 2-fluoro-4-chlorophényle, 4-fluoro-3-chlorophényle, 4-fluoro-3-méthylphényle, 4-tert-butylphényle, 4-fluoro-3-chlorophényle, 4-bromo-3-fluorophényle, 3,5-bis(trifluorométhyl)phényle, 4-chloro-2-trifluorométhylphényle, 2-méthoxy-5-méthylphényle, 5-chloro-2-méthoxyphényle, 4-phénoxyphényle, 2-méthylthiophényle, 3-méthylthiophényle, 4-méthylthiophényle, 5-fluoro-2-méthoxyphényle, 4-chloro-3-trifluorométhyle ou 4-bromo-2-méthylphényle ;
en particulier
$R^3$ représente un radical naphtyle, thiophényle ou pyridyle, chacun non substitué ou une ou plusieurs fois substitué ; un radical cycloalkyle en $C_5$-$C_6$, phényle, naphtyle, thiophényle, pyridyle, chacun non substitué ou une ou plusieurs fois substitué, lié par un groupe alkyle en $C_1$-$C_2$ saturé, non ramifié ; un radical phényle, 2-fluorophényle, 3-fluorophényle, 4-fluorophényle, 2-chlorophényle, 4-chlorophényle, 3-chlorophényle, 2-cyanophényle, 3-cyanophényle, 4-cyanophényle, 2-méthylphényle, 3-méthylphényle, 4-méthylphényle, 2-méthoxyphényle, 3-méthoxyphényle, 4-méthoxyphényle, 2-trifluorométhylphényle, 3-trifluorométhylphényle, 4-trifluorométhylphényle, 2-éthylphényle, 3-éthylphényle, 4-éthylphényle, 2-éthoxyphényle, 3-éthoxyphényle, 4-éthoxyphényle, 2,3-dichlorophényle, 3,4-dichlorophényle, 3,5-dichlorophényle, 2,4-dichlorophényle, 2,3-difluorophényle, 3,4-difluorophényle, 3,5-difluorophényle, 2,4-difluorophényle, 2-fluoro-3-chlorophényle, 2-chloro-3-fluorophényle, 2-chloro-4-fluorophényle, 2-fluoro-4-chlorophényle, 4-fluoro-3-chlorophényle, 4-fluoro-3-méthylphényle, 4-tert-butylphényle, 4-fluoro-3-chlorophényle, 3,5-bis(trifluorométhyl)phényle, 4-chloro-2-trifluorométhylphényle, 2-méthoxy-5-méthylphényle, 5-chloro-2-méthoxyphényle, 4-phénoxyphényle, 2-méthylthiophényle, 3-méthylthiophényle, 4-méthylthiophényle ou 4-chloro-3-trifluorométhyle.

5. Dérivés d'acide cyclohexylacétique substitués, selon l'une quelconque des revendications 1 à 3, **caractérisés en ce que** $R^3$ représente le groupe pyridyle, phényle, 3-fluorophényle ou 4-fluorophényle.

6. Dérivés d'acide cyclohexylacétique substitués, selon l'une quelconque des revendications 1 à 5, **caractérisés en ce que** $R^6$ représente H et $R^7$ représente H, $CH_3$ ou $COOR^9$, ou $R^6$ et $R^7$ forment un cycle $(CH_2)_kCHR^8(CH_2)_m$ où k = 1, 2 ou 3 et m = 1 ou 2.

7. Dérivés d'acide cyclohexylacétique substitués, selon l'une quelconque des revendications 1 à 6, **caractérisés en ce que** $R^5$ représente H.

8. Dérivés d'acide cyclohexylacétique substitués, selon l'une quelconque des revendications 1 à 7, **caractérisés en ce que** $R^8$ représente un radical cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxanyle, benzodioxolanyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinoléinyle, isoquinoléinyle, phtalazinyle ou quinazolinyle, chacun non substitué ou une ou plusieurs fois substitué,
en particulier
$R^8$ représente un radical cyclopentyle, cyclohexyle, cycloheptyle, cyclo-octyle, anthracényle, indolyle, naphtyle, benzofurannyle, benzothiophényle, indanyle, benzodioxanyle, benzodioxolanyle, acénaphtyle, carbazolyle, phényle, thiophényle, furyle, pyridyle, pyrrolyle, pyrazinyle ou pyrimidyle, chacun non substitué ou une ou plusieurs fois substitué.

9. Dérivés d'acide cyclohexylacétique substitués, selon l'une quelconque des revendications 1 à 7, **caractérisés en ce que** $R^8$ représente un radical phényle ou indolyle, chacun une ou plusieurs fois substitué.

10. Dérivés d'acide cyclohexylacétique substitués, selon l'une quelconque des revendications 1 à 9, choisis dans l'ensemble
2-(4-diméthylamino-4-phényl-cyclohexylidène)-N-[2-(1H-indol-3-yl)-éthyl]-acétamide ; chlorhydrate
2-[2-(4-diméthylamino-4-phényl-cyclohexylidène)-acétylamino]-3-(1H-indol-3-yl)-propionate de méthyle ; chlorhydrate
2-(4-diméthylamino-4-phényl-cyclohexylidène)-N-(3-phényl-propyl)-acétamide ; chlorhydrate
N-benzyl-2-(4-diméthylamino-4-phényl-cyclohexylidène)-acétamide ; chlorhydrate
2-[2-(4-diméthylamino-4-phényl-cyclohexylidène)-acétylamino]-3-(1H-indol-3-yl)-propionate de méthyle ; chlorhydrate
2-(4-diméthylamino-4-phényl-cyclohexylidène)-N-phénéthyl-acétamide ; chlorhydrate
2-(4-diméthylamino-4-phényl-cyclohexylidène)-N-[2-(4-fluoro-phényl)-éthyl]-acétamide ; chlorhydrate

2-(4-diméthylamino-4-phényl-cyclohexylidène)-N-(4-fluoro-benzyl)-acétamide ; chlorhydrate
2-(4-diméthylamino-4-phényl-cyclohexylidène)-N-(2-trifluorométhyl-benzyl)-acétamide ; chlorhydrate
2-(4-diméthylamino-4-phényl-cyclohexylidène)-N-(4-fluoro-phényl)-acétamide ; chlorhydrate
2-(4-diméthylamino-4-phényl-cyclohexylidène)-N-[2-(1H-indol-3-yl)-1-méthyl-éthyl]-acétamide ; chlorhydrate
2-(4-diméthylamino-4-phényl-cyclohexylidène)-N-(4-phényl-butyl)-acétamide ; chlorhydrate
2-(4-diméthylamino-4-phényl-cyclohexylidène)-N-(1H-indol-3-ylméthyl)-acétamide ; chlorhydrate
2-(4-diméthylamino-4-phényl-cyclohexylidène)-N-[4-(1H-indol-3-yl)-butyl]-acétamide ; chlorhydrate
2-(4-diméthylamino-4-phényl-cyclohexylidène)-N-[3-(1H-indol-3-yl)-propyl]-acétamide ; chlorhydrate
2-(4-diméthylamino-4-phényl-cyclohexylidène)-N-[5-(1H-indol-3-yl)-pentyl]-acétamide ; chlorhydrate
2-(4-diméthylamino-4-phényl-cyclohexylidène)-N-[6-(1H-indol-3-yl)-hexyl]-acétamide ; chlorhydrate
2-[4-diméthylamino-4-(4-fluoro-phényl)-cyclohexylidène]-N-(3-phényl-propyl)-acétamide ; chlorhydrate
2-[4-diméthylamino-4-(4-fluoro-phényl)-cyclohexylidène]-N-[2-(1H-indol-3-yl)-éthyl]-acétamide ; chlorhydrate
2-[4-diméthylamino-4-(4-fluoro-phényl)-cyclohexylidène]-N-[2-(1H-indol-3-yl)-1-méthyl-éthyl]-acétamide ; chlorhydrate
2-(4-diméthylamino-4-pyridin-2-yl-cyclohexylidène)-N-[2-(1H-indol-3-yl)-éthyl]-acétamide ; chlorhydrate
2-(4-diméthylamino-4-pyridin-2-yl-cyclohexylidène)-N-(3-phényl-propyl)-acétamide ; chlorhydrate
2-[4-diméthylamino-4-(3-fluoro-phényl)-cyclohexylidène]-N-[2-(1H-indol-3-yl)-éthyl]-acétamide ; chlorhydrate
2-[4-diméthylamino-4-(3-fluoro-phényl)-cyclohexylidène]-N-(3-phényl-propyl)-acétamide ; chlorhydrate
2-[4-diméthylamino-4-(3-fluoro-phényl)-cyclohexylidène]-N-[2-(1H-indol-3-yl)-1-méthyl-éthyl]-acétamide ; chlorhydrate
2-(4-diméthylamino-4-phénylcyclohexyl)-N-(2-trifluorométhyl-benzyl)-acétamide ; chlorhydrate
2-(4-diméthylamino-4-phénylcyclohexyl)-N-[2-(1H-indol-3-yl)-éthyl]-acétamide ; chlorhydrate
2-(4-diméthylamino-4-phénylcyclohexyl)-N-(3-phénylpropyl)-acétamide ; chlorhydrate
2-(4-diméthylamino-4-phénylcyclohexyl)-N-phénéthylacétamide ; chlorhydrate
2-(4-diméthylamino-4-phénylcyclohexyl)-N-(4-phénylbutyl)-acétamide ; chlorhydrate
2-[2-(4-diméthylamino-4-phénylcyclohexyl)-acétylamino]-3-(1H-indol-3-yl)-propionate de méthyle ; chlorhydrate
2-[2-(4-diméthylamino-4-phénylcyclohexyl)-acétylamino]-3-(1H-indol-3-yl)-propionate de potassium
2-(4-diméthylamino-4-phénylcyclohexyl)-N-(1H-indol-3-ylméthyl)-acétamide ; chlorhydrate
2-(4-diméthylamino-4-phénylcyclohexyl)-N-[4-(1H-indol-3-yl)-butyl]-acétamide ; chlorhydrate
2-(4-diméthylamino-4-phénylcyclohexyl)-N-[3-(1H-indol-3-yl)-propyl]-acétamide ; chlorhydrate
2-(4-diméthylamino-4-phénylcyclohexyl)-N-[5-(1H-indol-3-yl)-pentyl]-acétamide ; chlorhydrate
2-(4-diméthylamino-4-phénylcyclohexyl)-N-[2-(1H-indol-3-yl)-1-méthyléthyl]-acétamide ; chlorhydrate
2-[4-diméthylamino-4-(4-fluorophényl)-cyclohexyl]-N-(3-phénylpropyl)-acétamide ; chlorhydrate
2-(4-diméthylamino-4-phénylcyclohexyl)-N-[6-(1H-indol-3-yl)-hexyl]-acétamide ; chlorhydrate
2-[4-diméthylamino-4-(4-fluorophényl)-cyclohexyl]-N-[2-(1H-indol-3-yl)-éthyl]-acétamide ; chlorhydrate
2-[4-diméthylamino-4-(4-fluorophényl)-cyclohexyl]-N-[2-(1H-indol-3-yl)-1-méthyléthyl]-acétamide ; chlorhydrate
2-(4-diméthylamino-4-pyridin-2-yl-cyclohexyl)-N-(3-phénylpropyl)-acétamide ; chlorhydrate
2-(4-diméthylamino-4-pyridin-2-yl-cyclohexyl)-N-[2-(1H-indol-3-yl)-éthyl]-acétamide ; chlorhydrate
2-[4-diméthylamino-4(3-fluorophényl)-cyclohexyl]-N-(2-(1H-indol-3-yl)-éthyl]-acétamide ; chlorhydrate
2-[4-diméthylamino-4-(3-fluorophényl)-cyclohexyl]-N-(3-phénylpropyl)-acétamide ; chlorhydrate
2-[4-diméthylamino-4-(3-fluorophényl)-cyclohexyl]-N-[2-(1H-indol-3-yl)-1-méthyléthyl]-acétamide ; chlorhydrate
2-(4-diméthylamino-4-phényl-cyclohexyl)-N-phényl-acétamide-chlorhydrate
2-(4-diméthylamino-4-phényl-cyclohexyl)-N-(4-fluoro-phényl)-acétamide-chlorhydrate
2-(4-diméthylamino-4-phényl-cyclohexyl)-N-p-tolyl-acétamide-chlorhydrate
2-(4-diméthylamino-4-phényl-cyclohexyl)-N-(4-méthoxy-phényl)-acétamide-chlorhydrate
sous forme du racémate ; des énantiomères, des diastéréoisomères, de mélanges des énantiomères ou diastéréoisomères ou d'un énantiomère individuel ou d'un diastéréoisomère individuel ; des bases et/ou des sels avec des acides ou cations physiologiquement acceptables.

**11.** Procédé pour la préparation de dérivés d'acide cyclohexylacétique substitués, selon l'une quelconque des revendications 1 à 10, **caractérisé en ce que**

(i) on fait réagir un ester d'acide phosphonoacétique avec des dérivés d'aminocyclohexanone, en présence d'une base forte, de préférence le tert-butylate de potassium, l'hydrure de sodium ou le butyllithium, et on fait réagir les produits, après hydrolyse d'ester à l'aide de réactifs de déshydratation, de préférence d'un carbodiimide, avec des amines de formule $R_4R_5NH$, pour aboutir aux composés correspondants conformes à l'invention, selon la formule Ia

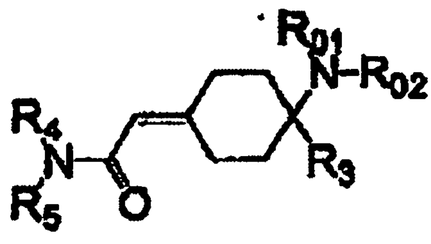

Ia

qui sont éventuellement réduits en composés conformes à l'invention selon la formule Ib

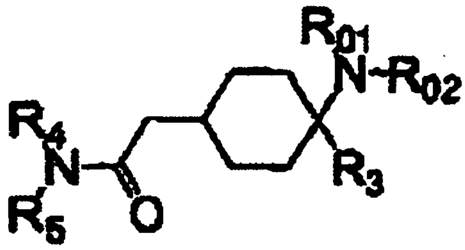

Ib

de préférence par hydrogénation catalytique hétérogène sur des catalyseurs au platine ou au palladium ou par hydrogénation avec catalyse homogène à l'aide de catalyseurs au rhodium ; ou

(ii) on convertit directement l'ester d'acide phosphonoacétique en présence d'une base, de préférence nBuLi, et d'une amine, en les composés conformes à l'invention selon la formule Ic ;

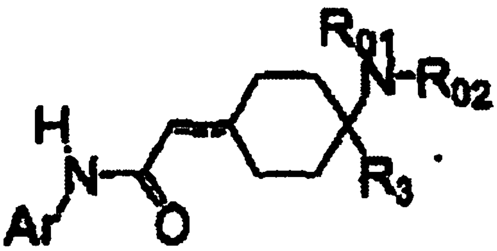

Ic

ou

(iii) on fait réagir des esters d'acide phosphonoacétique avec des dérivés de cyclohexane-1,4-dione en présence d'une base forte, de préférence le tert-butylate de potassium, l'hydrure de sodium ou le butyllithium, et on fait réagir les produits, après hydrolyse d'ester à l'aide de réactifs de déshydratation, de préférence d'un carbodiimide, avec des amines de formule $R_4R_5NH$ et ensuite avec un cyanure, de préférence le cyanure de potassium ou TMSCN en présence de $HNR_{01}R_{02}$, et enfin on les fait réagir avec des réactifs organométalliques, de préférence des réactifs de Grignard ou organolithiés de formule métal-$R_3$, pour aboutir aux composés conformes à l'invention selon la formule Ia

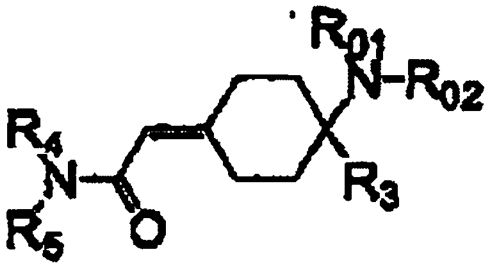

Ia

qui sont éventuellement réduits en composés conformes à l'invention selon la formule Ib

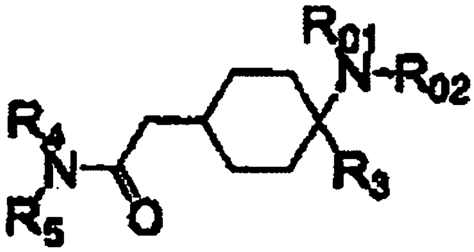

Ib

de préférence par hydrogénation catalytique hétérogène sur des catalyseurs au platine ou au palladium ou par hydrogénation avec catalyse homogène à l'aide de catalyseurs au rhodium.

**12.** Procédé pour la préparation de dérivés d'acide cyclohexylacétique substitués, selon la revendication 11, méthode (i), **caractérisé en ce qu'**à la suite de la réaction avec un ester d'acide phosphonoacétique la double liaison est réduite, de préférence par hydrogénation catalytique hétérogène sur des catalyseurs au platine ou au palladium ou par hydrogénation avec catalyse homogène à l'aide de catalyseurs au rhodium.

**13.** Procédé pour la préparation de dérivés d'acide cyclohexylacétique substitués, selon la revendication 11, méthode (iii), **caractérisé en ce qu'**à la suite de la réaction avec des amines de formule $R_4R_5NH$ la double liaison est réduite, de préférence par hydrogénation catalytique hétérogène sur des catalyseurs au platine ou au palladium ou par hydrogénation avec catalyse homogène à l'aide de catalyseurs au rhodium.

**14.** Médicament contenant au moins un dérivé d'acide cyclohexylacétique substitué selon l'une quelconque des revendications 1 à 10 ainsi qu'éventuellement des adjuvants et additifs appropriés et/ou éventuellement d'autres substances actives.

**15.** Utilisation d'un dérivé d'acide cyclohexylacétique substitué selon l'une quelconque des revendications 1 à 10, pour la fabrication d'un médicament destiné au traitement de la douleur, en particulier de la douleur aiguë, viscérale, névropathique ou chronique ; ou au traitement d'états anxieux, du stress et de syndromes liés au stress, de dépressions, de la catalepsie, de l'épilepsie, de la maladie d'Alzheimer, de la démence sénile, de dysfonctionnements cognitifs généraux, de troubles de la mémoire et de l'apprentissage (en tant qu'agent nootrope), de manifestations inflammatoires, d'abus d'alcool et/ou de drogues et/ou de médicaments et/ou de la dépendance à ces substances, de dysfonctionnements sexuels, de maladies cardiovasculaires, de l'hypotension, de l'hypertension, de l'acouphène, du prurit, de la migraine, de troubles de l'audition, de la motilité intestinale insuffisante, de l'alimentation perturbée, de l'anorexie, de l'adiposité, de troubles locomoteurs, de la diarrhée, de la cachexie, de l'incontinence urinaire ou en tant que myorelaxant, anticonvulsivant ou anesthésique, ou à l'administration simultanée dans le traitement par un analgésique opioïde ou par un anesthésique, à la diurèse ou à l'antinatriurèse, à l'anxiolyse, à la modulation de l'activité motrice, à la modulation de la sécrétion de neurotransmetteurs et au traitement de maladies neurodégénératives qui y sont liées, au traitement de manifestations inflammatoires et/ou à la réduction du potentiel de dépendance des opioïdes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1323710 A
- US 2003236250 A
- WO 02090317 A
- WO 0290317 A


### In der Beschreibung aufgeführte Nicht-Patentliteratur


- *Opioids: Introduction,* 127-150
- Further Opioid Receptors. Analgesics - From Chemistry and Pharmacology to Clinical Applikation. Wiley VCH, 2002, 455-476
- Opioids with Clinical Relevance: Tramadol. **Tramadol, s.** Analgesics - From Chemistry and Pharmacology to Clinical Application. Wiley VCH, 2002, 228-230
- **Manabe et al.** *Nature,* 1997, vol. 394, 577-581
- **Nishi et al.** *EMBO J.,* 1997, vol. 16, 1858-1864
- **Calo et al.** *Br.J. Pharmacol.,* 2000, vol. 129, 1261-1283
- **Lednicer et al.** *J. Med. Chem.,* 1980, vol. 23, 424-430
- **Ardati et al.** *Mol. Pharmacol.,* 1997, vol. 51, 816-824
- **Whittaker.** *J. Anat.,* 1962, vol. 76, 79-88
- **M.Ch. Frink ; H.-H. Hennies ; W. Englberger ; M. Haurand ; B. Wilffert.** *Arzneim.-Forsch./Drug Res.,* 1996, vol. 46 (III), 1029-1036
- **E.G. Gray ; V.P. Whittaker.** *J. Anat.,* 1962, vol. 76, 79-88